# EUROPEAN PATENT APPLICATION

(11) **EP 4 098 649 A2**
(43) Date of publication of application: **07.12.2022**
(21) Application number: 21748005.2
(22) Date of filing: 28.01.2021
(51) Int. Cl.: C07C 59/40, C07C 59/42, A61K 31/201, A61K 31/202, A61P 35/00, A61P 9/00

(54) **ALPHA-HYDROXYLATED FATTY-ACID METABOLITES, MEDICAL USES OF SAME AND USE AS BIOMARKERS**

(30) Priority: 29.01.2020 ES 202030070; 28.02.2020 EP 20382145; 17.11.2020 ES 202031155
(71) Applicant: Universitat de les Illes Balears, 07122 Palma de Mallorca (Islas Baleares) (ES)
(72) Inventor: ESCRIBÁ RUIZ, Pablo Vicente, 07122 Palma de Mallorca (ES); TORRES CANALEJO, Manuel, 07122 Palma de Mallorca (ES); BUSQUETS XAUBET, Xavier, 07122 Palma de Mallorca (ES); LLADÓ CAÑELLAS, Victoria, 07121 Palma de Mallorca (ES); FERNÁNDEZ GARCÍA, Paula, 07121 Palma de Mallorca (ES); ROSSELLÓ CASTILLO, Catalina Ana, 07121 Palma de Mallorca (ES); PARETS BARRIOS, Sebastià, 07121 Palma de Mallorca (ES); BETETA GOBEL, Roberto, 07121 Palma de Mallorca (ES); CANO URREGO, Emilce, 07121 Palma de Mallorca (ES); ARBONA GONZÁLEZ, Laura, 07121 Palma de Mallorca (ES); RODRÍGUEZ LORCA, Raquel, 07121 Palma de Mallorca (ES); CABOT BAUZÁ, Juan, 07121 Palma de Mallorca (ES); MILLARES PIZÀ, Marc, 07121 Palma de Mallorca (ES)
(74) Representative: Manuel Illescas y Asociados, S.L.
(86) International application number: PCT/ES2021/070068
(87) International publication number: WO 2021/152201

(57) **Abstract**

Described are fatty acids with one or more unsaturations, having an odd hydrocarbon chain, the fatty acids having the chemical structure of the therapeutically active metabolites of even-chain mono- or polyunsaturated alpha-hydroxylated fatty acids. Also described are compositions comprising said fatty acids, medical uses thereof, and the use thereof as indicators of the efficacy of and/or response to the treatment of a patient with the even-chain mono- or polyunsaturated alpha-hydroxylated fatty acids from which they are derived.

## Description

### TECHNICAL FIELD OF THE INVENTION

The present invention relates to fatty acids with one or more unsaturations, of odd hydrocarbon chain, wherein the chemical structure of said fatty acids corresponds to that of the metabolites of mono- or polyunsaturated alpha-hydroxylated fatty acids. The present invention also relates to compositions comprising said odd-chain fatty acids, to their medical uses, as well as to their use as efficacy indicators for the treatment of a patient with the mono- or polyunsaturated alpha-hydroxylated fatty acids of which they are metabolites.

### BACKGROUND OF THE INVENTION

It is known that changes in the lipid composition of the membranes influence cell signaling, which can lead to the development of diseases, or reverse them, as well as to prevent them. Similarly, therapeutic interventions focused on regulating membrane lipid levels can prevent and reverse (cure) pathological processes.

In general, fatty acids whose chemical structure presents an odd number of carbon atoms have not been considered of therapeutic relevance since, in humans and, in general, in mammals, the immense majority of the fatty acids present are of even chain, usually between 14 and 24 carbon atoms, the presence of fatty acids of odd chain being very rare and limited to traces.

Currently, the data available in the scientific literature indicate that small structural differences in fatty acids have important effects for biological activity and, therefore, for therapeutic activity. It is well known that many drugs have known adverse effects or are toxic to various cells or tissues. Prodrugs are compounds that when ingested undergo metabolic reactions and give rise to a drug or medicine, their metabolite, which has an effect on the health of a patient or subject.

Thus, on the one hand, the administration of a therapeutically active compound in the form of a prodrug allows modulating the distribution and absorption of said compound over time, since its metabolism allows generating the drug, i.e. the metabolite, only in those cells or tissues in which the metabolic reactions that transform said prodrug into its active metabolite occur. In this regard, these prodrugs have other advantages, such as allowing a delayed or controlled administration of the active metabolite, avoiding accumulations thereof which could have harmful effects on the body. On the other hand, the identification and synthesis of said therapeutically active metabolites allow to act more intensely, making it possible to administer higher therapeutically active doses and in controlled timeframes, than those that would occur during the spontaneous metabolism of the corresponding prodrug. Therefore, it is an object of the present invention to provide therapeutically active compounds (metabolites) which are derived from other compounds or prodrugs, so that the administration of such metabolites, alone, by means of their prodrugs, or in combination with their respective prodrugs, makes it possible to modulate the therapeutic effect and the possible adverse side effects, depending on the condition of the patient and the pathological condition to be treated.

### BRIEF DESCRIPTION OF THE INVENTION

A compound selected from the group consisting of: a compound of formula (II), or a pharmaceutically or nutraceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically or nutraceutically acceptable salt or ester thereof:

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

wherein a is an integer between 1 and 14; *b* is an integer between 1 and 7; ***c*** is 0, 3 or 6 and *m=0;* and wherein a+3b+c+3 is an even integer.

More particularly, the present invention relates to a compound selected from the group consisting of: a compound of formula (II), or a pharmaceutically or nutraceutically acceptable salt or ester thereof:

COOH -(CH₂)***ₐ***-(CH=CH-CH₂)***_{b}***-(CH₂)***_{c}***-CH₃ (II)

wherein: a=6, b=1 and c=6; or a =6, b=2 and c=3; or a=6, b=3 and c=0; or a=3, b=3 and c=3; or a=2, *b=4* and c=3; or a=2, *b=5* and c=0; and a compound of formula (III), or a pharmaceutically or nutraceutically acceptable salt or ester thereof:

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

where a=1, b=6, c=0 and m= 0.

The present invention also relates to a compound selected from the group consisting of a compound of formula (II) and a compound of formula (III), as described herein, for use as a medicament and, in particular, for use in the induction of neuroregeneration and in the prevention and/or treatment (including maintenance treatment) of a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

The present invention also relates to a pharmaceutical or nutraceutical composition comprising at least a first compound selected from the group consisting of a compound of formula (II) and a compound of formula (III), and optionally a compound of formula (I), as described herein.

Finally, the present invention relates to an *in vitro* method of determining the efficacy of a therapeutic or preventive treatment of a disease or pathology with a compound of formula (I), or with a pharmaceutically acceptable salt or ester thereof, in a subject, wherein said method comprises determining *in vitro* in a biological sample of said subject, the amount of a compound of formula (II) or of formula (III), as described herein, or of its carboxylate anion, or of a derivative formed therefrom *in vivo* or *in vitro,* wherein said amount is related to the efficacy of the treatment of said disease or pathology

### DESCRIPTION OF THE INVENTION

The present invention relates to a compound selected from the group consisting of: a compound of formula (II), or a pharmaceutically or nutraceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically or nutraceutically acceptable salt or ester thereof:

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

wherein ***a*** is an integer between 1 and 14; ***b*** is an integer between 1 and 7; c is an integer between 0 and 14; ***m*** is an integer between 0 and (***b*** - 1); and wherein ***a*+3*b*+*c*+3** is an even integer.

All values of ***a, b, c*** and ***m*** of the present invention are integers greater than or equal to zero. Once the value of ***b*** is defined in a formula, the value of ***m*** is defined as an integer between 0 and (***b*** - 1) where said value of ***b*** is the one that has already been defined between 1 and 7. For purposes of the present invention, when no particular formula or composition is indicated, the values of ***a, b, c*** and ***m*** are applicable to all formulas and compositions described herein.

In one embodiment of the invention, ***a*** is an integer between 1 and 7; ***b*** is an integer between 2 and 7; ***c*** is 0, 3 or 6, ***m*** is 0 and ***a*+3*b*+*c*+3** is an even integer. In another embodiment of the invention, ***a*** is an integer between 1 and 14; ***b*** is 1; ***c*** is 0, 3 or 6, ***m*** is 0; and wherein ***a*+3*b*+*c*+3** is an even integer.

Most preferably, for all embodiments of the present invention ***m***=0 and thus the present invention relates to a compound selected from the group consisting of: a compound of formula (II), or a pharmaceutically or nutraceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically or nutraceutically acceptable salt or ester thereof:

COOH -(CH₂)**_{*a*+*3*}**-(CH=CH-CH₂)***_{(b-1)}***-(CH₂)***_{c}***-CH₃ (III);

wherein ***a*** is an integer between 1 and 14; ***b*** is an integer between 1 and 7; ***c*** is 0, 3 or 6; and wherein ***a*+3*b*+*c*+3** is an even integer.

A preferred embodiment of the invention relates to a pharmaceutically or nutraceutically acceptable salt or ester of a compound of formula (II) or formula (III). More preferably, said salt is a sodium salt, and said ester is a methyl or ethyl ester.

The compounds of formula (II) or formula (III) of the present invention correspond to the formulas of metabolites of a compound of formula (I), or of a pharmaceutically or nutraceutically acceptable salt or ester thereof:

COOH -CHOH-(CH₂)***ₐ***-(CH=CH-CH₂)***_{b}***-(CH₂)***_{c}***-CH₃ (I)

wherein ***a*** is an integer between 1 and 14; ***b*** is an integer between 1 and 7; **c** is an integer between 0 and 14; **m** is an integer between 0 and (***b*** - 1); and wherein ***a*+3*b*+*c*+3** is an even integer.

The compounds of formula (I) are therefore mono- or polyunsaturated alpha-hydroxylated fatty acids with an even number of carbon atoms **(*a*+3*b*+*c*+3** is an even integer).

On the one hand, the 2-hydroxymonounsaturated fatty acids of formula (I), of even chain, are prodrugs of other monounsaturated fatty acids of formula (II), of odd chain, as said prodrugs undergo a decarboxylation process.

On the other hand, the 2-hydroxypolyunsaturated fatty acids of formula (I), of even chain, are prodrugs of other mono- or polyunsaturated fatty acids, of odd chain, which, in the case in which a decarboxylation occurs, but the hydrogenation of one of the double bonds of the compound of formula (I) does not occur, the compound derived from the prodrug of formula (I) will be a compound of formula (II), while, in the case in which the hydrogenation of one of the double bonds and a decarboxylation of the compound of formula (I) occurs, the compound derived from the prodrug of formula (I) will be a compound of formula (III), in which the hydrogenated double bond may be in a different position depending on the value of ***m*.**

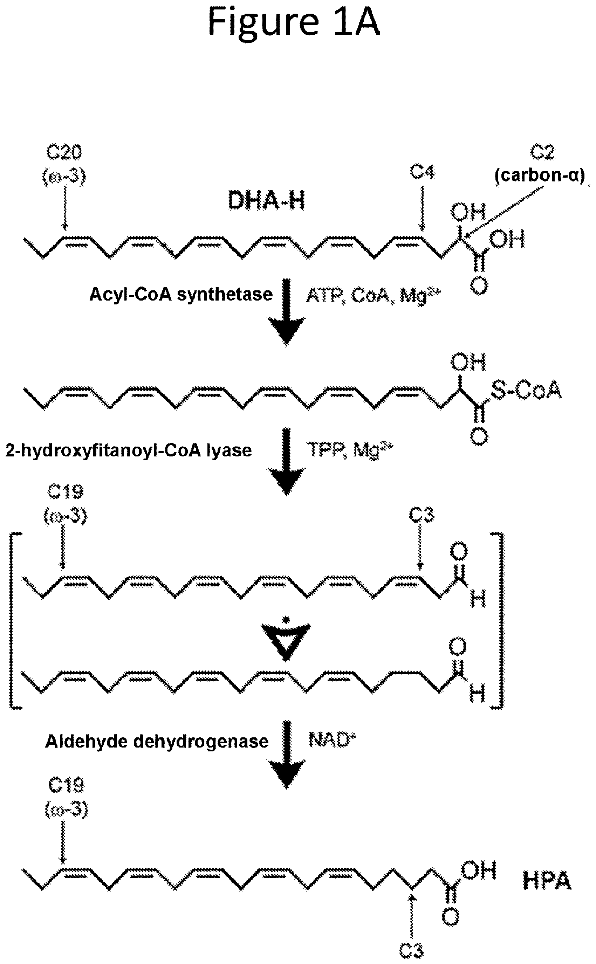

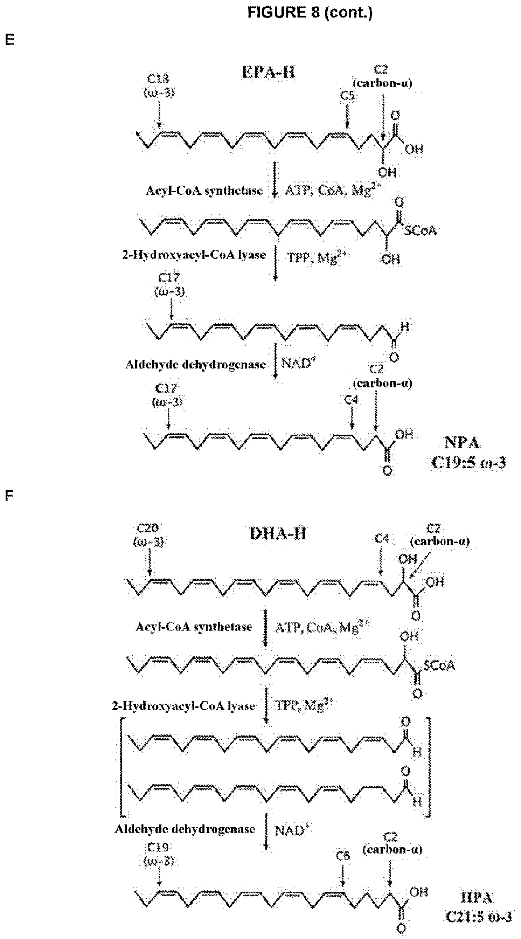

By way of illustration, figure 1A and figure 8F show a scheme of metabolism by α-oxidation of 2-hydroxidocosahexaenoic acid (DHA-H), a compound of formula (I), at the cellular level resulting in (6Z,9Z,12Z,15Z,18Z)-heneicose-6,9,12,15,18-pentaenoic acid (HPA), its metabolite of formula (III). According to this route, the DHA-H prodrug is a 2-hydroxylated polyunsaturated fatty acid, which is converted to HPA by a sequence of metabolic steps: (1) activation of DHA-H by conjugation with coenzyme A; (2) cleavage mediated by 2-hydroxyacyl-CoA lyase, resulting in an aldehyde with an odd number of carbon atoms; (3) action of the aldehyde dehydrogenase on the aldehyde, hydrogenating one of the double bonds, to transform it into an acid (HPA).

Thus, HPA, according to the invention, is a compound of formula (III), wherein ***m***=0**,** if HPA is the metabolite of a prodrug of formula (I) wherein ***a***=1, ***b**=6, **c***=0, which is an omega-3 polyunsaturated alpha-hydroxylated fatty acid of 22 carbon atoms and 6 conjugated double bonds (DHA-H). However, HPA can be a compound of formula (II), for cases where HPA is the metabolite of a prodrug of formula (I), wherein a=4, *b=5,* and c=0, which is an omega-3 polyunsaturated alpha-hydroxylated fatty acid of 22 carbon atoms and 5 double bonds (2-hydroxy-docosapentaenoic acid). An embodiment of the present invention relates to a compound of formula (III), wherein ***a***=1, ***b**=6, **c***=0 and ***m**=0.*

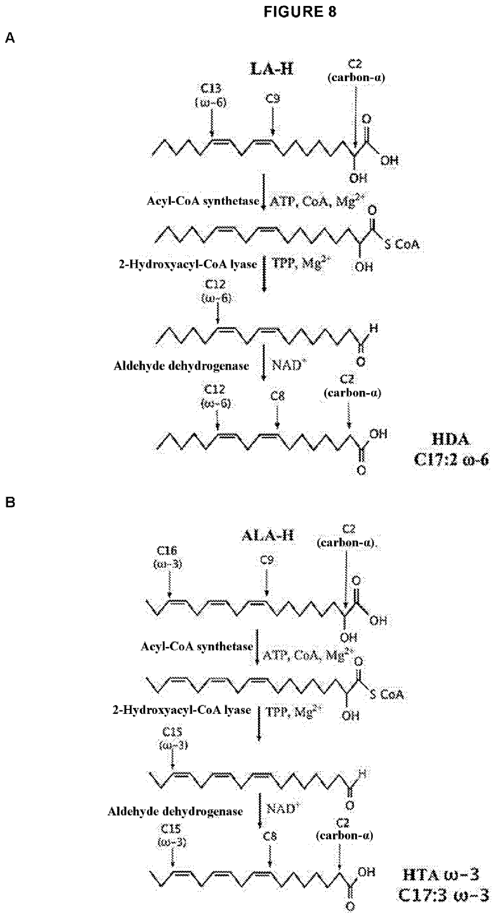

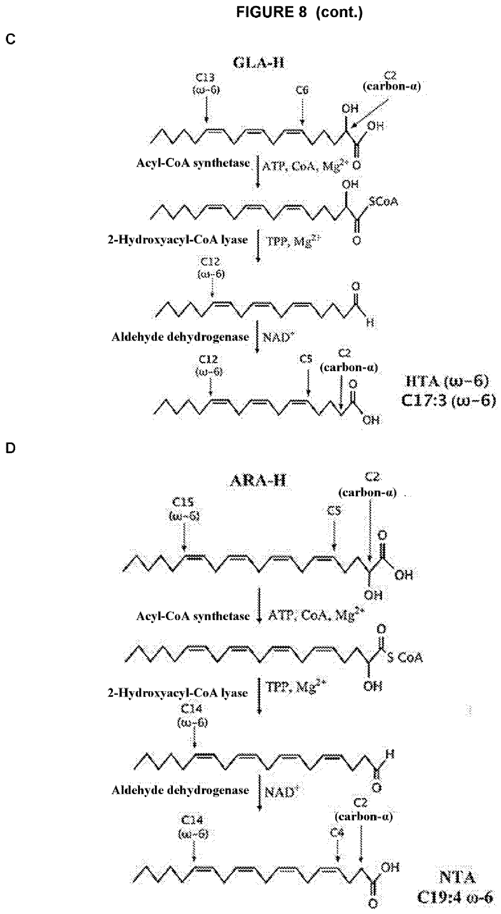

Additionally, figure 8 shows metabolism schemes for other fatty acids of formula (I). Specifically, figure 8A shows the scheme of metabolism of 2-hydroxy-linoleic acid (LA-H), compound of formula (I) in which ***a*=6, *b*=2** and ***c*=3,** to produce, after undergoing a decarboxylation, (8Z,11Z)-heptadeca-8,11-dienoic acid (HDA, C17:2 ω-6), which is a compound of formula (II) in which ***a*=6, *b*=2** and ***c*=3.** Figure 8B shows the scheme of metabolism of 2-hydroxy-alpha (a)-linolenic acid (ALA-H), which is a compound of formula (I) in which ***a*=6, *b*=3** and ***c*=0,** to produce, after undergoing a decarboxylation, (8Z,11Z,14Z)-heptadeca-8,11,14-trienoic acid (HTA ω-3, C17:3 ω-3), which is a compound of formula (II) in which ***a***=6**, *b***=3 and ***c***=0**.** On the other hand, figure 8C shows the scheme of the metabolism of 2-hydroxy-gamma (γ)-linolenic acid (GLA-H), which is a compound of formula (I) in which ***a***=3**, *b***=3 and ***c***=3**,** to produce, after undergoing a decarboxylation, (5Z,8Z,11Z)-heptadeca-5,8,11-trienoic acid (HTA ω-6, C17:3 ω-6), which is a compound of formula (II) in which ***a***=3**, *b***=3 and ***c***=3**.** Figure 8D shows the scheme of metabolism of 2-hydroxy-arachidonic acid (ARA-H), which is a compound of formula (I) in which ***a*=** 2, ***b***=4 and ***c***=3**,** to produce, after undergoing a decarboxylation, (4Z,7Z,10Z,13Z)-nonadeca-4,7,10,13-tetraenoic acid (NTA, C:19:4 ω-6), which is a compound of formula (II) in which **a**=2**, *b***=4 and ***c***=3**.** Finally, figure 8E shows the scheme of metabolism of 2-hydroxy-eicosapentaenoic acid (EPA-H), which is a compound of formula (I) in which ***a***=2**, *b***=5 and ***c***=0**,** to produce, after undergoing a decarboxylation, (4Z,7Z,10Z,13Z,16Z)-nonadeca-4,7,10,13,16-pentaenoic acid (NPA, C19:5 ω-3), which is a compound of formula (II) in which ***a***=2**, *b***=5 and ***c***=0**.**

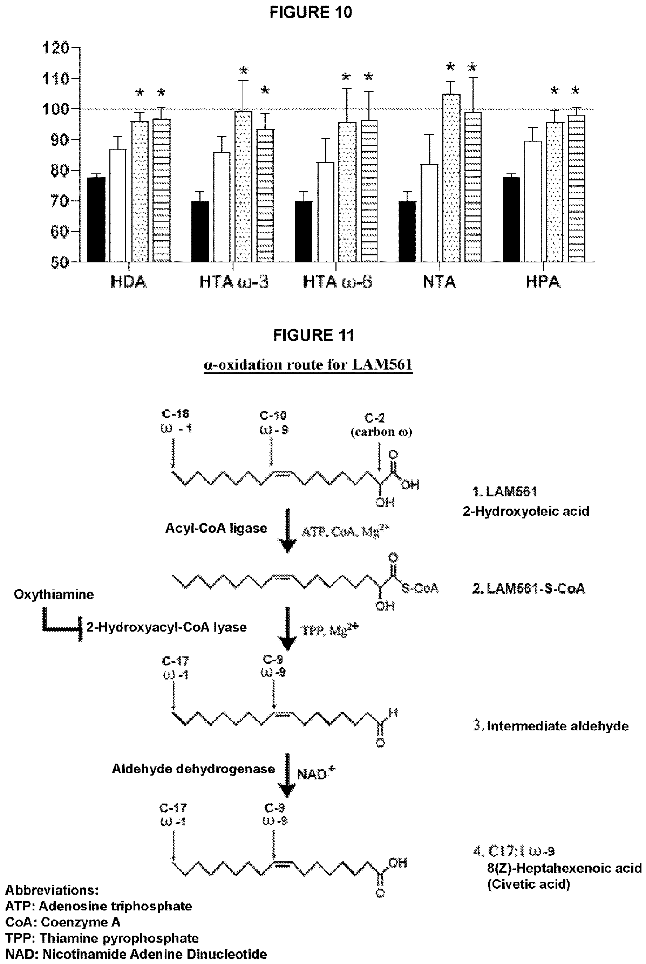

On the other hand, according to the metabolism scheme of figure 11, the compounds of formula (I) can also be monounsaturated alpha-hydroxylated fatty acids with an even number of carbon atoms. According to this route, the prodrug sodium salt of 2-hydroxyoleic acid (2OHOA) is a 2-hydroxylated monounsaturated fatty acid, which is converted to 8Z-heptadecenoic acid (8Z-heptadecenoic or C17:1n-9), by a sequence of metabolic steps: (1) activation by an Acyl-CoA ligase, in a process dependent on ATP (adenosine triphosphate) and magnesium (Mg²⁺); (2) 2OHOA-CoA would be subject to the activity of 2-hydroxyfitanoyl-CoA lyase (2-hydroxyacyl-CoA lyase 1, HACL1), forming an intermediate monounsaturated aldehyde; (3) the aldehyde dehydrogenase enzyme would be responsible for the conversion of said intermediate aldehyde to 8Z-heptadecenoic in a process dependent on NAD⁺ (Nicotinamide Adenine Dinucleotide). Thus, 8Z-heptadecenoic acid, according to the invention, is a compound of formula (II), wherein ***a***=6**, *b***=1 and ***c***=6**,** which is an omega-9 monounsaturated alpha-hydroxylated fatty acid of 17 carbon atoms, resulting from the metabolization of 2-hydroxyoleic acid, or a pharmaceutically acceptable salt or ester thereof, which is a prodrug of formula (I).

Although the medical uses of compounds of formula (I) are known, the present invention describes the formula of their metabolites, compounds of formula (II) or formula (III) that provide a specific and differentiated therapeutic action, in the body, after the metabolism of said compounds of formula (I), which therefore act as prodrugs thereof. Thus, the present invention provides a way of adapting a therapeutic treatment depending on the nature of the disease and prognosis of the patient to be treated.

Specifically, the present invention discloses concrete formulas of metabolites of alpha-hydroxylated, mono- or poly-unsaturated fatty acids which are therapeutically effective. Thus, the present invention further describes the uses as a medicament of said metabolites of formula (II) or formula (III), alone, allowing to control the amount administered; or its use as a medicament in combination with its prodrug of formula (I); or its use as a medicament by administering said prodrug of formula (I), allowing to regulate the intensity and dose administered over time during a treatment. Furthermore, the administration of the compounds of formula (II) or formula (III), by means of the administration of its prodrug of formula (I), thus makes it possible to modulate the distribution and absorption of a drug, since its metabolism makes it possible to generate the corresponding drug only in those cells or tissues in which the metabolic reactions that transform said prodrug occur, the active compound being obtained in said cells. In any case, this specification relates to both the compounds of formula (II) and the compounds of formula (III), whether obtained by chemical synthesis (see example 1), or obtained during the metabolism of the compounds of formula (I).

In this regard, for the purposes of the present invention, the term "metabolites" is used to designate such compounds of formula (II) or formula (III), whether their origin is the metabolism of a compound of formula (I) in the body of a subject, or whether such compounds of formula (II) or formula (III) are synthetically obtained products. Thus, for the purposes of the present invention the term compound of formula (I) is used interchangeably with the term "prodrug of formula (I)" and, likewise, the terms "compound of formula (II)" and "compound of formula (III)" are used interchangeably, respectively, with the terms "metabolite of formula (II)" and "metabolite of formula (III)", because, said compounds of formula (II) and formula (III), whether obtained by chemical synthesis or resulting from the natural metabolism of a compound of formula (I), have the chemical structure or chemical formula of a metabolite of the compound of formula (I) which consequently acts as a prodrug thereof.

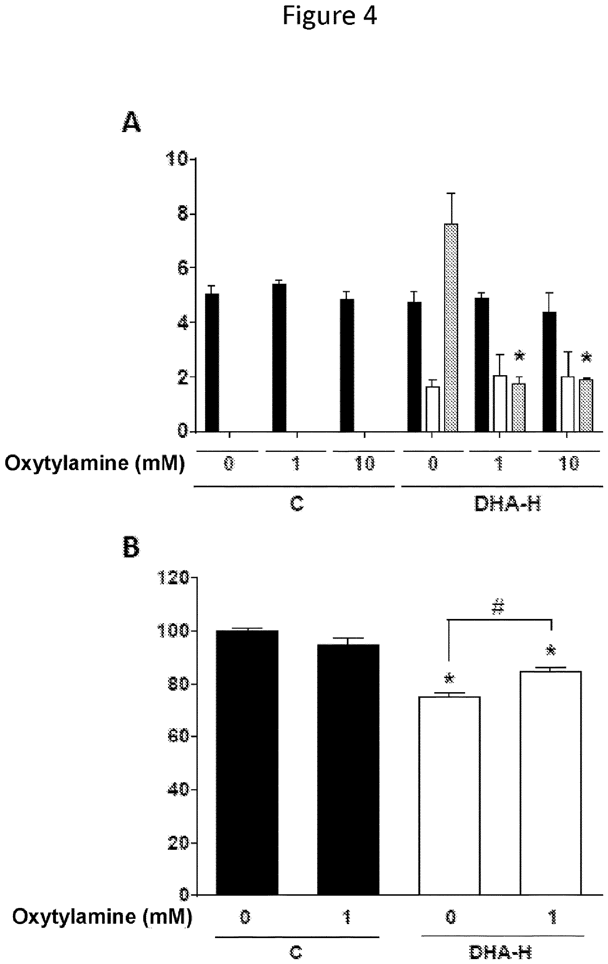

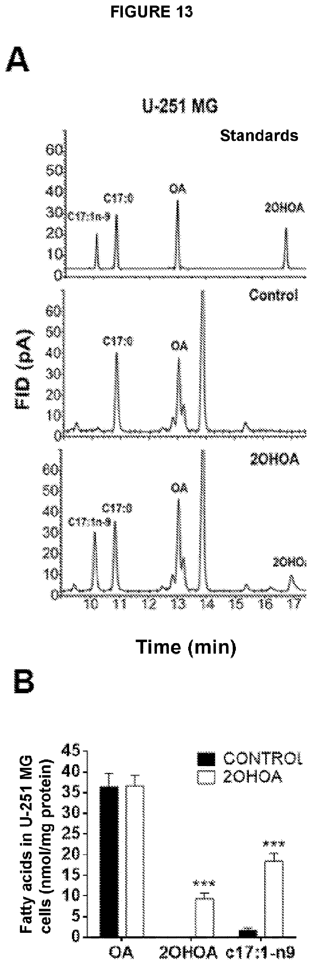

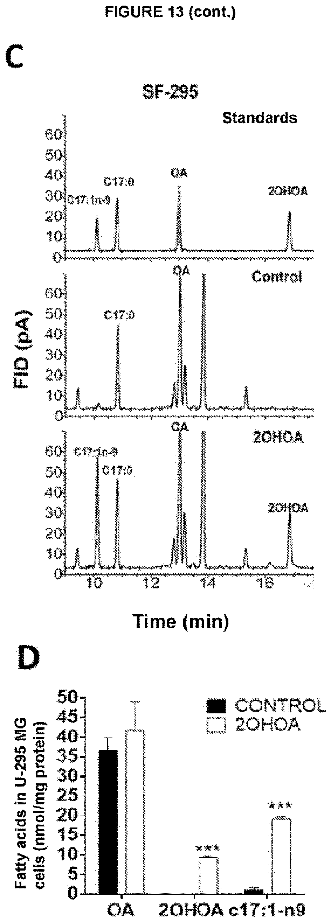

To illustrate the invention, the examples of the present invention show how the therapeutic effect exerted by the prodrugs of formula (I) is directly related to the therapeutic effect exerted by the metabolite of formula (II) or formula (III) on the body, and also show the therapeutic effect exerted, *per se,* by the compounds of formula (II) and formula (III). Thus, as shown in example 7.3, administration of the sodium salt of 2-hydroxyoleic acid (OHOA), compound of formula (I), produced a greater reduction in the size of xenographic tumors in mice the greater the cellular accumulation of the metabolite C17:1n-9 of formula (II). Thus, the therapeutic action of the sodium salt of 2OHOA is in part related to its conversion to the metabolite C17:1n-9. On the other hand, example 6.2 of the present invention demonstrates that the formation of the metabolite C17:1n-9 (8Z-heptadecenoic acid), from the incorporation of 2OHOA, differs between tumor and non-tumor cells. Glioma cells showed a significant increase in their C17:1n-9 levels versus 2OHOA (figure 13B and D), while, in non-tumor cells, the detected levels of 2OHOA were significantly higher than those of its metabolite C17:1n-9. Furthermore, the examples and figures show that the anti-proliferative effect mediated by the compounds of formula (I), exemplified by DHA-H, is mediated, at least in part, by its HPA metabolite (compound of formula (III)), since the inhibition of the formation of this compound from DHA-H results in a lower anti-proliferative effect of DHA-H (figure 4B). These results show that the therapeutic value of the compounds of formula (I) is linked in part to the biological activity of its metabolite of formula (II) or of formula (III), said compound of formula (I) acting as a true prodrug of the compound of formula (II).

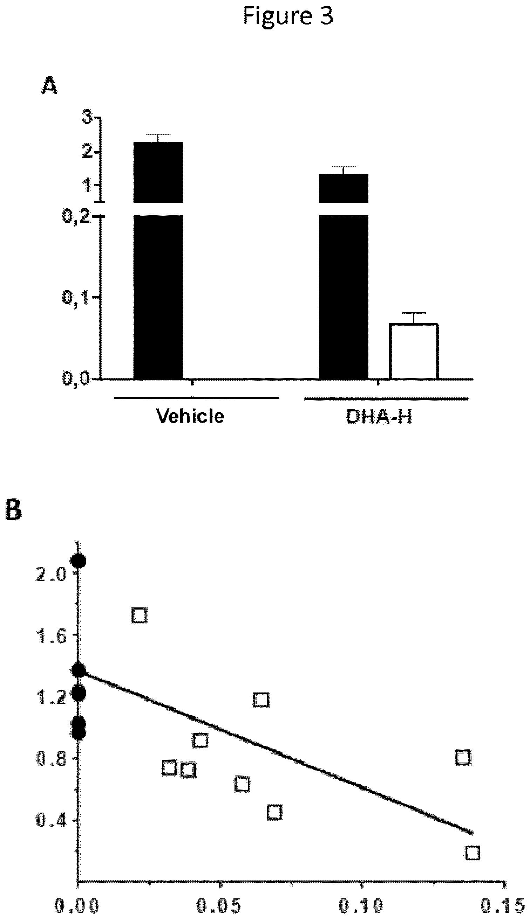

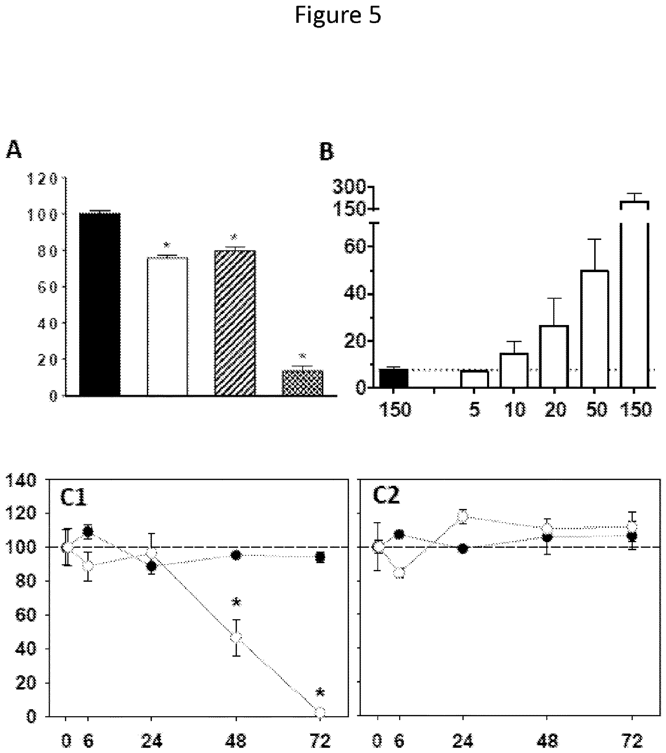

On the other hand, administration of a compound of formula (II) or of formula (III), such as HPA, under the same experimental conditions as administration of a compound of formula (I), such as DHA-H, results in HPA levels an order of magnitude higher than those originating from DHA-H, which would imply that the therapeutic activity of HPA could be higher than that of DHA-H. This effect is due to the structural differences between the prodrug of formula (I) (DHA-H) and its metabolite of formula (III) (HPA). In fact, in the present invention it is demonstrated that the uptake of the alpha-hydroxylated form of DHA (DHA-H) is prevented compared to that of the non-hydroxylated analogue (figure 5C). If DHA-H uptake is prevented compared to non-hydroxylated fatty acids, the different intracellular levels of HPA observed following administration of DHA-H and HPA must be due to the different uptake of these compounds by the cells from the extracellular medium. In addition, as shown in example 6.3, IC₅₀ values of the metabolite C17:1n-9 were lower than those of its prodrug 2OHOA, confirming its greater antiproliferative potency. Additionally, as shown in figure 3B, HPA levels in a tumor are inversely correlated with tumor size in xenographic models. Thus, the therapeutic action of the compound of formula (I), prodrug, is enhanced by the increased presence of the metabolite.

On the other hand, as shown in figure 5, treatment with the sodium salt of HPA (metabolite obtained by chemical synthesis, as described in Example 1) induces a much more evident degree of mortality on the culture than that induced by treatment with the sodium salt of DHA-H (prodrug) or the sodium salt of DHA (natural analogue), under the same conditions. Thus, the administration of the metabolite allows to provide a more accentuated therapeutic action than that obtained by the administration of the prodrug.

However, as shown in example 6.3, C17:1n-9 had an antiproliferative effect by being administered directly in place of its prodrug, both in tumor cells and in non-tumor cells, whereby the administration of said metabolite of formula (II), C17:1n-9, through its prodrug of formula (I), 2OHOA, provides a selective way of producing a therapeutic effect, allowing a longer administration of said therapy without producing undesirable adverse effects and being equally useful in maintenance therapy. Specifically, odd-chain fatty acids are metabolized by β-oxidation, resulting in propionyl-CoA. Unlike even chain fatty acids, whose metabolism ends in the production of acetyl-CoA which, in turn, is metabolized via the Krebs cycle. Propionyl-CoA can be transformed into propionic acid, which causes, as an adverse effect, metabolic acidosis if it accumulates. Propionyl-CoA can be metabolically transformed into succinyl-CoA (which is metabolized via the Krebs cycle), in a biotin and vitamin B12 dependent process. This process is not a usual metabolic pathway of fatty acids, because in the body of mammals, the vast majority of fatty acids are of even chain. Accordingly, this metabolic pathway selectively affects the odd-chain polyunsaturated fatty acids, such as the metabolites of formula (II) and formula (III), and may be saturated in the event that there are too high intracellular concentrations of such odd-chain metabolites or specific pathological situations that result in biotin or vitamin B12 deficiency, leading to the aforementioned adverse effect of propionic acidosis.

Thus, the metabolite administered directly to the cells has a toxin that in some cases is undesirable. This toxicity can be modulated when the prodrug or compound of formula (I) is administered, can regulate the effect and the toxicity of the metabolite of formula (II) or (III). In this regard, slower uptake of the prodrug compared to non-hydroxylated fatty acids could be useful in avoiding excessively high intracellular metabolite concentrations and, consequently, a possible accumulation of propionic acid. Thus, depending on the metabolic condition, the regimen of administration and the pathology to be treated, and in particular in those cases where a more intense therapeutic action is desired, or in treatments of reduced duration, it may be desirable to use a metabolite of formula (II) or of formula (III), or a pharmaceutically acceptable salt or ester thereof; while in other cases, such as in long-term treatments, or maintenance treatments, it may be desirable to use a time-controlled administration, by using the prodrug of formula (I), or a pharmaceutically acceptable salt or ester thereof, such as the sodium salt of DHA-H. Thus, the administration of the metabolites of formula (II) or of formula (III) by using the compounds of formula (I), of even chain, as prodrugs, allows a time-regulated administration of their metabolites of formula (II) or of formula (III), which have an odd chain.

For all these reasons, the present invention shows how the administration of the compounds of formula (II) or of formula (III) by means of the administration of their prodrugs of formula (I), allows a time-regulated administration of their odd-chain metabolites.

Thus, the present invention makes it possible to adapt the therapy, depending on the nature of the disease and prognosis of the patient to be treated, to use the metabolite, or the compound of formula (I), i.e., the prodrug, as a medicament. On the one hand, in cases where short-term acute therapeutic activity is required, the use of the metabolite would be more appropriate or prioritized, in order to obtain a rapid and significant effect. On the other hand, when long-term treatment is required, in chronic diseases, for example, or if maintenance treatment is required, the use of the prodrug, or compositions that combine prodrug and metabolite in different ratios, may be recommended or prioritized, depending on the time and situation/severity of the disease.

Thus, in general, the use of the compounds of formula (II) and (III), and their pharmaceutically or nutraceutically acceptable salts, is beneficial to the organism as shown in the examples of the present application. The action of these compounds of formula (II) and (III), by administering the corresponding prodrug of formula (I), makes it possible to avoid adverse effects derived from their metabolism and accumulation, when a prolonged or high dose administration is required, when administering said compounds of formula (II) and (III) in a controlled manner, as a result of its metabolism. In this way, the prodrugs having formula (I) provide a way of administering the metabolites of formula (II) or of formula (III) with a lower risk of occurrence of adverse side effects and providing a therapeutically effective amount of said metabolites in a sustained manner over time, as the prodrug having formula (I), once administered, is metabolized. Thus, depending on the metabolic condition, the regimen of administration and the pathology to be treated, it may be desirable to use directly a compound of formula (II) or formula (III), or a pharmaceutically acceptable salt or ester thereof (metabolites), or it may be desirable to use a time-controlled administration, by using the prodrug of formula (I), or a pharmaceutically acceptable salt or ester thereof (prodrug), or a combination thereof (prodrug + metabolites).

Thus, one aspect of the invention relates to a compound selected from the group consisting of a compound of formula (II), or a pharmaceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically acceptable salt or ester thereof:

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III);

wherein ***a*** is an integer between 1 and 14; ***b*** is an integer between 1 and 7; ***c*** is an integer between 0 and 14; ***m*** is an integer between 0 and (***b*** - 1); and wherein ***a***+3***b***+***c***+3 is an even integer, for use as a medicament, and in particular for use in the induction of neuroregeneration and in the prevention and/or treatment (including maintenance treatment) of a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

For the purposes of the present invention the term "maintenance treatment" or "maintenance therapy" is defined as a therapeutic treatment administered as a complement to a primary or primary treatment or therapy, for the purpose of either preventing or delaying the recurrence of the disease, which has been completely or partially relieved after treatment with a primary treatment or therapy, or to slow the development of a disease after the end of treatment with a primary therapy.

Preferably, the present invention relates to a compound of formula (II) or a compound of formula (III), or a pharmaceutically acceptable salt or ester thereof, for use in a treatment or therapy of maintenance of a disease or pathology, and more preferably in a treatment or maintenance therapy of cancer.

An embodiment of the invention therefore relates to the use of a compound selected from the group consisting of a compound of formula (II), or a pharmaceutically acceptable salt or ester thereof, and a compound of formula (III), or a pharmaceutically acceptable salt or ester thereof, as described herein, in the manufacture of a medicament for the induction of neuroregeneration or for the prevention and/or treatment (including maintenance treatment) of a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

Another embodiment of the present invention relates to a method of preventing and/or treating a disease or pathology, or to a method of inducing neuroregeneration in a patient, wherein said method comprises administering to said patient an effective amount of a compound selected from the group consisting of a compound of formula (II), or a pharmaceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically acceptable salt or ester thereof:

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III);

wherein ***a*** is an integer between 1 and 14; ***b*** is an integer between 1 and 7; ***c*** is an integer between 0 and 14; ***m*** is an integer between 0 and (***b*** - 1); and wherein ***a***+3***b***+***c***+3 is an even integer.

For the purposes of the present invention, effective amount or therapeutically effective amount shall be understood as an amount that provides a therapeutic effect without causing unacceptable toxic effects on the patient. The effective amount or dose of the medicament depends on the compound and the condition or disease treated, and for example the age, weight and clinical condition of the treated patient, the form of administration, the clinical history of the patient, the seriousness of the disease and the potency of the compound administered. Additionally, one embodiment of the invention relates to a compound selected from the group consisting of a compound of formula (II):

COOH -(CH₂)***ₐ***-(CH=CH-CH₂)***_{b}***-(CH₂)***_{c}***-CH₃ (II)

and a compound of formula (III):

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III);

for use in preventing and/or treating a disease or pathology or in inducing neuroregeneration, wherein the prevention and/or treatment or the induction of neuroregeneration is characterized by administering a compound or prodrug of formula (I), or a pharmaceutically acceptable salt or ester thereof:

COOH -CHOH-(CH₂)***ₐ***-(CH=CH-CH₂)***_{b}***-(CH₂)***_{c}***-CH₃ (I)

and wherein said compound of formula (I) is metabolized to produce a therapeutically effective amount of: a compound of formula (II):

COOH -(CH₂)***ₐ***-(CH=CH-CH₂)***_{b}***-(CH₂)***_{c}***-CH₃ (II)

or a compound of formula (III):

COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III);

and wherein ***a*** is an integer between 1 and 14; ***b*** is an integer between 1 and 7; ***c*** is an integer between 0 and 14; ***m*** is an integer between 0 and (***b*** - 1); and wherein ***a***+3***b***+**c**+3 is an even integer.

Preferably, said disease is selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

Thus, another aspect of the present invention relates to a method of administering an effective amount of a compound of formula (II), or of a compound of formula (III), as described herein, for the prevention and/or treatment of a disease or pathology, or for the induction of neuroregeneration and/or prevention of neurodegeneration, wherein said compound of formula (II) or said compound of formula (III), is administered as a prodrug of formula (I), or as a pharmaceutically acceptable salt or ester thereof, as described herein.

The invention further relates to a method of preventing and/or treating a disease or pathology; wherein said method comprises administering an effective amount of a prodrug of a compound of formula (II), or a pharmaceutically acceptable salt or ester thereof; or a prodrug of a compound of formula (III), or a pharmaceutically acceptable salt or ester thereof, as described herein; wherein said prodrug of compounds of formula (II) or formula (III) has formula (I), or a pharmaceutically acceptable salt or ester thereof, as described herein.

Additionally, the present invention relates to a method of preventing and/or treating a disease or pathology, or inducing neuroregeneration and/or preventing neurodegeneration, wherein said method comprises administering, to a patient in need thereof, an effective amount of a compound of formula (I), or a pharmaceutically acceptable salt or ester thereof:

COOH -CHOH-(CH₂)***ₐ***-(CH=CH-CH₂)***_{b}***-(CH₂)***_{c}***-CH₃ (I)

wherein the compound of formula (I) is metabolized in the body of said patient to produce a therapeutically effective amount of a metabolite:
- having formula (II):

   COOH -(CH₂)***ₐ***-(CH=CH-CH₂)***_{b}***-(CH₂)***_{c}***-CH₃ (II)

   or
- having formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III);
wherein ***a*** is an integer between 1 and 14; **b** is an integer between 1 and 7; ***c*** is an integer between 0 and 14; ***m*** is an integer between 0 and **(*b* -** 1); and ***a***+3***b***+***c***+3 is an even integer, wherein said metabolite is responsible for the prevention and/or treatment of said disease or pathology and for the induction of neuroregeneration and/or prevention of neurodegeneration in the patient. Preferably, upon administration of an effective amount of a compound of formula (I), the metabolite having formula (II), or having formula (III), is present in the body of said patient.

In a preferred embodiment, said compound of formula (I) is metabolized by more than 1%, 10%, more than 40%, more than 50%, and up to 99% into a metabolite of formula (II) or formula (III) upon administration.

Another embodiment relates to a method of preventing and/or treating a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease, and for inducing neuroregeneration and/or preventing neurodegeneration; wherein said method comprises administering to a patient an effective amount of a prodrug having the structure of formula (I), or a pharmaceutically acceptable salt or ester thereof:

COOH -CHOH-(CH₂)***ₐ***-(CH=CH-CH₂)***_{b}***-(CH₂)***_{c}***-CH₃ (I)

wherein said prodrug is converted *in vivo* to release an active compound into cells of said patient; wherein said active compound has a structure:
- of formula (II):

   COOH -(CH₂)***ₐ***-(CH=CH-CH₂)***_{b}***-(CH₂)***_{c}***-CH₃ (II)

   or
- of formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)
wherein ***a*** is an integer between 1 and 14; ***b*** is an integer between 1 and 7; ***c*** is an integer between 0 and 14; ***m*** is an integer between 0 and **(*b* -** 1); and ***a***+3***b***+***c***+3 is an even integer.

Preferably said conversion is a chemical or physiological process. For purposes of the present invention the term "chemical process" refers to the conversion of the prodrug *in vivo* to release the active compound by a chemical reaction, wherein the prodrug is a reagent or substrate of the chemical reaction, and the active compound is a reaction product. Furthermore, for the purposes of the present invention the term "physiological process" refers to a conversion due to an event or process that occurs in an organism naturally, for example, due to the activity of enzymes.

Furthermore, although the compounds of formula (I) act therapeutically via their metabolites of formula (II), or of formula (III), said compounds of formula (I) also show biological activity independent of said metabolic pathway, as shown in example 6.4 of the present invention.

Thus, another embodiment of the invention relates to a compound selected from the group consisting of a compound of formula (II) and a compound of formula (III), or to a pharmaceutically acceptable salt or ester thereof, as described herein, for use as a medicament and, in particular, for use in inducing neuroregeneration and/or preventing neurodegeneration and/or for use in preventing and/or treating a disease or pathology, according to the present invention, characterized in that said compound is administered before, after or in conjunction with a compound of formula (I), or with a pharmaceutically acceptable salt or ester thereof:

COOH -CHOH-(CH₂)***ₐ***-(CH=CH-CH₂)***_{b}***-(CH₂)***_{c}***-CH₃ (I)

wherein ***a*** is an integer between 1 and 14; ***b*** is an integer between 1 and 7; ***c*** is an integer between 0 and 14 and ***a***+3***b*** +***c***+3 is an even integer; and wherein said values of a, ***b*** and ***c*** are equal to or different from the values of ***a, b*** and ***c*** of the compound of formula (II) or the compound of formula (III).

Additionally, the present invention relates to a method of inducing neuroregeneration and/or preventing neurodegeneration, or to a method of preventing and/or treating a disease or pathology, comprising administering an effective amount of a compound of formula (II), or a compound of formula (III), or a pharmaceutically acceptable salt or ester thereof, to a patient and, wherein said method is characterized in that it comprises further administering a compound of formula (I), or a pharmaceutically acceptable salt or ester thereof, as described herein; and wherein said compound of formula (I) is administered before, after or in conjunction with said compound of formula (II) or formula (III).

For purposes of the present invention, the term "prodrug" refers to a compound that upon administration to a subject is transformed, by a metabolic process, into a second therapeutically active compound.

On the other hand, the term "subject" refers, for the purposes of the present invention, to a human or an animal.

The term "pharmaceutically acceptable" refers, for purposes of the present invention, to that compound or substance authorized or authorized by a regulatory agency of the federal government or a state government or listed in the European, U.S., or other generally recognized pharmacopoeia for use in animals or humans. Throughout this specification, said term applies primarily to the salts and esters of the compounds of formulae (I), (II), and (III), which are defined according to the present disclosure.

Thus, the term "pharmaceutically acceptable salt" refers to a salt of a compound that also possesses the desired pharmacological activity of the parent compound from which it is derived. Preferably, the pharmaceutically acceptable salt is the sodium salt.

For purposes of the present invention, the term "ester" refers to any compound in which the hydroxyl group belonging to a carboxylic acid moiety has been replaced by an alkoxide group. In a preferred embodiment of the invention, the ester is a methyl or ethyl ester. More preferably, the ester is an ethyl ester.

The term "nutraceutically acceptable" refers, for the purposes of the present invention, to everything that is of use in nutraceutical products. Thus, for the purposes of the present invention the term "nutraceutical" or "nutraceutical composition" refers to a dietary supplement, to be taken alone, or in combination with other foods and which produces a beneficial effect for the health of the subject who ingests it, especially in the prevention of diseases. Throughout this specification, said term applies primarily to the salts and esters of the compounds of formulae (I), (II), and (III), which are defined according to the present disclosure.

For the purposes of the present invention, the term "stereoisomer" refers to those compounds that have the same chemical formula and the same sequence of atoms, but have a different three-dimensional orientation in space, and includes the stereoisomers R and S (which also use the nomenclature (+) and (-)) resulting from the presence of a chiral carbon, as well as the stereoisomers E and Z (which also use the cis/trans nomenclature) resulting from the arrangement of the substituents of the carbons that constitute a double bond. Thus, since the prodrugs of formula (I) comprise a chiral carbon (alpha carbon to the carboxylic group), the invention also includes the two stereoisomers R and S, as well as any mixture of both, with respect to the configuration of said chiral carbon. On the other hand, since both the prodrugs of formula (I) and their metabolites of formula (II), or (III), comprise C=C double bonds, the invention also includes all of the E and Z stereoisomers for each of their double bonds. In a preferred embodiment, all the double bonds of the prodrug of formula (I), of the compound of formula (II), and of the compound of formula (III), have an all-cis configuration. Thus, if the prodrug of formula (I) has a cis/trans (or E/Z) stereochemical configuration determined from its double bonds, the metabolite of formula (II) or formula (III) will also have such a configuration for the double bonds it contains.

For purposes of the present invention, the term "comprises" indicates that it includes a group of certain features (e.g., a group of features A, B, and C) and is interpreted to mean that it includes those features (A, B, and C), but does not exclude the presence of other features (e.g., features D or E), provided that they do not render the claim impracticable. Additionally, the terms "contains", "includes", "has" or "encompasses", and the plural forms thereof, should be taken as synonyms of the term "comprises" for the purposes of the present invention. On the other hand, if the term "consists of" is used, then no additional features are present in the apparatus/method/product other than those following said term. In this regard, for the purposes of the present invention, the term "comprises" may be replaced by any of the terms "consists of", or "consists essentially of". Accordingly, "comprises" may refer to a group of features A, B, and C, which may further include other features, such as E and D, provided that such features do not render the claim impracticable, but such term "comprises" also includes the situation in which the group of features "consists of" or "consists essentially" of A, B, and C.

Furthermore, the present invention makes it possible to support an administration of a compound of formula (I), in particular of 2OHOA, or a pharmaceutically acceptable salt or ester thereof, more preferably the sodium salt of 2OHOA, in a maintenance treatment (maintenance therapy), wherein said compound of formula (I), or a pharmaceutically acceptable salt or ester thereof, is administered at different intervals over a period of time, the cumulative concentration of its metabolite of formula (II), or of formula (III), being a measure of the effectiveness of the treatment. Thus, said *in vitro* method of determining the efficacy of a treatment with a compound of formula (I), or with a pharmaceutically acceptable salt or ester thereof, comprises determining the amount of a compound of formula (II), or of formula (III), or of its carboxylate anion, or of a derivative formed therefrom.

In this regard, for purposes of the present invention the term "biomarker" refers to a first compound or substance, or a derivative of said first compound or substance, which can be used to determine the response and/or efficacy of a treatment with a second compound or substance. Thus, for purposes of the present invention, the metabolites of formula (II), or of formula (III), can be used as biomarkers for determining the response and/or efficacy of a treatment with a compound of formula (I).

Thus, another aspect of the invention relates to an *in vitro* method for determining the efficacy of a therapeutic or preventive treatment of a disease or pathology, or of a neuroregeneration induction treatment, with a compound of formula (I), or with a pharmaceutically acceptable salt or ester thereof:

COOH -CHOH-(CH₂)***ₐ***-(CH=CH-CH₂)***_{b}***-(CH₂)***_{c}***-CH₃ (I)

in a subject, wherein said method comprises determining *in vitro in* a biological sample of said subject, the amount of a compound:
- of formula (II):

   COOH -(CH₂)***ₐ***-(CH=CH-CH₂)***_{b}***-(CH₂)***_{c}***-CH₃ (II)

   or
- of formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)
or of its carboxylate anion, or of a derivative formed therefrom *in vivo or in vitro,* wherein said amount is related to the efficacy of the treatment; and wherein ***a*** is an integer between 1 and 14; ***b*** is an integer between 1 and 7; ***c*** is an integer between 0 and 14; ***m*** is an integer between 0 and **(*b* -** 1); and wherein ***a***+3***b***+***c***+3 is an even integer.

Said method therefore comprises determining the amount of a compound of formula (II), or of formula (III), of their respective carboxylate anions, or of a derivative formed therefrom.

Said derivative of the compound of formula (II), or (III), may be formed *in vitro* by reacting said compound of formula (II) or (III), comprised in the *in vitro* sample, with a substance to obtain a derivative thereof. In this case, the method of the invention comprises determining the amount of said derivative of formula (II), or of formula (III), formed *in vitro.* For example, some techniques for the detection of fatty acids require their prior chemical modification and thus, it is customary for detection by gas chromatography to require that the fatty acid sample (in this case a compound of formula (II), or of formula (III)) be transformed into its respective methyl ester for detection and quantification.

On the other hand, said derivative of the compound of formula (II), or of formula (III), can be a metabolic derivative or a derivative formed *in vivo* (result of a reaction occurring *in vivo*)*,* formed as a result of the reaction of said compound of formula (II), or of formula (III), with another lipid, protein, enzyme, nucleotide, carbohydrate, etc. Thus, said derivative can be an ester of said compound of formula (II), or of formula (III), such as for example, a glycerophospholipid (such as phosphatidylcholine, phosphatidylethanolamine, phosphatatidylserine, phosphatidylinositol, phosphatidylglycerol, phosphatidic acid or any of its smooth forms, such as lysophosphatidylcholine, Iysophosphatidylethanolamine, etc.), a plasmalogen (alkyl or alkenyl), a cholesterol ester, a glycerolipid such as triacylglyceride (triglyceride) or diglycerolglycerol, a cardiolipin, a sphyngolipid, a thioester with coenzyme A (acyl-CoA), or an acylcarnitine, inter alia. In this case, the method of the invention comprises determining in vitro the amount of said metabolic derivative (or derivative formed *in vivo*) in the biological sample.

Thus, the amount of said compound of formula (II), or of formula (III), or of their respective carboxylate anions, or of a derivative formed therefrom *in vivo* or in *vitro,* is related to the efficacy of the treatment and/or prevention of a disease or pathology, or to a neuroregeneration induction treatment, in a subject with the compound of formula (I), wherein the levels of said compound of formula (II), or of formula (III), or of its carboxylate anion, or of its derivative, compared to a control group, are related to the efficacy of the therapeutic or preventive treatment of a disease or pathology, with said compound of formula (I), or with a pharmaceutically acceptable salt, or with an ester thereof.

Another aspect of the invention relates to the use of a compound:
- of formula (II):

   COOH -(CH₂)***ₐ***-(CH=CH-CH₂)***_{b}***-(CH₂)***_{c}***-CH₃ (II)

   or
- of formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

   or of the respective carboxylate anion, or of a derivative formed therefrom *in vivo or in vitro,* for determining *in vitro* the efficacy of a therapeutic or preventive treatment of a disease or pathology, or of a neuroregenerative induction treatment, with a compound of formula (I), or with a pharmaceutically acceptable salt, or with an ester thereof:

   COOH -CHOH-(CH₂)***ₐ***-(CH=CH-CH₂)***_{b}***-(CH₂)***_{c}***-CH₃ (I)
in a subject, wherein ***a*** is an integer between 1 and 14; ***b*** is an integer between 1 and 7; ***c*** is an integer between 0 and 14; ***m*** is an integer between 0 and (***b*** - 1); and wherein ***a***+3***b***+***c***+3 is an even integer.

More preferably, the disease or pathology is selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease. Still more preferably, the disease or pathology is selected from a neurological or neurodegenerative disease; a cancer; an inflammatory disease; and a metabolic disease.

In a more preferred embodiment, the method determines the efficacy of a treatment with a pharmaceutically acceptable salt of the compound of formula (I) and even more preferably with the sodium salt of the compound of formula (I).

In an embodiment of the invention, the biological sample is a blood sample (including plasma or serum), a urine sample, a saliva sample, a biopsy of a tissue, cerebrospinal fluid, or a sweat sample.

The present invention also relates to a pharmaceutical composition comprising at least a first compound selected from the group consisting of:
- a compound of formula (II), or a pharmaceutically acceptable salt or ester thereof:

   COOH-(CH₂)***ₐ***-(CH=CH-CH₂)***_{b}***-(CH₂)***_{c}***-CH₃ (II)

   and
- a compound of formula (III), or a pharmaceutically acceptable salt or ester thereof:

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

   wherein said composition optionally comprises a second compound of formula (I), or a pharmaceutically acceptable salt or ester thereof:

   COOH -CHOH-(CH₂)***ₐ***-(CH=CH-CH₂)***_{b}***-(CH₂)***_{c}***-CH₃ (I)

   and wherein ***a*** is an integer between 1 and 14; **b** is an integer between 1 and 7; ***c*** is an integer between 0 and 14; ***m*** is an integer between 0 and **(*b* -** 1); and ***a***+3***b*+*c***+3 is an even integer; and at least one pharmaceutically acceptable excipient.

The present invention also relates to a pharmaceutical composition comprising at least a first compound selected from the group consisting of:
- a pharmaceutically acceptable salt or ester of a compound of formula (II):

   COOH-(CH₂)***ₐ***-(CH=CH-CH₂)***_{b}***-(CH₂)***_{c}***-CH₃ (II)

   and
- a pharmaceutically acceptable salt or ester of a compound of formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

   wherein said composition optionally comprises a pharmaceutically acceptable salt or ester of a compound of formula (I):

   COOH -CHOH-(CH₂)***ₐ***-(CH=CH-CH₂)***_{b}***-(CH₂)***_{c}***-CH₃ (I)
and wherein ***a*** is an integer between 1 and 14; ***b*** is an integer between 1 and 7; ***c*** is an integer between 0 and 14; ***m*** is an integer between 0 and **(*b* -** 1); and ***a***+3***b***+**c**+3 is an even integer; and at least one pharmaceutically acceptable excipient.

Another embodiment of the invention relates to a pharmaceutical composition comprising at least a first compound selected from the group consisting of a compound of formula (II) and a compound of formula (III), wherein said composition optionally comprises a pharmaceutically acceptable salt or ester of a compound of formula (I), as described above; and at least one pharmaceutically acceptable excipient; for use as a medicament; and in particular, for use in inducing neuroregeneration and/or preventing neurodegeneration, and for use in preventing and/or treating a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

In a preferred embodiment of the invention, said at least first compound is a pharmaceutically acceptable salt or ester of a compound of formula (II), or of a compound of formula (III), and/or said second compound is a pharmaceutically acceptable salt or ester of a compound of formula (I).

One embodiment of the invention relates to the use of a pharmaceutical composition comprising at least a first compound selected from the group consisting of a compound of formula (II) and a compound of formula (III), or a pharmaceutically acceptable salt or ester thereof, wherein said composition optionally comprises a compound of formula (I), or a pharmaceutically acceptable salt or ester thereof, as described above; and at least one pharmaceutically acceptable excipient; in the manufacture of a medicament for inducing neuroregeneration and/or preventing neurodegeneration, and/or for preventing and/or treating a disease or pathology.

Another embodiment of the invention relates to a method of preventing and/or treating a disease or pathology, or for inducing neuroregeneration and/or preventing neurodegeneration; wherein said method comprises administering, to a patient in need thereof, an effective amount of a pharmaceutical composition comprising at least a first compound selected from the group consisting of a compound of formula (II) and a compound of formula (III), or a pharmaceutically acceptable salt or ester thereof, wherein said composition comprises, optionally, a compound of formula (I), or a pharmaceutically acceptable salt or ester thereof, as described above; and at least one pharmaceutically acceptable excipient.

Preferably, said disease or pathology is selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

A person skilled in the art may select one or more pharmaceutically acceptable vehicles or excipients known in the art, such that the pharmaceutical compositions are suitable for administration to both a human subject and an animal.

In a preferred embodiment of the invention, said excipient is albumin, for example: ovalbumin, lactalbumin, native or recombinant albumin of human, bovine, murine, or rabbit origin, more preferably, human serum albumin or bovine serum albumin.

The pharmaceutical compositions disclosed in the present invention may also be co-administered, prior to or subsequent to further therapy. Preferably, such additional therapy is radiation therapy, electric fields for the treatment of tumors (Tumor Treatment Fields), immunotherapy or chemotherapy. More preferably, the pharmaceutical compositions disclosed in the present invention may also be co-administered, prior to, or subsequent to, a therapy comprising the administration of temozolomide.

Such administration may be part of the treatment of an adult or a pediatric patient. In a preferred embodiment, said pharmaceutical composition is co-administered, prior to, or subsequent to a radiotherapeutic treatment, a chemotherapeutic treatment, a treatment with electric fields for the treatment of tumors (Tumor Treatment Fields), or an immunotherapeutic treatment.

In an embodiment of the invention, the pharmaceutical compositions disclosed herein comprise at least one additional therapeutic component or active compound. Said additional therapeutic component or active compound provides additive or synergistic biological activities. For purposes of the present disclosure, the terms "active compound" or "therapeutic component" should be taken to mean a chemical or biological entity that exerts therapeutic effects when administered to humans or animals. Such active compound or additional therapeutic component can be a cell therapy, a small molecule therapy, an immunotherapy, radiation therapy, among others.

Among the additional therapeutic components or active compounds are compounds for the treatment of neurodegenerative diseases, anticancer agents, metabolism-regulating compounds, cardiovascular agents, and obesity- and overweight-regulating agents.

Also within the therapeutic components or additional active compounds are compounds for the treatment of neurodegenerative diseases, chemotherapeutic agents, metabolism-regulating compounds, cardiovascular agents, and obesity and overweight-regulating agents. Preferably, said active compound or said therapy is a chemotherapeutic agent, a cell therapy agent or an immunotherapeutic agent.

In a preferred embodiment, said pharmaceutical composition further comprises a chemotherapeutic agent selected from the group consisting of: platinum-based antineoplastic agents; anti-mitotic chemotherapeutic agents; a poly adenosine diphosphate ribose polymerase (PARP) inhibitor; type I topoisomerase inhibitors; type II topoisomerase inhibitors; epothilones; cyclo-skeletal perturbers; alkylating agents; histone deacetylase inhibitors; kinase inhibitors; antifolates; peptide antibiotics; retinoids; vinca alkaloids and thymidylate synthase inhibitors. More preferably, the chemotherapeutic agent is selected from the group consisting of: bevacizumab, carmustine, cyclophosphamide, melphalan, ifosfamide, busulfan, temozolomide, mechlorethamine, chlorambucil, melphalan, dacarbazine, daunorubicin, doxorubicin, epirubicin, idarubicin, mitoxantrone, valubicin, paclitaxel, docetaxel, abraxane, taxotere, epothilone, vorinostat, romidepsin, irinotecan, topotecan, camptothecin, exatecan, lurtotecan, etoposide, teniposide, tafluposide, bortezomib, erlotinib, gefitinib, imatinib, vemurafenib, vismodegib, azacytidine, azathioprine, capecitabine, cytarabine, cladribine, fludarabine, doxifluridine, fluorouracil, gemocytebine, hydroxyurea, mercaptopurine, methotrexate, pemetrexed, azathiopyrene, thioguanine, retinoic acid, bleomycine, actinomicine, carboplatin, cisplatin, oxaliplatin, tretinoine, alitretinoin, bexarotene, topotecan, vinblastin, vincristin, vindesin and vinorelbin. More preferably, the additional chemotherapeutic agent is temozolomide.

In this regard, the fact that the lipids when integrating the cell membrane can control cell signaling, supposes that they can also regulate the physiological state of the cells and, therefore, the general state of health. Thus, the compounds disclosed herein are useful in inducing neuroregeneration and in preventing and/or treating different diseases and pathologies, particularly selected from the group consisting of a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

For purposes of the present invention, the diseases of the nervous system are all those diseases that affect the nervous system (both central and peripheral). Within this group are neurodegenerative diseases which, for the purposes of the present invention, are a heterogeneous group of disorders characterized by the progressive degeneration of the structure and function of the central nervous system or the peripheral nervous system.

Preferably, the neurodegenerative disease is selected from the group consisting of spinal cord injury and pain of neurological origin. For the purposes of the present invention, the term "induction of neuroregeneration" refers to the regeneration of neurological functions. On the other hand, for the purposes of the present invention the term "prevention of neurodegeneration" indicates that the treatment results in the arrest of a neurodegenerative process already in progress, or that the treatment prevents the onset or progression of neurodegeneration.

Some of these neurodegenerative processes involve a significant decrease in the patients' cognitive capacity or motor impairment. Neurodegenerative processes, neurological disorders and neuropsychiatric disorders have a common basis of neuronal degeneration or alterations of their components, such as lipids (e.g. myelin) or membrane proteins (e.g. adrenergic receptors, serotonergic receptors, etc.).

In particular, the neurodegenerative diseases are selected from the group consisting of: (i) inflammatory diseases of the central nervous system such as bacterial meningitis, nonbacterial meningitis, acute hemorrhagic necrotizing encephalopathy, other encephalitis, myelitis and encephalomyelitis, cerebral ventriculitis not otherwise specified (NOS), intracranial and intrathecal abscess and granuloma, extradural and subdural abscess, phlebitis, intracranial thrombophlebitis in intrathecal and sequelae of inflammatory diseases of the central nervous system; (ii) systemic atrophies affecting mainly the central nervous system such as Guillan-Barre, diabetic neuropathy, Wallerian degeneration, Lewy body dementia, frontotemporal dementia, Huntington's chorea, Huntington's dementia, hereditary ataxia; spinal muscular atrophy and related syndromes such as Werdnig-Hoffman; systemic atrophies affecting mainly the central nervous system, post-polio syndrome; motor neuron diseases such as amyotrophic lateral sclerosis and progressive bulbar palsy; (iii) extrapyramidal and movement disorders such as Parkinson's disease, secondary Parkinsonism, neuroleptic malignant syndrome, drug-induced secondary Parkinsonism post-encephalitic Parkinsonism, vascular Parkinsonism, degenerative diseases of the basal nuclei, Hallervorden-Spatz, progressive supranuclear ophthalmoplegia, progressive supranuclear palsy, striatonigral degeneration, essential tremor dystonia, drug-induced tremor, myclonia, drug-induced chorea, drug-induced tics, tics of organic origin, drug-induced movement disorders, akathisia, restless leg syndrome, stiff man syndrome and benign shivering attacks; (iv) other degenerative diseases of the nervous system such as Alzheimer's disease, early or late onset Alzheimer's disease, frontotemporal dementia such as Pick's disease, nervous system degeneration due to alcohol, Alpers disease, Leigh's disease, Lewy body dementia, mild cognitive impairment, corticobasal degeneration, primary degenerative dementia including Alzheimer's dementia, senile and presenile forms, stroke; (v) demyelinating diseases of the central nervous system such as multiple sclerosis of the medulla, brainstem, disseminated, generalized or not otherwise specified (NOS); acute disseminated demyelinations, diffuse central nervous system sclerosis; (vi) episodic and paroxysmal disorders such as recurrent epilepsy and seizures, idiopathic epilepsy and seizures, grand mal seizures, nonspecific atonic or clonic epilepsy, Lennox-Gastaut syndrome, epileptic spasms, epilepsy of unspecified type, migraine, headache, transient cerebral ischemic accidents and related syndromes, sleep disorders and vertigo; (vii) nerve, nerve root and nerve plexus disorders such as trigeminal nerve disorders, facial nerve disorders, cranial nerve disorders, nerve root and nerve plexus disorders, upper or lower extremity mononeuropathies and Wallerian degeneration; (viii) polyneuropathies and other disorders of the peripheral nervous system including hereditary and idiopathic neuropathy such as Roussy-Levy syndrome, Refsum's disease; inflammatory polyneuropathy; sequelae of polyneuropathy such as Guillan-Barre sequelae, serum neuropathy; other polyneuropathies such as drug, alcoholic, toxic agent, radiation neuropathy; sequelae of inflammatory and toxic polyneuropathies; (ix) muscle and neuromuscular junction diseases such as myasthenia gravis and other myoneural disorders, muscle and neuromuscular junction disorders; (x) cerebral palsy and other paralytic syndromes including hemiplegia, paraplegia, tetraplegia; (xi) other nervous system disorders such as complex regional pain syndrome, neuropathic pain, atonal nervous system disorders, peripheral autonomic neuropathy, hydrocephalus, brain cysts, Riley-Day syndrome, multisystem autonomic nervous system degeneration, hippocampal sclerosis, mesial sclerosis, diabetic neuropathy or Wolfram Syndrome, adrenoleukodystrophy, leukodystrophy, and spinal cord injury; and (xii) mental and behavioral disorders due to physiological conditions such as vascular dementia, unspecified dementia, depression; behavioral disorders related to psychoactive substance abuse; schizophrenia, schizotypal disorder, delusional disorder and other non-mood related psychotic disorders; mood (affective) disorders such as manic episode, bipolar disorder, major depressive disorder, cyclothymic disorder, dysthymic disorder; anxiety disorder, dissociative, stress-related and other non-psychotic somatoform mental disorders; behavioral syndromes associated with physiological disorders and physical factors such as eating disorders, sleep disorders; personality disorder, impulse disorder, pathological gambling; intellectual disability; speech disorders, writing, learning disorder, psychoactive substance use disorder and addictive behaviors.

For the purposes of the present invention "neuropathic pain" is defined as pain caused by injury or disease of the somatosensory nervous system, as defined by the International Association for the Study of Pain (IASP). The somatosensory nervous system comprises sensory neurons and neural pathways that respond to changes on the surface or within the body. The term paralysis refers, for the purposes of the present invention, to the partial or total loss of mobility in some part of the body, caused by injury or disease of the central or peripheral nervous system. On the other hand, the term sleep disorders refers to those disorders that include problems in sleep initiation and maintenance caused by a central or peripheral nervous system problem or pathology. Non-limiting examples of such sleep disorders include insomnia, hypersomnia such as narcolepsy, sleep apnea, restless leg syndrome, circadian rhythm disorders and parasomnia, among others.

Certain neurodegenerative diseases can result in processes in which blindness, hearing problems, disorientation, mood disturbances, etc. are developed. An example of a wellcharacterized neurodegenerative disorder is Alzheimer's disease, in which the formation of plaques has been observed, mainly formed by the β-amyloid peptide that comes from altered protein processing, followed by an accumulation on the outside of cells. In addition, neurofilament tangles of hyperphosphorylated tau protein appear inside the cell. This process has been associated with alterations in cholesterol metabolism and the consequent alteration in the levels of certain membrane lipids, such as docosahexaenoic acid. On the other hand, several neurodegenerative pathologies, such as Parkinson's disease, Alzheimer's disease, senile dementia (or Lewy body dementia), have been related to the pathological accumulation of fibrillar aggregates of the α-synuclein protein, which give rise to an important alteration in the metabolism of cellular triglycerides. Indeed, the development of these and other neurodegenerative diseases is related to alterations in the serum or cellular levels of lipids, such as cholesterol, triglycerides, sphingomyelin, phosphatidylethanolamine, etc. This, again, suggests that lipids play a crucial role in the proper functioning of neurons, glia cells, nerves, brain, cerebellum and spinal cord, which is logical considering the great abundance of lipids in the central nervous system.

Alzheimer's disease (AD) is a neurodegenerative disease that to date does not have an effective therapy or treatment and whose pathophysiology is still, to a large extent, unknown. Multiple drugs and therapies have been designed and developed in the last decade in order to stop or slow down the neurodegenerative process characteristic of this disease. However, no treatment is yet known that has successfully completed a phase III human clinical trial. Most therapies have been inspired by the hypothesis of the amyloid cascade, which is currently in question, due to the almost complete failure of clinical trials of antiamyloid/tau therapies.

On the other hand, various types of sclerosis and other neurodegenerative processes are related to "demyelination", whose net result is the loss of lipids in the cover of neural axons, with the consequent alterations in the process of propagation of electrical signals that this implies. Myelin is a lipid layer that surrounds the axons of many neurons and is formed by a succession of spiral folds of the plasma membrane of glia cells (Schwann cells and oligodendrocytes, at the peripheral and central level, respectively). For all these reasons, it has been shown that lipids play an important role in the development of neurodegenerative diseases. Furthermore, natural polyunsaturated fatty acids have been shown to have a moderate preventive effect on the development of neurodegenerative processes. Indeed, the most abundant lipid in the central nervous system is docosahexaenoic acid (DHA), whose abundance is altered in many neurodegenerative processes, such as Alzheimer's disease.

In addition, metabolic disease is preferably selected from the group consisting of obesity, overweight, hypercholesterolemia, hypertriglyceridemia, diabetes, and insulin resistance. Metabolic diseases form a set of pathologies characterized by the accumulation or deficit of certain molecules. A typical example is the accumulation of glucose, cholesterol and/or triglycerides above normal levels. Increased levels of glucose, cholesterol and/or triglycerides, both at the systemic level (e.g., increased plasma levels) and at the cellular level (e.g., in cell membranes) are associated with alterations in cellular signaling leading to dysfunctions at various levels, and are usually due to errors in the activity of certain enzymes or the control of such proteins. Among the most important metabolic disorders are hypercholesterolemia (high cholesterol levels) and hypertriglyceridemia (high triglyceride levels). These diseases have high rates of incidence, morbidity and mortality, so their treatment is a primary concern. Other important metabolic disorders are diabetes and insulin resistance, characterized by problems in the control of glucose levels. These metabolic pathologies are involved in the appearance of other pathological processes, such as cancer, hypertension, obesity, arteriosclerosis, etc. Another pathological process has been identified related to the metabolic pathologies described above and that could per se constitute a new metabolic pathology, which is metabolic syndrome.

For purposes of the present invention, a neoplasm is defined as an abnormal mass of tissue that appears when cells multiply more than they should or are not destroyed at the appropriate time. Neoplasms are either benign (noncancerous) or malignant (cancerous). The term "neoplasm" is equivalent to "tumor." There are multiple types of cancer, including, for example, oral cavity and pharyngeal cancer, cancer of other digestive organs, cancer of other respiratory organs, bone and joint cartilage cancer, melanoma and other malignant skin neoplasms, cancer of mesothelial and soft tissues, cancer of genital organs, cancer of the urinary tract, cancer of the eye, brain and other regions of the nervous system, cancer of the thyroid and other endocrine glands, neuroendocrine malignancies, cancer of lymphoid, hematopoietic and related tissues, *in situ carcinomas,* benign tumors, neoplasms of uncertain behavior, polycythemia vera and myelodysplastic syndromes, neoplasms of other locations, and neoplasms of unspecified behavior.

The lipidic modification of the cell membrane can be used as a strategy for the prevention or treatment of multiple types of cancer. In one embodiment of the invention, the cancer is selected from the group consisting of: colon cancer, pancreatic cancer, bile duct cancer, neuroblastoma, colon cancer, gastric cancer, liver cancer, glioblastoma, non-Hodgkin lymphoma, kidney cancer, esophageal cancer, stomach cancer, cervical cancer or lymphoma tumors, colorectal carcinoma, colorectal adenocarcinoma, prostate cancer, prostate adenocarcinoma, prostate carcinoma, breast cancer, breast carcinoma, breast adenocarcinoma, triple negative breast cancer, brain cancer, brain adenocarcinoma, brain neuroblastoma, lung cancer, lung adenocarcinoma, lung carcinoma, small cell lung cancer, large cell lung cancer, ovarian cancer, ovarian carcinoma, ovarian adenocarcinoma, uterine cancer, gastroesophageal cancer, renal cell carcinoma, clear cell renal cell carcinoma, endometrial cancer, endometrial carcinoma, endometrial stromal sarcoma, cervical carcinoma, thyroid carcinoma, metastatic papillary thyroid carcinoma, follicular thyroid carcinoma, bladder carcinoma, urinary bladder carcinoma, transitional cell carcinoma of urinary bladder, liver cancer, metastatic liver cancer, pancreatic cancer, neuroendocrine cancers, squamous cell carcinoma, osteosarcoma, rhabdomyosarcoma, embryonic cancers, glioblastoma, glioma, neuroblastoma, medulloblastoma, retinoblastoma, nephroblastoma, hepatoblastoma, melanoma, hematologic malignancies such as leukemias, lymphomas, and myelomas.

Preferably, the cancer is selected from the group consisting of lung cancer, brain cancer, glioma, glioblastoma, breast cancer, leukemia, liver cancer, endometrial cancer, and pancreatic cancer. More preferably, the cancer is selected from the group consisting of lung cancer, brain cancer, breast cancer, leukemia, liver cancer and pancreatic cancer.

For purposes of the present invention, a cardiovascular disease is defined as a set of diseases or disorders of the heart and blood vessels. Such cardiovascular diseases are selected from the group consisting of: cerebral ischemic attack, acute rheumatic fever, chronic heart disease, hypertensive disease, ischemic heart disease, pericarditis, endocarditis, valve disorders, cardiomyopathy, tachycardia, heart failure, amyloid angiopathy, cerebrovascular diseases and disorders, sequelae of cerebral hemorrhage, sequelae of cerebral infarction, sequelae of cerebrovascular diseases, diseases of arterial and capillary arteries; diseases of veins, vessels, and lymph nodes.

The term "pathology of the skin and subcutaneous tissue" refers, for the purposes of the present invention, to pathologies of the dermal tissue among which are: bullous disorders, dermatitis, eczema, papulosquamous disorders, disorders of the skin appendages, postoperative complications, urticaria and erythema.

Inflammatory processes include a broad spectrum of pathologies characterized by the presence of inflammation. For purposes of the present invention, said inflammatory processes are selected from the group consisting of: cardiovascular inflammation; inflammation caused by tumors; inflammation of rheumatoid origin; respiratory inflammation; acute and chronic inflammation; inflammatory hyperalgesia; and edema and inflammation caused by trauma or burns.

The term digestive pathology refers, for purposes of the present invention, to diseases of the oral cavity and salivary glands; diseases of the esophagus, stomach and duodenum; diseases of the appendix; non-infectious enteritis and colitis; diseases of the peritoneum and retroperitoneum; diseases of the liver; disease of the gallbladder, bile ducts and pancreas.

For purposes of the present invention a musculoskeletal and connective tissue disease refers to pathologies of muscles, joints and bones which may or may not have an autoimmune origin. Said musculoskeletal and connective tissue diseases are selected from the group consisting of: arthropathies, connective tissue disorders, muscle and soft tissue disorders; synovial and tendon membrane disorder; osteopathies and chondropathies.

The term genitourinary pathology refers, for purposes of the present invention, to glomerular diseases; tubulo-interstitial kidney diseases; acute kidney failure; chronic kidney disease; lithiasis; and inflammatory and non-inflammatory disorders of the renal tract.

The present invention also relates to a nutraceutical composition comprising at least a first compound selected from the group consisting of:
- a compound of formula (II), or a nutraceutically acceptable salt, or ester thereof:

   COOH-(CH₂)***ₐ***-(CH=CH-CH₂)***_{b}***-(CH₂)***_{c}***-CH₃ (II)

   and
- a compound of formula (III), or a nutraceutically acceptable salt or ester thereof:

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

   wherein said composition optionally comprises a second compound of formula (I), or a nutraceutically acceptable salt or ester thereof:

   COOH -CHOH-(CH₂)***ₐ***-(CH=CH-CH₂)***_{b}***-(CH₂)***_{c}***-CH₃ (I)

   and wherein ***a*** is an integer between 1 and 14; ***b*** is an integer between 1 and 7; c is an integer between 0 and 14; ***m*** is an integer between 0 and **(*b* -** 1); and ***a***+3***b***+***c***+3 is an even integer; and at least one nutraceutically acceptable excipient.

The present invention also relates to a nutraceutical composition comprising at least a first compound selected from the group consisting of:
- a nutraceutically acceptable salt or ester of a compound of formula (II):

   COOH-(CH₂)***ₐ***-(CH=CH-CH₂)***_{b}***-(CH₂)***_{c}***-CH₃ (II)

   and
- a nutraceutically acceptable salt or ester of a compound of formula (III):

   COOH -(CH₂)***ₐ***-(CH=CH-CH₂)***ₘ***-(CH₂)₃-(CH=CH-CH₂)***_{(b-1-m)}***-(CH₂)***_{c}***-CH₃ (III)

   wherein said composition optionally comprises a nutraceutically acceptable salt or ester of a compound of formula (I):

   COOH -CHOH-(CH₂)***ₐ***-(CH=CH-CH₂)***_{b}***-(CH₂)***_{c}***-CH₃ (I)

   and wherein ***a*** is an integer between 1 and 14; ***b*** is an integer between 1 and 7; ***c*** is an integer between 0 and 14; ***m*** is an integer between 0 and **(*b* -** 1); and ***a***+3***b***+***c***+3 is an even integer; and at least one nutraceutically acceptable excipient.

The present invention also relates to a nutraceutical composition comprising at least a first compound selected from the group consisting of a compound of formula (II) and a compound of formula (III), or a nutraceutically acceptable salt or ester thereof, wherein said composition comprises, optionally, a compound of formula (I), or a nutraceutically acceptable salt or ester thereof, as described above, for use in the prevention of a disease or pathology.

Additionally, the present invention also relates to a method of preventing a disease or pathology, said method comprising administering to a subject an effective amount of a nutraceutical composition comprising at least a first compound selected from the group consisting of a compound of formula (II) and a compound of formula (III), or a nutraceutically acceptable salt or ester thereof, said composition optionally comprising a compound of formula (I), or a nutraceutically acceptable salt or ester thereof, as described above.

Preferably, said disease or pathology is selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

Preferably ***m***=0 and, each of the disclosed embodiments of the present invention, including those embodiments referring to compounds of formula (II), or of formula (III), pharmaceutical and nutraceutical compositions comprising them, their first and second medical uses, methods of inducing neuroregeneration and/or preventing neurodegeneration, or methods of preventing and/or treating a disease or pathology, as well as the use and *in vitro* method of determining the efficacy of a treatment, refer to a compound selected from the group consisting of a compound of formula (II), or a pharmaceutically or nutraceutically acceptable salt or ester thereof:

COOH -(CH₂)***ₐ***-(CH=CH-CH₂)***_{b}***-(CH₂)***_{c}***-CH₃ (II)

and a compound of formula (III), or a pharmaceutically or nutraceutically acceptable salt or ester thereof:

COOH -(CH₂)_{***a***+***3***}--(CH=CH-CH₂)***_{(b-1)}***-(CH₂)***c***-CH₃ (III)

wherein ***a*** is an integer between 1 and 14; ***b*** is an integer between 1 and 7; ***c*** is 0, 3 or 6; and wherein ***a***+3***b***+***c***+3 is an even integer.

Also preferably ***m***=0 and, each of the disclosed embodiments of the present invention, including those embodiments referring to compounds of formula (II), or of formula (III), pharmaceutical and nutraceutical compositions comprising them, their first and second medical uses, methods of inducing neuroregeneration and/or preventing neurodegeneration, or methods of preventing and/or treating a disease or pathology, as well as the use and *in vitro* method of determining the efficacy of a treatment, refer to a pharmaceutically or nutraceutically acceptable salt or ester of a compound of formula (II):

COOH -(CH₂)***ₐ***-(CH=CH-CH₂)***_{b}***-(CH₂)***_{c}***-CH₃ (II)

or a pharmaceutically acceptable salt or ester of a compound of formula (III):

COOH -(CH₂)**_{*a*+*3*}**-(CH=CH-CH₂)***_{(b-1)}***-(CH₂)***_{c}***-CH₃ (III)

wherein ***a*** is an integer between 1 and 14; ***b*** is an integer between 1 and 7; ***c*** is 0, 3 or 6; and wherein ***a***+3***b***+***c***+3 is an even integer.

Still more preferably ***m***=0 and, each of the disclosed embodiments of the present invention, including those embodiments referring to compounds of formula (II), or of formula (III), pharmaceutical and nutraceutical compositions comprising them, their first and second medical uses, methods of inducing neuroregeneration and/or preventing neurodegeneration, or methods of preventing and/or treating a disease or pathology, as well as the use and *in vitro* method of determining the efficacy of a treatment, refer to a compound selected from the group consisting of:
- a compound of formula (II), or a pharmaceutically or nutraceutically acceptable salt or ester thereof:

   COOH -(CH₂)***ₐ***-(CH=CH-CH₂)***_{b}***-(CH₂)***_{c}***-CH₃ (II)

   wherein: ***a***=6, ***b***=1 and ***c***=6; or ***a*** =6, ***b***=2 and ***c***=3; or ***a***=6, ***b***=3 and ***c***=0; or ***a***=3, ***b***=3 and ***c***=3; or ***a***=2, ***b***=4 and ***c***=3; or ***a***=2, ***b***=5 and ***c***=0; and
- a compound of formula (III), or a pharmaceutically or nutraceutically acceptable salt or ester thereof:

   COOH -(CH₂)**_{*a*+*3*}**-(CH=CH-CH₂)***_{(b-1)}***-(CH₂)***_{c}***-CH₃ (III)

   where ***a***=1***, b***=6 and ***c***=0***.***

Also more preferably ***m***=0 and, each of the disclosed embodiments of the present invention, including those embodiments referring to compounds of formula (II), or of formula (III), pharmaceutical and nutraceutical compositions comprising them, their first and second medical uses, methods of inducing neuroregeneration and/or preventing neurodegeneration, or methods of preventing and/or treating a disease or pathology, as well as the use and *in vitro* method of determining the efficacy of a treatment, refer to a compound selected from the group consisting of:
- a pharmaceutically or nutraceutically acceptable salt or ester of a compound of formula (II):

   COOH -(CH₂)***ₐ***-(CH=CH-CH₂)***_{b}***-(CH₂)***_{c}***-CH₃ (II)

   wherein: ***a***=6, ***b***=1 and ***c***=6; or a =6, ***b***=2 and ***c***=3; or ***a***=6, ***b***=3 and ***c***=0; or ***a***=3, ***b***=3 and ***c***=3; or ***a***=2, **b***=4* and ***c***=3; or ***a***=2, ***b***=5 and ***c***=0; and
- a pharmaceutically or nutraceutically acceptable salt or ester of a compound of formula (III):

   COOH -(CH₂)**_{*a*+*3*}**-(CH=CH-CH₂)***_{(b-1)}***-(CH₂)***_{c}***-CH₃ (III)

   where ***a***=1***, b***=6 and ***c***=0***.***

Still more preferably, said salt is a sodium salt, and said ester is an ethyl ester.

In one embodiment of the invention, the pharmaceutical and nutraceutical compositions described herein comprise a compound of formula (I), together with a compound of formula (II) or a compound of formula (III), in a concentration between 0.01% to 99.99% w/w, preferably the composition comprises 10% to 80% w/w, or even more preferably in a concentration between 20% to 80% w/w. In another embodiment of the invention, the compositions described herein comprise a prodrug of formula (I) together with a compound of formula (II) or a compound of formula (III), wherein said combination is in a ratio in the range of 0.01:100 to 100:0.01, preferably 1:5 to 5:1, and most preferably 1:2 to 2:1.

In a further aspect, the pharmaceutical or nutraceutical compositions of the invention may be presented in vials, ampoules, powders, capsules, tablets, sachets, solutions, syrups, ointment, creams, emulsions, gels, patches, controlled release formulations, suppositories, eggs, etc. The formulations are useful to be administered by, among others, oral, sublingual, gastroenteric, rectal, parenteral (intravenous, intraarterial, intramuscular and subcutaneous), respiratory, topical (ophthalmic, otic, transdermal). The route of administration can be determined in a simple manner by the person skilled in the art.

The compositions of the present invention may be in the form of a gastro-resistant composition to prevent degradation of their components by the low pH of the gastric environment. In certain embodiments, the composition of the invention further includes one or more additional components or excipients, such as diluents, antioxidants, sweeteners, gelling agents, flavoring agents, fillers or other vehicles, such as colloidal anhydrous silica and glyceryl monostearate. Said compositions may be in the form of a capsule, envelope, paper, or other packaging. Conventional techniques for preparing pharmaceutical compositions can be used to prepare said compositions. For example, the compounds disclosed herein above may be mixed with a carrier, or diluted by a carrier, or enclosed within a carrier that may be in the form of an ampoule, capsule, envelope, paper, or other packaging. When the carrier is a diluent, it may be a solid, semi-solid, or liquid material that acts as a vehicle, excipient, or medium for the active compound. Some examples of suitable diluentss are water, saline solutions, alcohols, polyethylene glycols, polyhydroxyethoxylated castor oil, peanut oil, olive oil, lactose, terra alba, saccharose, cyclodextrins, amylose, magnesium stearate, talcum, gelatin, agar, pectin, acacia, stearic acid, cellulose alkyl ethers, silicic acid, fatty acids, fatty acid amines, fatty acid monoglycerids and diglycerids, fatty esters of pentaerythrol, polyethylene, hydroxymethylcellulose and polyvinylpirrolidone. Similarly, the carrier or diluent can include any sustained release material known in the art, such as glyceryl monostearate or glyceryl distearate, alone or mixed with a wax. Said compositions may also include wetting agents, antioxidants, emulsifying and suspending agents, preserving agents, sweetening agents, and flavoring agents. The compositions of the invention may be formulated to provide rapid, sustained or delayed release of the compounds disclosed herein after administration to the patient using methods well known in the art.

The disclosed compositions may be solid compositions or liquid solutions. In one non-limiting embodiment of the invention, said composition is a solid composition which may comprise 20-80%, of the compound of formula (I) and/or the compound of formula (II) or formula (III), 20-80% of a diluent, 0.1-20% of an antioxidant, 0.01-10% of a sweetener, 0.1-20% of a gelling agent, and 0.01-10% of a flavoring agent. In another, non-limiting embodiment of the invention, said composition is a solution for oral administration comprising 20 to 80% of the compound of formula (I) and/or of the compound of formula (II) or of formula (III), 20 to 80% of diluent, 0.1 to 20% of antioxidant, 0.01 to 10% of a sweetener, 0.1 to 20% of a gelling agent, and 0.01 to 10% of a flavoring agent.

The pharmaceutical compositions may be sterilized and mixed, if desired, with auxiliary agents, emulsifiers, salt to influence osmotic pressure, buffers and/or coloring substances and the like, which do not react adversely with the compounds disclosed above.

### BRIEF DESCRIPTION OF THE FIGURES

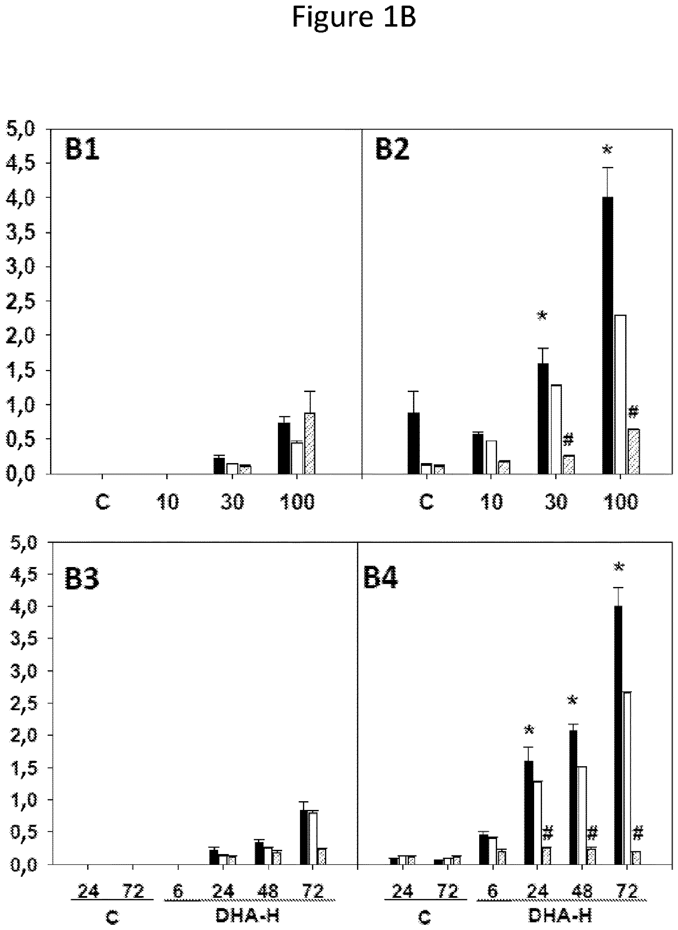

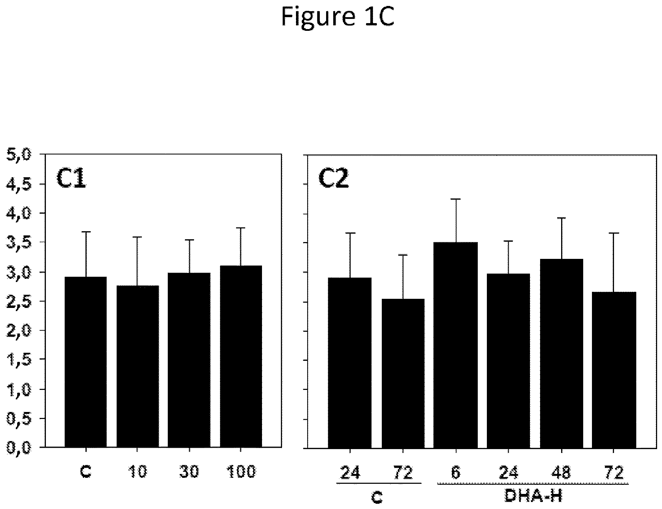

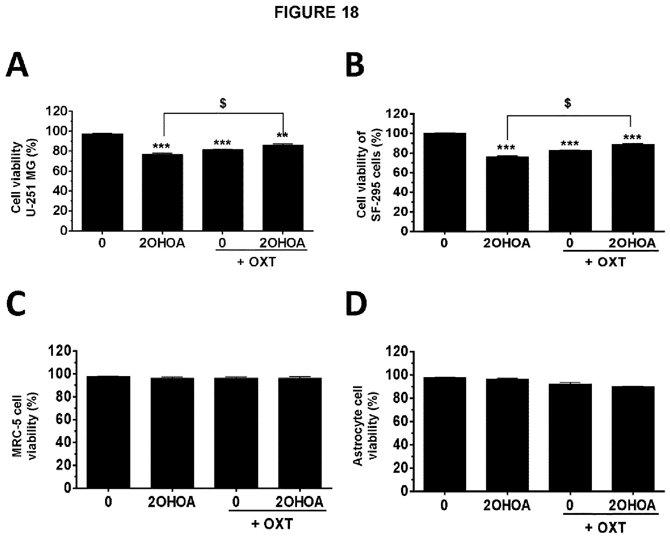

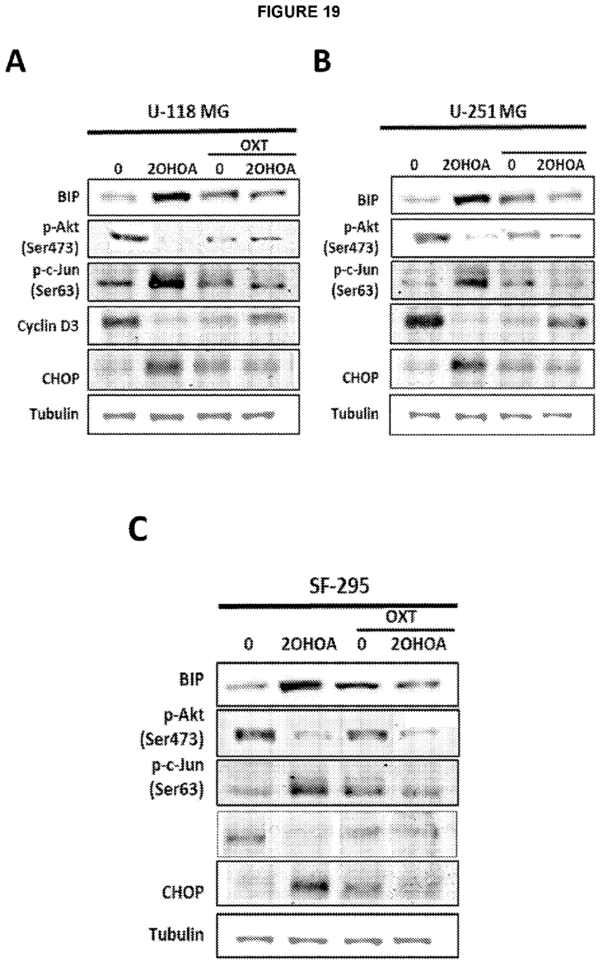

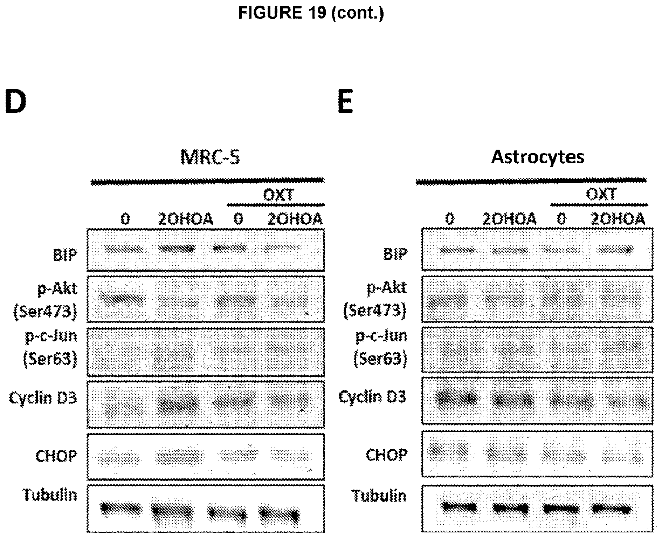

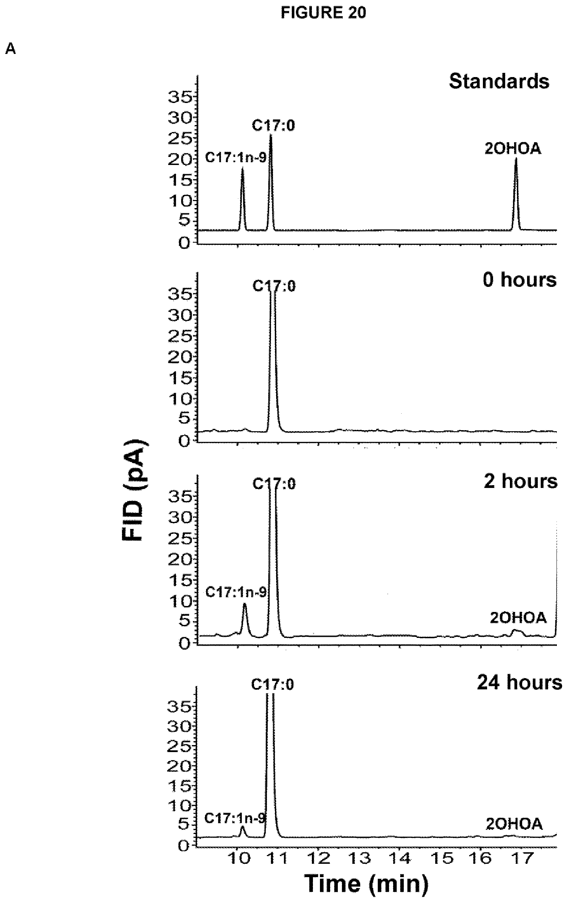

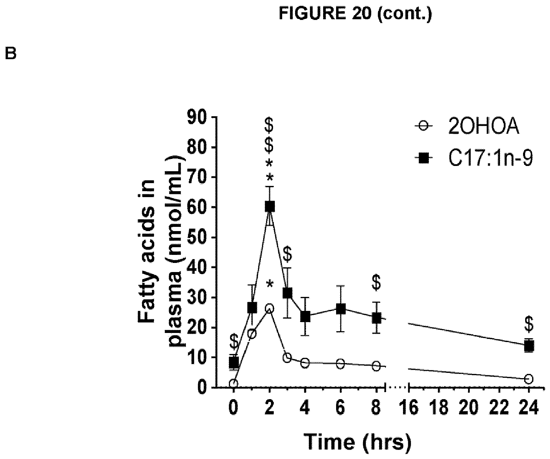

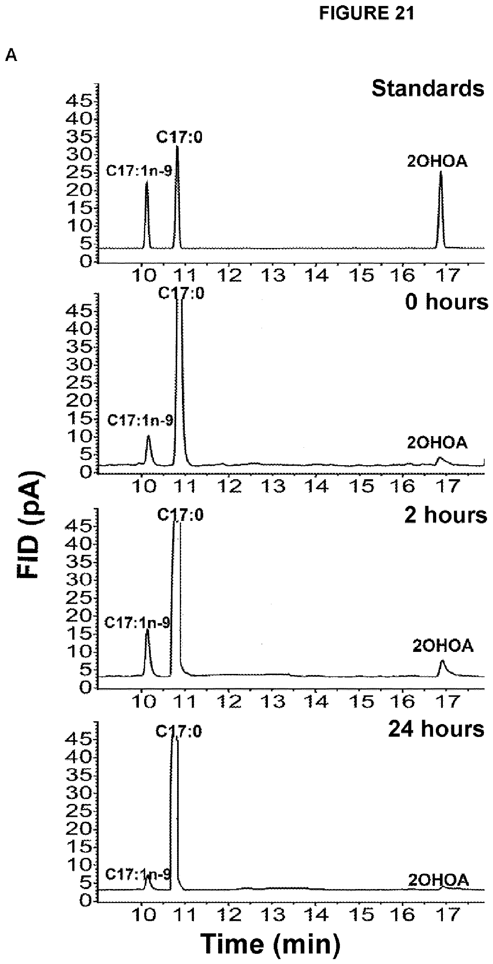

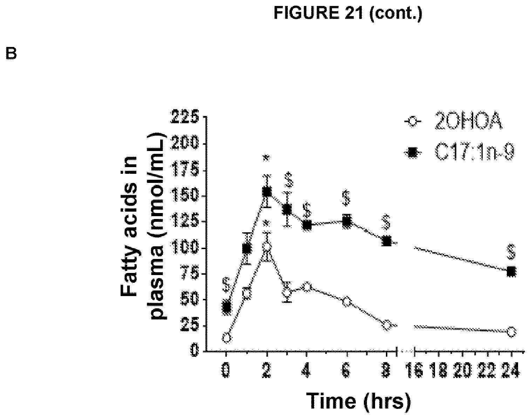

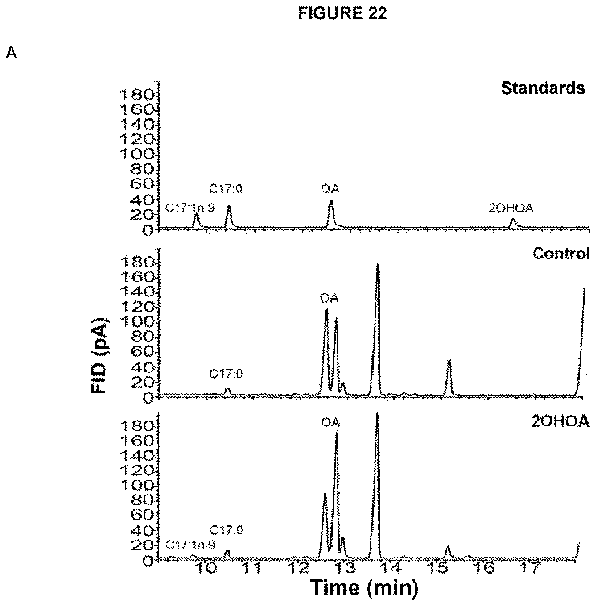

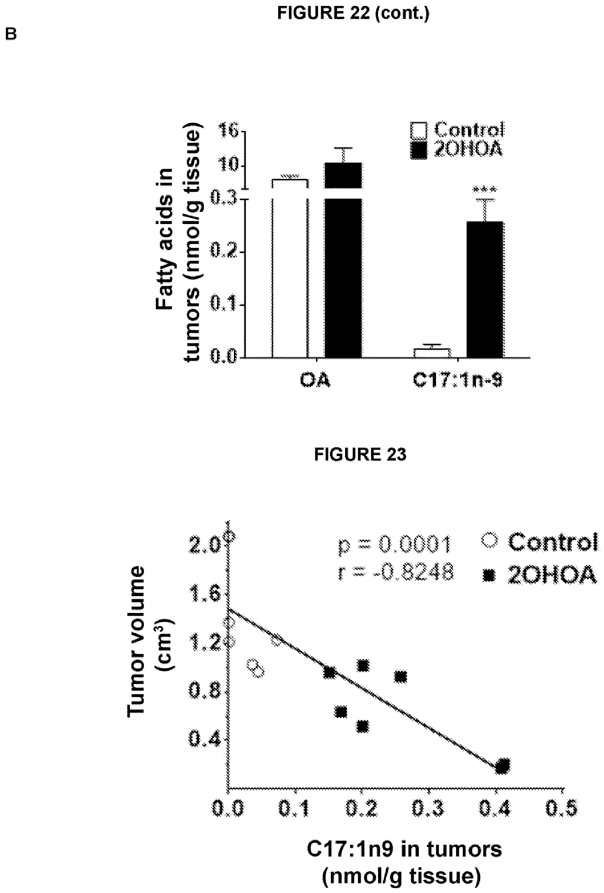

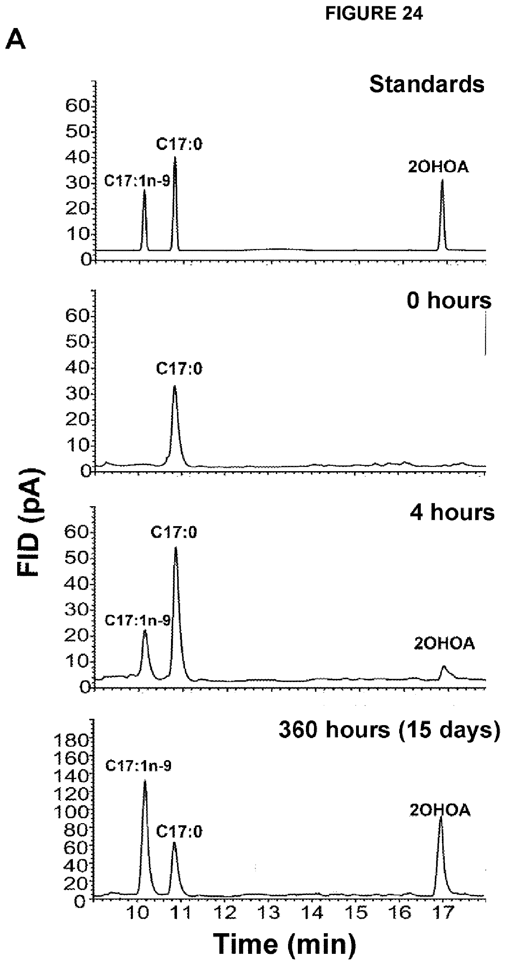

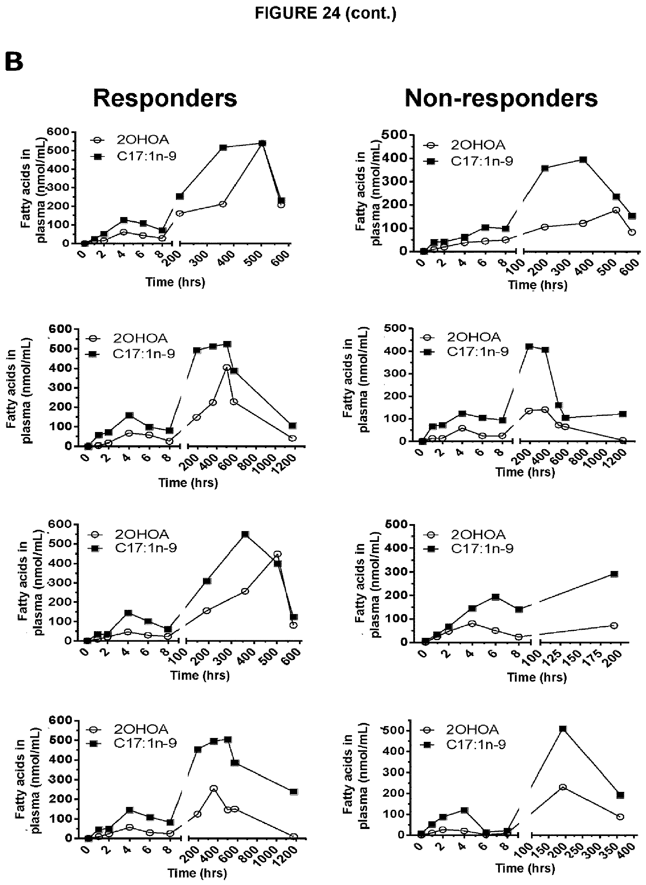

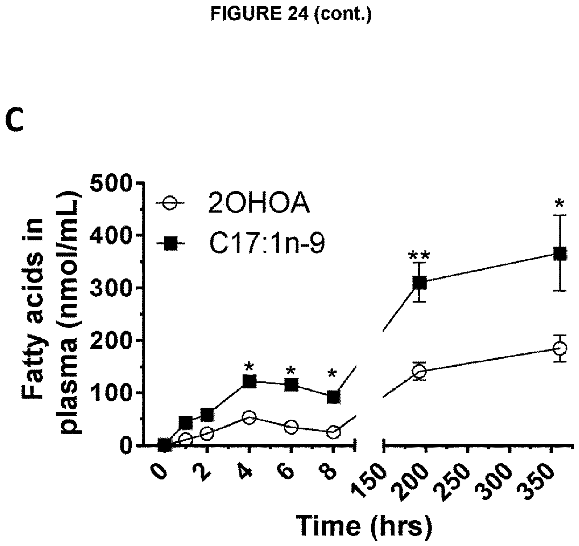

**Figure 1****. A** Scheme illustrating the cellular metabolism of 2-hydroxidocosahexaenoic acid (DHA-H) resulting in (6*Z*,*9Z*,12*Z*,15*Z*,18*Z*)-heneicose-6,9,12,15,18-pentaenoicacid (HPA) by α-oxidation. DHA-H requires activation by an Acyl-CoA synthetase, in a process dependent on ATP (adenosine triphosphate) and magnesium (Mg²⁺). DHA-H-CoA would be subject to the activity of 2-hydroxyfitanoyl-CoA lyase (2-hydroxyacyl-CoA lyase 1, HACL1), leading to the formation of an intermediate polyunsaturated aldehyde that should contain 5 or 6 double bonds. The activity of HACL1 is dependent on thiamine pyrophosphate (TPP) and Mg²⁺, and can be inhibited by a competitive antagonist (e.g. oxythiamine). The aldehyde dehydrogenase enzyme would be responsible for the conversion of the intermediate aldehyde to HPA in a a process dependent on NAD⁺ (Nicotinamide Adenine Dinucleotide). **B.** DHA-H is metabolically transformed into HPA by α-oxidation in HEK293T cells. Intracellular levels of DHA-H (B1 and B3) and HPA (B2 and B4) are represented in the ordinate axis (nmoles /mg protein), versus treatment concentration with DHA-H sodium salt (µM) for 24 hours (B1 and B2) or incubation time (h) with a constant concentration of DHA-H sodium salt of 30 µM (B3 and B4), including untreated controls (C), in the abscissa axis. Black bars represent the result in cells without additional stimulus, white bars represent the result after simultaneous treatment with 1 mM oxythiamine, and striped bars represent the result after treatment with 10 mM oxythiamine. Both DHA-H and HPA increased as a function of concentration and incubation time, with HPA levels significantly higher than those of DHA-H at 30 µM exposure of DHA-H sodium salt from 24 hours. This increase in HPA is inhibited in the presence of 10 mM oxythiamine (which inhibits the HCLA1 enzyme), demonstrating the involvement of α-oxidation in this metabolic conversion. The bars represent the mean ± standard error, and the statistical analysis was performed using one-way ANOVA and the Tukey multiple evaluation test: * p < 0.05 when comparing HPA levels with those of DHA-H under the same condition; #, p<0.05 when comparing values in the presence and absence of 10 mM oxythiamine. C. Endogenous levels of DHA (non-hydroxy native form of docosahexaenoic acid) in HEK293T are not altered after treatment with DHA-H sodium salt. Intracellular levels of DHA are represented on the ordinate axis (nmoles/mg protein), versus treatment concentration with DHA-H sodium salt (µM) for 24 hours (C1) or incubation time (h) with a constant concentration of DHA-H sodium salt of 30 µM (C2), including untreated controls (C), on the abscissa axis. Treatment with DHA-H sodium salt did not have any significant effect on DHA levels either as a function of concentration or incubation time. The bars represent the mean ± standard error, and the statistical analysis was done by unidirectional ANOVA and Tukey multiple evaluation test. From these results it can be concluded that the administration of DHA-H, or its sodium salt in this case, does not alter the endogenous levels of DHA (docosahexaenoic acid), nor of other cellular fatty acids studied, but that treatment with DHA-H results in an increase exclusively in the levels of HPA, implying that the therapeutic effect obtained through the treatment with DHA-H, and in particular with its sodium salt, is mediated by HPA and not by a modulation of the levels of other fatty acids of endogenous origin.

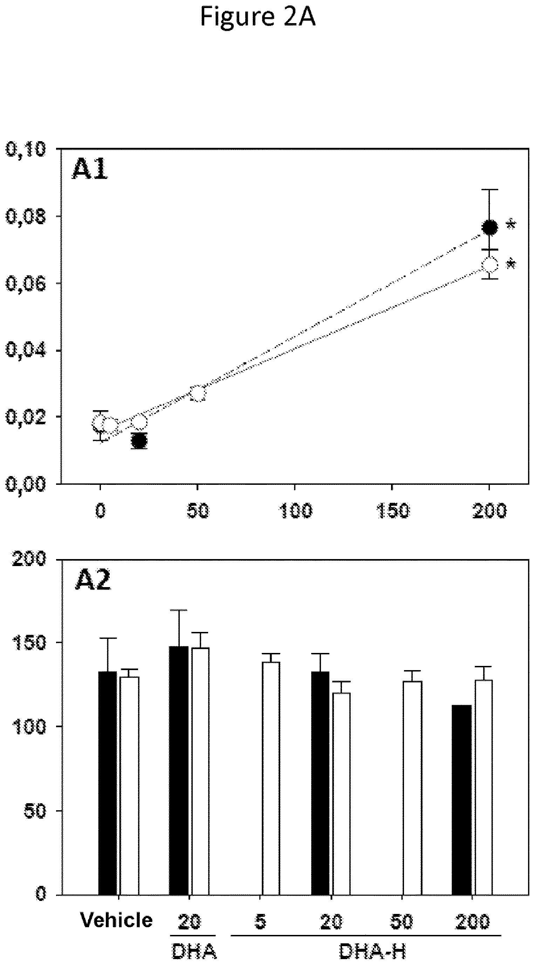

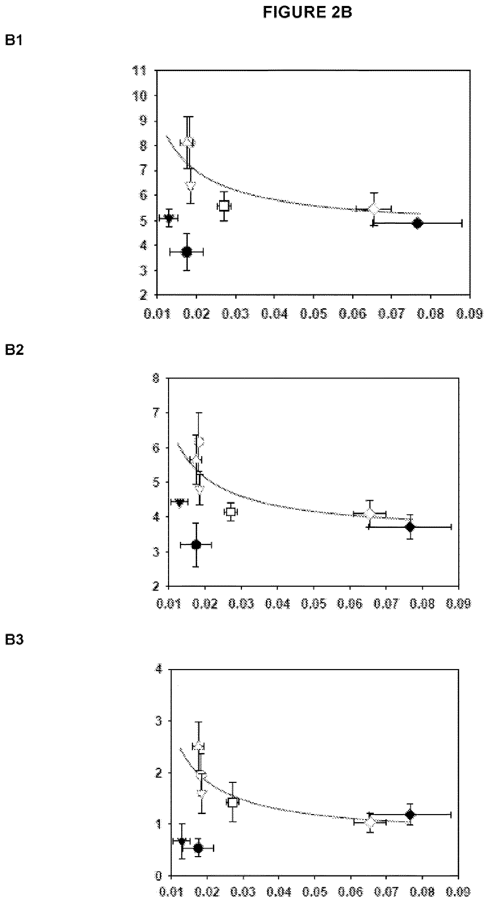

**Figure 2****. A.** Mice treated with DHA-H sodium salt exhibit dose-dependent brain accumulation of HPA, with DHA-H being undetectable in the brain. Brain levels of HPA **(A1)** or DHA **(A2)** (nmoles/mg of protein) are represented in the ordinate axis, with respect to treatment doses with the sodium salt of DHA-H (A1) and the sodium salt of DHA (A1 and A2) (mg/kg). A1: • animals WT; ∘ 5xFAD. A2: Black bars refer to WT animals and white bars to 5xFAD. The levels of HPA and DHA were determined in the brain of WT and 5xFAD mice after chronic administration of DHA-H sodium salt (4 months; 5 doses/week M-F; between 3 and 7 months of age; sacrifice at 7 months). HPA accumulates in the brain of both mouse strains similarly, depending on the dose of DHA-H sodium salt administered (A1: • r2= 0.9292, p= 0.0002; ∘ r2= 0.9704, p<0.0001). DHA levels did not vary significantly between experimental conditions (A2). Data are shown as mean ± standard error, and statistical analysis was done by one-way ANOVA and Tukey multiple evaluation test: * p< 0.05 compared to control (mice treated with vehicle). Therefore, when the prodrug DHA-H, in particular its sodium salt, is administered in healthy mice and transgenic models of Alzheimer's disease, a significant accumulation of HPA occurs at the brain level, without the presence of the prodrug (DHA-H) being detected, nor changes in endogenous levels of DHA. **B.** 5xFAD mice treated with DHA-H sodium salt exhibit cognitive improvement that directly correlates with brain HPA levels. The number of total errors **(B1),** reference memory errors (RME) **(B2)** or working memory errors (WME) **(B3),** committed, are represented in the ordinate axis against the brain levels of HPA (nmol/mg of protein) in the abscissa axis. The cognitive assessment was done by testing the 8-arm radial labyrinth during the last month of treatment of the same animals shown in figure 2A. Individual experimental points from the entire study animal population were plotted and the data from the 5xFAD animals were adjusted to an inverse polynomial regression f(x)=y₀+(a/x). The values of r² and p corresponding to the regression of each parameter are shown below: total errors, RME and WME versus the brain concentration of HPA: C1: r²=0.9146 and p=0.0311; C2: r²=0.9252 and p=0.0243; C3: r²=0.7785 and p=0.0346. The data obtained suggest that minimal increases in brain levels of HPA are associated with an improvement in spatial cognition. Each point in the graphs represents the average ± standard error for each pathology/treatment condition: • WT + vehicle, ▼ WT + DHA-H 20 mg/kg; ◆ WT + DHA-H 200 mg/kg; ∘ 5xFAD + vehicle; △ 5xFAD + DHA-H 5mg/kg; ∇ 5xFAD + DHA-H 20mg/kg; □ 5xFAD + DHA-H 50 mg/kg; ◇ 5xFAD + DHA-H 200mg/kg. These results show that moderate increases in brain HPA levels are significantly related to an improvement in spatial cognition, which is one of the most affected cognitive abilities in Alzheimer's disease.
**Figure 3****. A.** Mice treated with the sodium salt of DHA-H exhibit tumor accumulation of HPA, being undetectable DHA-H, in U118 cell xenographic tumors. The levels of DHA (black bars) and HPA (white bars) (pmoles/mg of tissue) in the tumor are represented in the ordinate axis, versus the treatment condition (vehicle and DHA-H 200 mg/kg) in the abscissa axis. NUDE (immunosuppressed) mice at 3 months of age were injected subcutaneously with 7.5.10⁶ U118 cells (grade IV human multiform glioblastoma). Tumor growth was allowed at the subcutaneous level for 10 days prior to initiation of oral treatments (vehicle or DHA-H sodium salt 200 mg/kg), which were maintained 42 days until sacrifice. Lipid analysis of the xenographic tumors revealed the absence (undetectable levels) of DHA-H. The bars represent the mean ± standard error for each treatment condition. **B.** Tumor HPA levels correlate inversely with tumor size in xenographic models. The size of the tumor (cm³) is represented, in the ordinate axis, versus the levels of HPA (pmoles/mg of tissue) in the tumor, in the abscissa axis, for two treatment conditions: ∘ vehicle and sodium • salt of DHA-H 200 mg/kg. The presence of HPA in the tumor of animals under treatment with DHA-H sodium salt has a statistically significant linear relationship with tumor volume (A2), where: r²= 0.4296 and p= 0.0029. The results showed that HPA levels in xenographic tumors have a statistically significant inverse linear relationship to tumor size. In the absence of the DHA-H parent molecule in the target organ, these evidences show that the presence of HPA in the target organ has an *in vivo* therapeutic effect.
**Figure 4****. A.** DHA-H is a prodrug that is metabolically transformed into HPA by α-oxidation in U118 cells. The intracellular levels of DHA (black bars), DHA-H (white bars) and HPA (striped bars) are represented on the ordinate axis (nmoles /mg of protein), with respect to the treatment conditions: Control (C) and sodium salt of DHA-H 150 µM (48 h), in the presence or absence of simultaneous treatment with oxythiamine 1 and 10 mM, in the abscissa axis. Both DHA-H and HPA increased in cells treated with the sodium salt of DHA-H. This increase in HPA is inhibited in the presence of 1 and 10 mM oxythiamine, demonstrating the involvement of α-oxidation in this metabolic conversion. The bars represent the mean ± standard error, and the statistical analysis was performed using one-way ANOVA and the Tukey multiple evaluation test: * p < 0.05 when comparing only HPA levels. **B.** Metabolic conversion of DHA-H to HPA is necessary for an anti-tumor effect to exist. The cell viability (% of the control -C-without oxythiamine) is represented in the ordinate axis, with respect to the treatment conditions: Control (C- black bars) and the sodium salt of DHA-H 150 µM), 48 h (white bars) in the presence and absence of simultaneous treatment with 1 mM oxythiamine, in the abscissa axis. Treatment with DHA-H on U118 cells significantly reduces the viability of the culture, while treatment with oxythiamine (alone) has no effect on cell viability. However, when treatment with the sodium salt of DHA-H is done simultaneously with oxythiamine, the antiproliferative effect of this compound decreases significantly compared to the effect without oxythiamine. The bars represent the mean ± standard error, and the statistical analysis was performed using one-way ANOVA and the Tukey multiple evaluation test: * p < 0.05 when compared to control (C); # p <0.05 when comparing the effect of DHA-H in the presence and absence of oxythiamine.
**Figure 5****. A.** Viability of U118 cells in culture after treatment with DHA-H sodium salt, DHA sodium salt, and HPA. The cell viability (% of the control without treatment) is represented in the ordinate axis, versus the different treatment conditions in the abscissa axis: Control (black bar), DHA-H sodium salt (150 µM), 48h - white bar), DHA (150 µM), 48 h - striped bar) and HPA (150 µM), 48 h - grid bar). Treatment with HPA under the same conditions induces a much more evident degree of mortality on the culture than that induced by DHA-H (prodrug) or DHA (natural analogue). This effect could be due to a mixture of anti-proliferative effect and toxic effects typical of HPA, which would not be attributable to DHA-H or DHA under the same experimental conditions. The bars represent the mean ± standard error, and the statistical analysis was performed using one-way ANOVA and the Tukey multiple evaluation test: * p < 0.05 when compared to control. **B.** Intracellular levels of HPA in U118 cells in culture, treated with the sodium salt of DHA-H and HPA. The levels of HPA (nmoles/mg of protein) are represented in the ordinate axis, versus the treatment conditions in the abscissa axis: sodium salt of DHA-H (150 µM), 48 h - black bar) and HPA (5-150 µM), 48 h - white bars). Administration of 150 µM of the DHA-H sodium salt results in HPA levels equivalent to those generated by the HPA treatment itself at 5 µM. Treatment with 150 µM HPA results in significantly higher intracellular levels of HPA than those generated by the same concentration of administration of the prodrug. The bars represent the mean ± standard error.
   C. Levels of DHA-H and DHA in HEK293T cells in the presence (C1) or absence (C2) of culture medium. The levels of DHA-H (•) and DHA (∘) in the culture medium (% of the initial levels at time 0) are represented in the ordinate axis, versus the incubation time (h) in the abscissa axis. The concentration of the lipid in the culture medium is 30 µM and the culture plates were incubated for up to 72 h. In the presence of cell culture (C1), DHA levels in the medium decreased significantly at 48 and 72 h, as a consequence of DHA uptake by the cells, while DHA-H levels remained unchanged up to 72 h. In the absence of cell culture (C2), the levels of both DHA and DHA-H remained constant over time. The bars represent the mean ± standard error, and the statistical analysis was performed using one-way ANOVA and the Tukey multiple evaluation test: * p < 0.05 when compared to control.

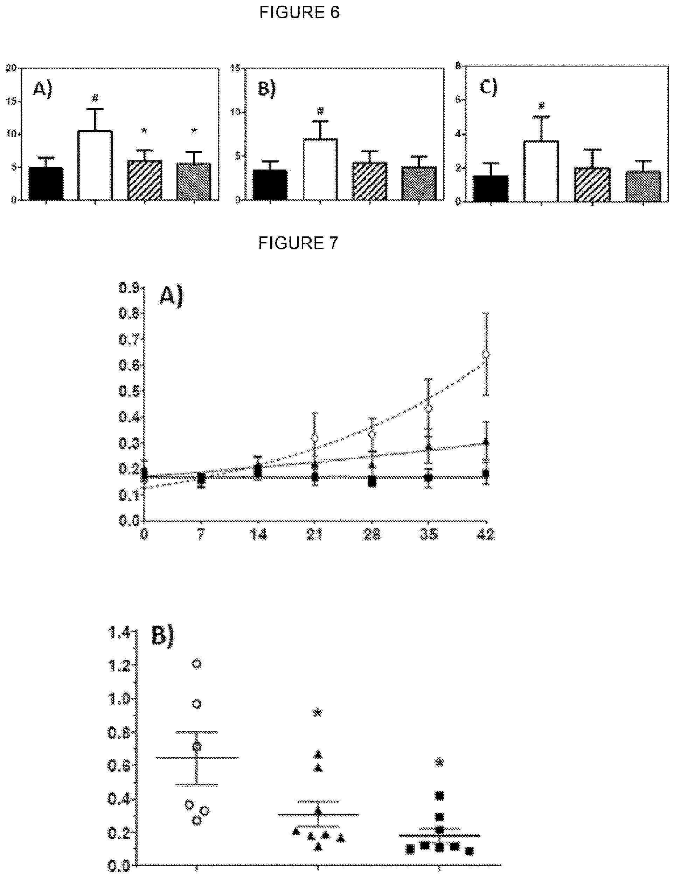

**Figure 6****. A, B and C:** Chronic treatment with HPA acid or its prodrug, DHA-H, prevents cognitive decline typical of Alzheimer's disease in the murine transgenic model (5xFAD). Cognitive assessment was performed by the 8-arm Radial Labyrinth test. The animals received treatment between 3 and 7 months of age and the test was performed during the last month of treatment. During this test, the total errors made during the test **(A),** the reference memory errors (RME) **(B)** and the working memory errors (WME) **(C)** were taken into account. Each column represents the mean ± SEM of errors during the last week of the radial labyrinth test. The black columns represent the errors made by WT mice. The blank columns represent the errors made by the 5xFAD transgenic mice treated with the vehicle (5% ethanol). Striped columns represent errors made by 5xFAD mice treated with DHA-H (20 mg/kg/day). Boxed columns represent errors made by 5xFAD mice treated with HPA (20 mg/kg/day). Results show a cognitive improvement of 5xFAD mice treated with DHA-H and HPA in a similar manner. The bars represent the mean ± standard error for each treatment condition and the statistical analysis was performed by unidirectional ANOVA and the Tukey multiple evaluation test: * p < 0.05 when compared to healthy control (WT) and # p < 0.05 when compared to 5xFAD control condition (treated with the vehicle).
**Figure 7****. A:** Tumor growth is inhibited *in vivo* in the presence of treatment with HPA sodium salt or its prodrug, DHA-H, in xenographic models. The size of the tumor (cm³) is represented in the ordinate axis, versus the days of treatment elapsed in the abscissa axis. To induce xenographic tumors in NUDE (immunosuppressed) mice 3 months of age, 7.5.10⁶ human grade IV (U-118 MG) glioblastoma cells were inoculated subcutaneously on both sides of the animal's dorsal flank (8-12 weeks of age, 30-35 g). After 10 days, the tumors became visible with an approximate volume of 0.1 cm³. Animals were randomly divided into groups with a similar mean tumor volume and received daily oral treatments for 42 days: ∘ vehicle (untreated control), DHA-H ▲ (200 mg/kg/day) and ■ HPA (200 mg/kg/day). Tumor volumes (v) were calculated as v = A² × L/2, where A is the width of the tumor and L is its length. The data obtained for each treatment condition were adjusted to an exponential growth curve. B: HPA and the prodrug, DHA-H, reduce the volume of the xenographic tumor significantly compared to the untreated control. The volume of the tumors induced 42 days after the start of the treatments is represented in the ordinate axis, versus the treatment conditions in the abscissa axis. The individualised data of the animals participating in the study are represented: ∘ carrier (untreated control), DHA-H ▲ (200 mg/kg/day) and ■ HPA (200 mg/kg/day). The bars represent the mean ± standard error for each treatment condition and the statistical analysis was performed by unidirectional ANOVA and the Tukey multiple evaluation test: * p < 0.05 when compared to the control condition.
**Figure 8****.** Illustrative schemes of the cellular metabolism of 2-hydroxylated polyunsaturated fatty acids (prodrugs, PUFA-H) giving rise via α-oxidation to their corresponding non-hydroxylated metabolites, the latter having one carbon atom less than the initial molecule. Hydroxylated fatty acid requires activation by an Acyl-CoA synthetase, in a process dependent on ATP (adenosine triphosphate) and magnesium (Mg²⁺). This PUFA-H-CoA would be subject to the activity of 2-hydroxyacyl-CoA lyase (HACL, isoforms 1 or 2 depending on the cell type), which would lead to the formation of an intermediate polyunsaturated aldehyde. The activity of HACL depends on thiamine pyrophosphate (TPP) and Mg²⁺, and can be inhibited by a competitive antagonist, such as oxythiamine. The aldehyde dehydrogenase enzyme would be responsible for the conversion of the intermediate aldehyde into the final fatty acid in a process dependent on a process dependent on NAD⁺ (Nicotinamide Adenine Dinucleotide). A. Scheme of cell conversion of 2-hydroxy-linoleic acid (LA-H) resulting in (8Z,11Z)-heptadeca-8,11-dienoicacid (HDA). **B.** Scheme of cell conversion of 2-hydroxy-alpha (a)-linolenic acid (ALA-H) to (8Z,11Z,14Z)-heptadeca-8,11,14-trienoic acid (HTA ω-3). **C.** Scheme of cell conversion of 2-hydroxy-gamma (γ)-linolenic acid (GLA-H) resulting in (5Z,8Z,11Z)-heptadeca-5,8,11-trienoicacid (HTA ω-6). **D.** Scheme of cell conversion of 2-hydroxy-arachidonic acid (ARA-H) resulting in (4Z,7Z,10Z,13Z)-nonadeca-4,7,10,13-tetraenoicacid (NTA). **E.** Scheme of cell conversion of 2-hydroxy-eicosapentaenoic acid (EPA-H) resulting in (4Z,7Z,10Z,13Z,16Z)-nonadeca-4,7,10,13,16-pentaenoicacid (NPA). **F.** Scheme of cell conversion of 2-hydroxidocosahexaenoic acid (DHA-H) resulting in (6Z,9Z,12Z,15Z,18Z)-heneicosa-6,9,12,15,18-pentaenoicacid (HPA).

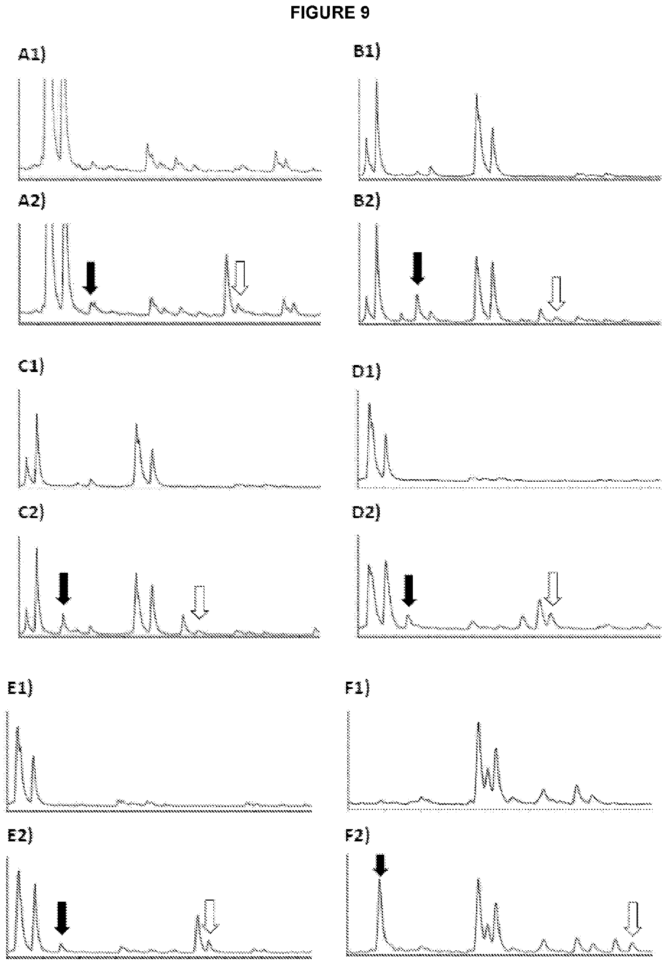

**Figure 9****.** Amplified regions of the different chromatograms obtained by gas chromatography with flame ionisation detector (GC-FID) by treating HEK293T cells with the corresponding prodrug: **A.** (1) control (vehicle) and (2) LA-H (100 µM), 24 h). The white arrow indicates the chromatographic peak of the LA-H parent molecule, the black arrow indicates the chromatographic peak corresponding to the HDA metabolite. The formation of HDA is inhibited in the presence of 10 mM oxyamine. **B.** (1) control (vehicle) and (2) ALA-H (100 µM), 24 h). The white arrow indicates the chromatographic peak of the ALA-H parent molecule, the black arrow indicates the chromatographic peak corresponding to the metabolite HTA ω-3. The formation of ω-3 HTA is inhibited in the presence of 10 mM oxythiamine. **C.** (1) control (vehicle) and (2) GLA-H (100 µM), 24 h). The white arrow indicates the chromatographic peak of the parent molecule GLA-H, the black arrow indicates the chromatographic peak corresponding to the metabolite HTA ω-6. The formation of ω-6 HTA is inhibited in the presence of 10 mM oxythiamine. **D.** (1) control (vehicle) and (2) ARA-H (100 µM), 24 h). The white arrow indicates the chromatographic peak of the ARA-H parent molecule, the black arrow indicates the chromatographic peak corresponding to the NTA metabolite. The formation of NTA is inhibited in the presence of 10 mM oxythiamine. **E.** (1) control (vehicle) and (2) EPA-H (100 µM), 24 h). The white arrow indicates the chromatographic peak of the EPA-H parent molecule, the black arrow indicates the chromatographic peak corresponding to the NPA metabolite. NPA formation is inhibited in the presence of 10 mM oxyamine. **F.** (1) control (vehicle) and (2) DHA-H (100 µM), 24 h). The white arrow indicates the chromatographic peak of the HPA parent molecule, the black arrow indicates the chromatographic peak corresponding to the HPA metabolite. The formation of HPA is inhibited in the presence of 10 mM oxythiamine.
**Figure 10****.** Treatment with HPA and other odd-chain polyunsaturated fatty acids prevents excitotoxicity-induced neuronal death. Neuronal culture was obtained by differentiation from human SH-SY5Y neuroblastomas by retinoic acid and BDNF (Brain Derived Neurotrophic Factor). Neuronal death by excitotoxicity was induced by the addition of NMDA (10 mM) and calcium/glycine (530 µM/10 mM) to the culture medium for 1 hour. To test the neuroprotective effect of the different study compounds (HDA or C17:2 ω-6, HTA ω-3 or C17:3 ω-3, HTA ω-6 or C17:3 ω-6, NTA or C19:4 ω-6 and HPA or C21:5 ω-3), pre-treatmentwas performed for 24 hours: vehicle (black bars), 1 µM (white bars), 3 µM (dotted bars) and 10 µM (striped bars). Treatments tested under these experimental conditions demonstrated that these compounds can prevent excitotoxicity-induced death from a 3 µM concentration. The bars represent the mean ± standard error for each treatment condition and the statistical analysis was performed by unidirectional ANOVA and the Tukey multiple evaluation test: * p < 0.05 when compared to the control condition (vehicle pre-treatment).
**Figure 11****.** Illustrative scheme of 2-OHOA (LAM561) cell metabolism resulting in 8Z-heptadecenoic acid (C17:1n9), by α-oxidation. 2-OHOA requires activation by an Acyl-CoA ligase, in a process dependent on ATP (adenosine triphosphate) and magnesium (Mg2+). 2OHOA-CoA would be subject to the activity of 2-hydroxyfitanoyl-CoA lyase (2-hydroxyacyl-CoA lyase 1, HACL1), leading to the formation of an intermediate monounsaturated aldehyde. The activity of HACL1 is dependent on thiamine pyrophosphate (TPP) and Mg²⁺, and can be inhibited by a competitive antagonist (e.g. oxythiamine). The aldehyde dehydrogenase enzyme would be responsible for the conversion of the intermediate aldehyde to 8Z-heptadecenoic in a a process dependent on NAD⁺ (Nicotinamide Adenine Dinucleotide).

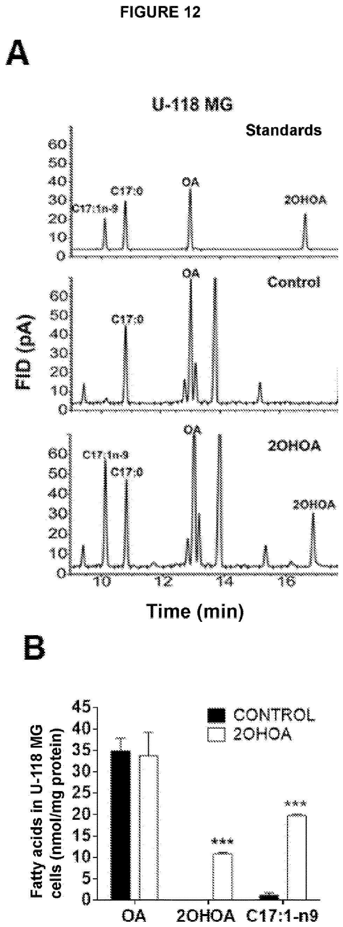

**Figure 12****.** Analysis of the composition of fatty acids in U-118 MG glioma cells. (A) Representative chromatograms showing the composition of fatty acids in U-118 MG cells incubated in the presence of 400 µM 2OHOA sodium salt or absence of treatment (Control) for 24h, determined by gas chromatography. Retention times (min): C17:1n-9 (10.12), OA (13.01), 2OHOA (16.87), and C17:0 margaric acid as internal control (10.81). **(B)** Quantification of different fatty acids identified in the chromatograms (OA, 2OHOA and C17:1n-9). The black bar corresponds to the concentration of each fatty acid in the control and the white bar corresponds to the concentration of the fatty acid after treatment with 2OHOA sodium salt. The columns show the mean ± SEM of three independent experiments expressed in nmoles and normalized per mg of protein. Statistical significance is determined with a Student's t test (***p<0.001 with respect to the control).

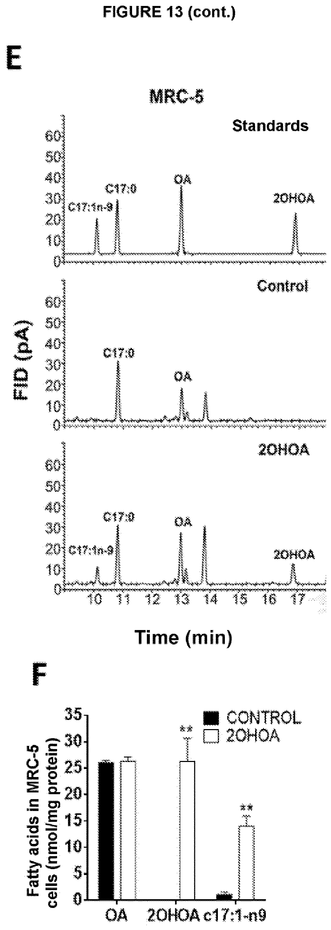

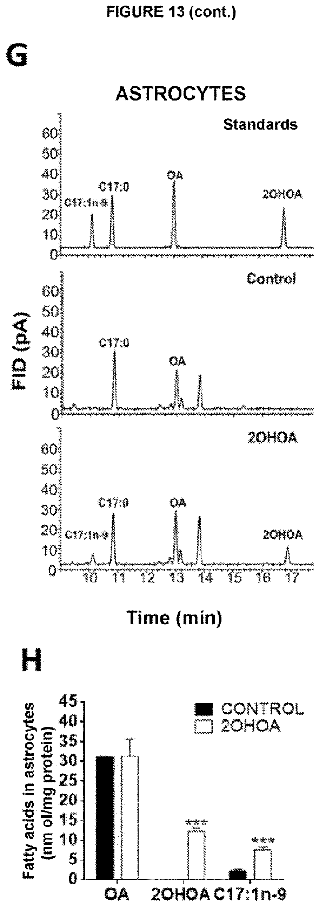

**Figure 13****.** Analysis of the composition of fatty acids in different glioma and non-tumor cell lines after treatment with 2OHOA sodium salt. Representative chromatograms showing the composition of fatty acids (left) and quantification of different fatty acids identified in the chromatograms (C17:0, OA, 2OHOA and C17:1n-9) (right) in glioma cells: **(A)** and **(B)** U-251 MG; **(C)** and **(D)** SF-295; and non-tumor: **(E)** and **(F)** MRC-5 (human fibroblasts); **(G)** and **(H)** mouse astrocytes, after treatment in the absence (control) or presence of 2OHOA sodium salt (400 µM, 24 hours) determined by gas chromatography analysis. The black bar corresponds to the concentration of each fatty acid in the control, and the white bar corresponds to the concentration of the fatty acid after treatment with 2OHOA sodium salt. C17:0 margaric acid is included as an internal control. The columns show the mean ± SEM of three independent experiments expressed in nmoles and normalized per mg of protein. Statistical significance is determined with a Student's t-test (**p<0.01, * **p<0.001 with respect to the control).

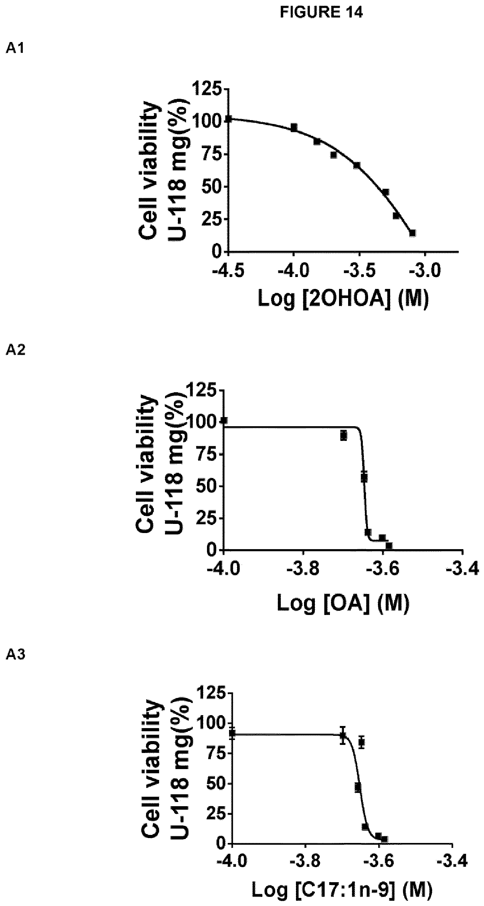

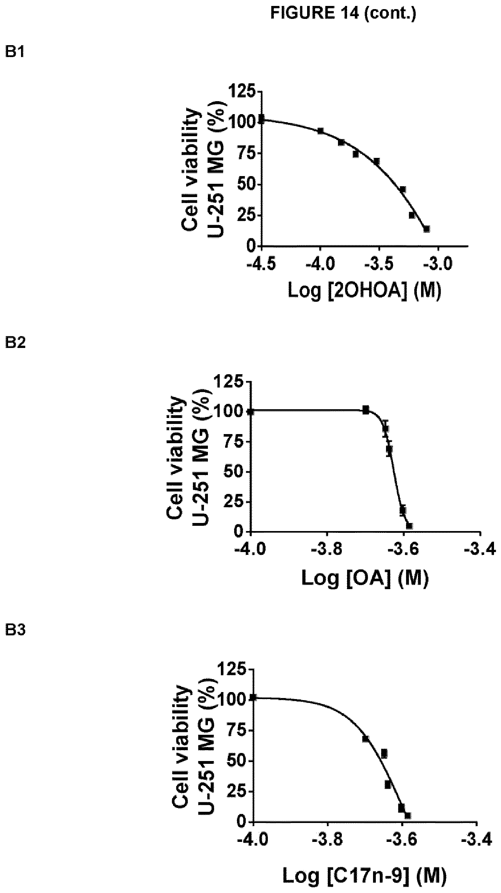

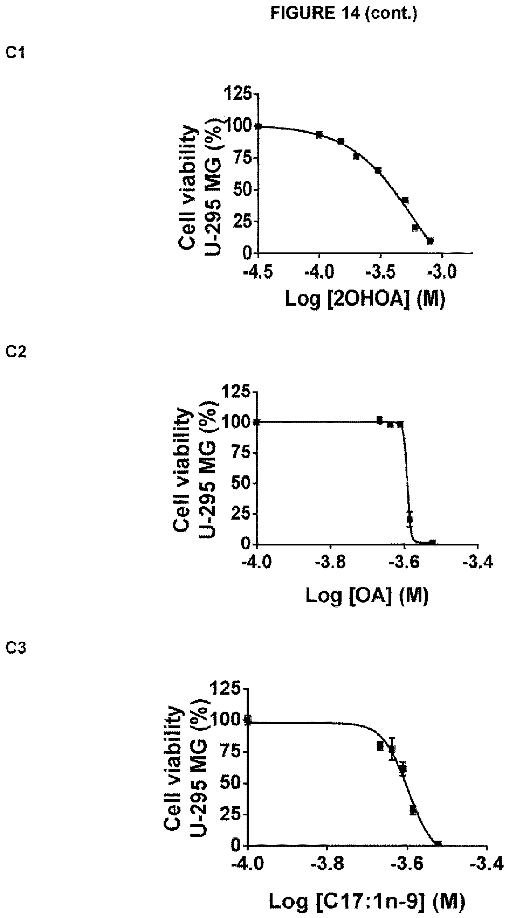

**Figure 14****.** Effect of 2OHOA sodium salt, OA and C17:1n-9 sodium salt on cell viability and proliferation of glioma cells. Viability curves of different glioma cell lines **(A1-A3)** U-118 MG; **(B1-B3)** U-251 MG; and **(C1-C3)** SF-295 treated with increasing doses of 2OHOA (0-1000 µM) sodium salt (A1, B1, and C1); OA (0-300 µM) (A2, B2, and C2); and C17:1n-9 (0-300 µM) sodium salt (A3, B3, and C3) for 72 hours. Viability was determined by violet crystal staining. Each value represents the mean ± SEM of three independent experiments with at least three biological replicates, expressed as a percentage with respect to the cells treated with vehicle (100%).

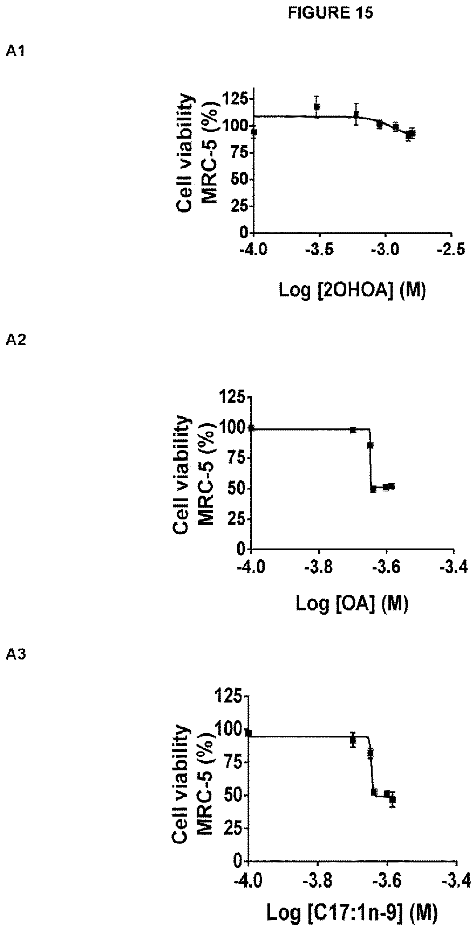

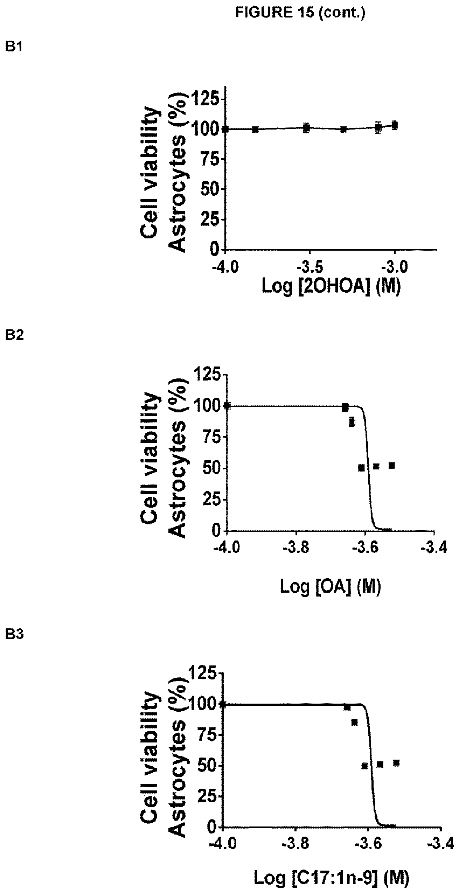

**Figure 15****.** Effect of 2OHOA sodium salt, C17:1n-9 sodium salt, and OA on cell viability and proliferation of non-tumor cells. Non-tumor cell viability curves **(A1-A3)** MRC-5 (human fibroblasts); and **(B1-B3)** mouse astrocytes treated with increasing doses of 2OHOA sodium salt (0-1000 µM) (A1 and B1); OA (0-300 µM) (A2 and B2); and C17:1n-9 sodium salt (0-300 µM) (A3 and B3) for 72 hours. Viability was determined by violet crystal staining. Each value represents the mean ± SEM of three independent experiments with at least three biological replicates, expressed as a percentage with respect to the cells treated with carrier (100%).

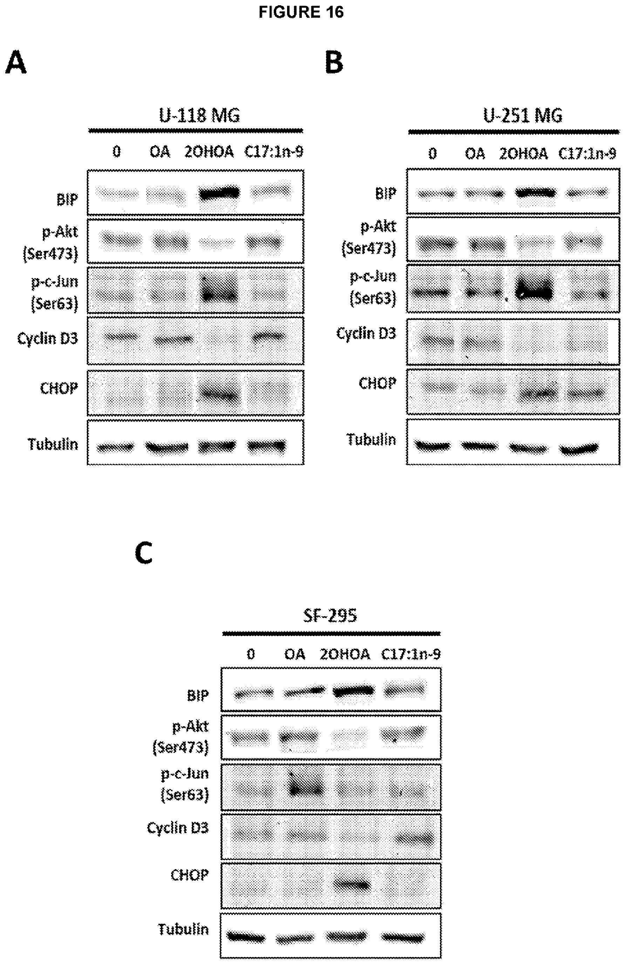

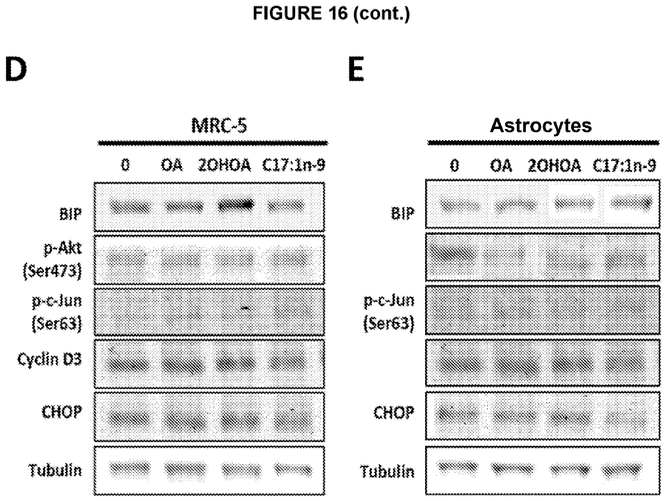

**Figure 16****.** Analysis of the effect of different fatty acids on markers of proliferation and death in different cell lines. Immunoblots representative of the effect of fatty acids (200 µM OA, 200 µM C17:1n-9 sodium salt and 400 µM 2OHOA sodium salt) on various proteins involved in the 2OHOA-regulated cell death and signaling pathways in glioma cells: **(A)** U-118 MG; **(B)** U-251 MG; and **(C)** SF-295; and non-tumor: **(D)** MRC-5 (human fibroblasts); and **(E)** mouse astrocytes, after 72h of treatment.

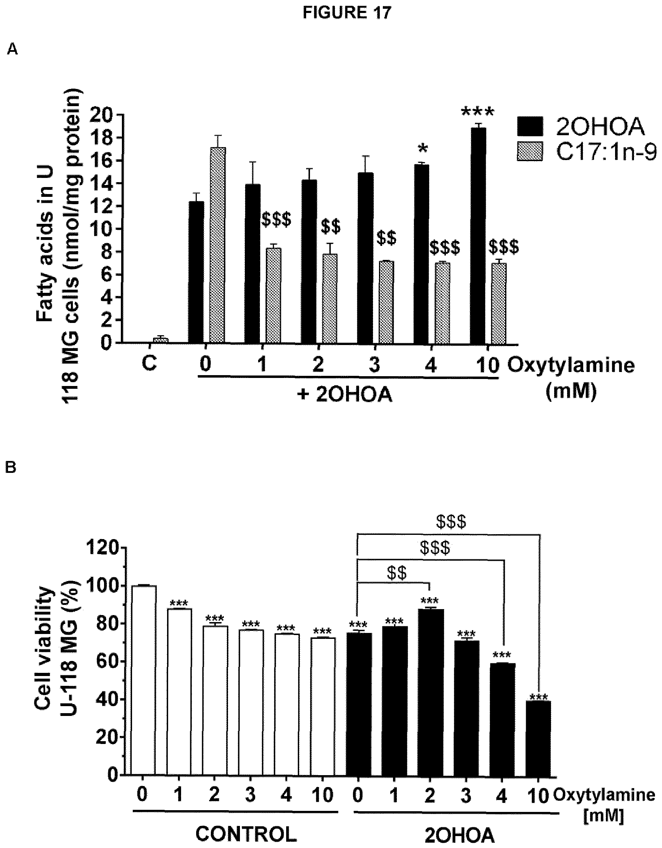

**Figure 17****.** Analysis of the composition of fatty acids in U-118 MG glioma cells after inhibition of α-oxidation and effect of oxythiamine on the cell survival of U-118 MG glioma cells. **(A)** Quantification of 2OHOA and C17:1n-9 fatty acids in U-118 MG cells treated with 400 µM 2OHOA for 24 hours, pre-incubated with increasing doses (1-10 mM) of oxythiamine (a-oxidation inhibitor) for 90 minutes, determined by gas chromatography. Results are shown as the mean ± SEM of three independent experiments expressed in nmoles and normalized per mg protein. The statistical significance is determined with a Student's t test (*p<0.05, ***p<0.001 comparing the amount of 2OHOA with that detected after 400 µM of 2OHOA in the absence of oxythiamine; $$p<0.01, $$$p<0.001 comparing the amount of C17:1n-9 with that formed after 400 µM of 2OHOA in the absence of oxythiamine). **(B)** Viability of U-118 MG cells pre-incubated with oxythiamine (for 90 minutes) and treated in the absence (Control) or in the presence of 2OHOA sodium salt (400 µM, 72 hours), determined by vital exclusion staining with trypan blue. The results are represented as the mean cell count ± SEM of three independent experiments. Statistical significance is determined with a Student's t test (***p<0.001 with respect to the absence of 2OHOA and oxythiamine, Control-0; and $$p<0.01, $$$p<0.001 with respect to treatment with 2OHOA without pre-incubation with oxythiamine).
**Figure 18****.** Effect of metabolite C17:1n-9 on the action of 2OHOA. Viability of different human glioma cell lines: **(A)** U-251 MG and **(B)** SF-295; and non-tumor cells: **(C)** human MRC-5 fibroblasts and (D) mouse astrocytes; all treated in the absence or presence of 2OHOA sodium salt (400 µM, 72 hours) and pre-incubated or not with oxythiamine (for 90 minutes). Cell viability was determined by vital exclusion staining with trypan blue. The results are represented as the mean cell count ± SEM of three independent experiments. Statistical significance is determined with a Student's t test (**p<0.01 and ***p<0.001 with respect to the absence of 2OHOA and oxyamine; and $p<0.05 with respect to treatment with 2OHOA alone).
**Figure 19****.** Analysis of the effect of the metabolite C17:1n-9 on the action of 2OHOA in markers of proliferation and death in different cell lines by inhibition of its formation by oxythiamine. Immunoblots representative of the effect of 2OHOA (400 µM) combined or not with oxythiamine (2 mM) on various proteins involved in the 2OHOA-regulated cell death and signaling pathways in glioma cells: **(A)** U-118 MG; **(B)** U-251 MG; and **(C)** SF-295; and non-tumor: **(D)** MRC-5 (human fibroblasts); and **(E)** mouse astrocytes, after 72h of treatment.
**Figure 20****.** Analysis of the composition of fatty acids in rat plasma after 24 hours of treatment with 2OHOA sodium salt. **(A)** Representative chromatograms showing the composition of fatty acids in rat plasma samples obtained at different times (0, 1, 2, 3, 4, 6, 8 and 24 hours) after acute treatment with 2OHOA (2mg/Kg, 24 hours) determined by gas chromatography. C17:0 margaric acid was quantified as internal control in the chromatogram. **(B)** Quantification of the 2OHOA and C17:1n-9 fatty acids identified in the chromatograms. Results are shown as the mean ± SEM of 4 animals and expressed in nmoles and normalized per ml of plasma. Statistical significance is determined with a Wilcoxon test (*p<0.05 and **p<0.01 with respect to baseline levels at 0 hours; $p<0.05 and $$p<0.01 with respect to 2OHOA fatty acid levels).
**Figure 21****.** Analysis of the composition of fatty acids in rat plasma after 15 days of treatment with 2OHOA sodium salt. **(A)** Representative chromatograms showing the composition of fatty acids in rat plasma samples obtained at different times (0, 1, 2, 3, 4, 6, 8 and 24 hours) after chronic treatment with 2OHOA (2mg/Kg, 15 days) determined by gas chromatography. C17:0 margaric acid was quantified as internal control. **(B)** Quantification of the 2OHOA and C17:1n-9 fatty acids identified in the chromatograms. Results are shown as the mean ± SEM of 4 animals, expressed in nmoles and normalized per ml of plasma. Statistical significance is determined by a Wilcoxon test (*p<0.05 and **p<0.01 with respect to baseline levels at 0 hours; $p<0.05 and $$p<0.01 with respect to 2OHOA fatty acid levels).
**Figure 22****.** Analysis of the composition in fatty acids of xenographic tumors of immunosuppressed mice. **(A)** Representative chromatograms showing the composition of fatty acids in xenographic tumors originating from U-118 MG glioblastoma cells in mice treated orally and daily with 2OHOA sodium salt (200 mg/kg, 42 days) determined by gas chromatography. **(B)** Quantification of the OA and C17:1n-9 fatty acids identified in the chromatograms. C17:0 margaric acid was quantified as internal control. The white bar corresponds to the concentration of each fatty acid in the control, and the black bar corresponds to the concentration of the fatty acid after treatment with 2OHOA sodium salt. Results are shown as the mean ± SEM of at least 7 xenographic tumors and expressed in nmoles and normalized per g of tissue. Statistical significance is determined by a Mann-Whitney test (* **p<0.01 with respect to control).
**Figure 23.** Inverse correlation between tumor volume and amount of C17:1n-9 metabolite. Representation of the amount of metabolite quantified by gas chromatography in xenographic tumours of mice, relative to the tumour volume measured on day 42 of treatment with 200 mg/kg sodium salt of 2OHOA (black boxes) or its vehicle (Control, white circles). Significance determined by Pearson's correlation coefficient (p = 0.0001; r = -0.825).
**Figure 24****.** Analysis of composition in fatty acids in human patients with advanced glioma. **(A)** Representative chromatogram of the fatty acid composition of a patient with glioma responsive to treatment with 2OHOA sodium salt (12 g/day, 21 days) and determined in plasma samples obtained at different treatment times (0, 4 and 360 hours, 15 days) by gas chromatography. **(B)** Quantification of 2OHOA and C17:1n-9 fatty acids identified in the chromatograms of 2OHOA responders and non-responders in plasma samples obtained at different times on the first day of treatment (0, 1, 2, 4, 6, 8 hours) and on days 8 (192 hours), 15 (360 hours), 21 (504 hours) and the first day of the second treatment cycle (574 hours). **(C)** Quantification of the 2OHOA and C17:1n-9 fatty acids identified in the chromatograms of the same responding and non-responding patients jointly. The C17:0 margaric acid in the chromatograms was quantified as internal control. Results are shown as the mean ± SEM of 8 patients (4 responders and 4 non-responders) and expressed in nmoles and normalized per ml of plasma. Statistical significance is determined by a Mann-Whitney test (*p<0.05 and **p<0.01 relative to the amounts of 2OHOA fatty acid).

### EXAMPLES

The examples described below are for purposes of illustration only and are not meant to limit the scope of the present invention.

### Example 1: Fatty acids, reagents and organic solvents

### 1.1. DHA, DHA-H and HPA

DHA (sodium salt of docosahexaenoic acid; C22:6 n-3), DHA-H (sodium salt of 2-hydroxy-drocosahexaenoic acid; 2OH-C22:6 n-3), EPA-H (sodium salt of 2-hydroxy-eicosapentaenoic acid), ARA-H (sodium salt of 2-hydroxy-arachidonic acid), GLA-H (sodium salt of 2-hydroxy-gamma (γ)-linolenic acid), ALA-H (sodium salt of 2-hydroxy-alpha (a)-linolenic acid), LA-H (2-hydroxy-linoleic acid), HPA (sodium salt of (6Z,9Z,12Z,15Z,18Z)-heneicosa-6,9,12,15,18-pentaenoic acid), NTA (sodium salt of (4Z,7Z,10Z,13Z)-nonadeca-4,7,10,13-tetraenoic acid), HTA ω-6 (sodium salt of (5Z,8Z,11Z)-heptadeca-5,8,11-trienoic acid), HTA ω-3 (sodium salt of (8Z,11Z,14Z)-heptadeca-8,11,14-trienoic acid) and HDA ((8Z,11Z)-heptadeca-8,11-dienoic acid) were obtained from Lipopharma Therapeutics (Spain). The margaric acid (C17:0) was purchased from Sigma-Aldrich and the heneicosapentanoic acids (HPA free acid; C21:5 n-3) and (4Z,7Z,10Z,13Z,16Z)-nonadeca-4,7,10,13,16-pentaenoic acid (NPA free acid; C19:5 ω-3) were purchased from Cayman Chemicals (Michigan, United States). The D(+)-Glucose (cell culture tested), sodium pyruvate, L-Gln (cell culture tested), acetyl chloride and N,O-bis (trimethylsilyl) acetamide, sodium chloride, sodium phosphate, EDTA (ethylene diamine tetraacetic acid) and tris-base were acquired from Sigma-Aldrich. In contrast, chloroform, ethanol, methanol, hydrochloric acid and hexane were obtained from Scharlab (Spain). Heparin (5000 units/mL) was purchased from Hospira Invicta S.A. (Spain), ketamine (Anesketin 100 mg/mL) from Eurovet Animal Health BV (Netherlands), xylazine (Xilagesic 20 mg/mL) from Laboratories Calier S.A. (Spain), and oxythiamine hydrochloride from Santa Cruz Biotechnology (Germany).

For the production of HPA, chemical synthesis is performed from the (5Z,8Z,11Z,14Z,17Z)-eicose-5,8,11,14,17-pentaenoicacid (EPA (C20:5, ω-3)), according to reaction scheme 1. The chemical synthesis of HPA is disclosed in the prior art (Larsen et al., 1997, Lipids 32(7), 707-714. doi: 10.1007/s11745-997-0090-4). The reactions were carried out in the absence of light and in a nitrogen atmosphere.

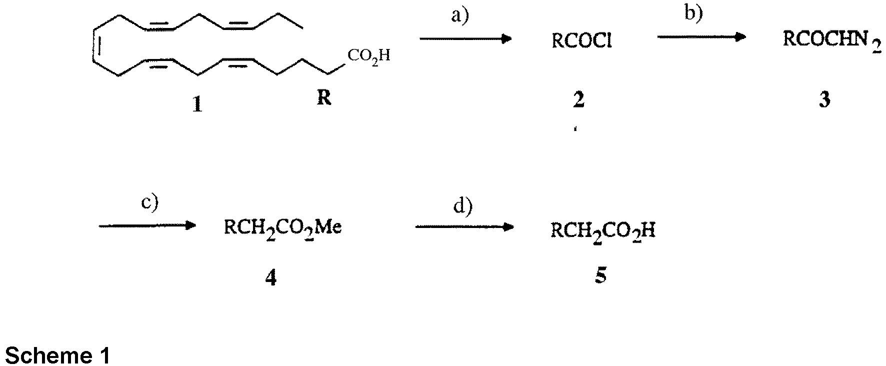

Reagents and conditions: a)(COCl)₂/PhH 1.5h rt., b)CH₂N₂/ether 20 min. 0°C, c)AgOBz(cat.), Et₃N/THF/H₂O

The synthesis of the sodium salt of the HPA of the present invention has been made from the compound designated with the number 5 when R is CH₃-CH₂-(CH=CH-CH₂)₅-CH₂CH₂-, which corresponds to HPA (C₂₁). The salt is obtained under an acid base reaction, a liquid-liquid extraction is performed with MTBE/HCI and the pH is adjusted with NaOMe to obtain the sodium salt of HPA with good yields. A similar procedure can be performed for the synthesis of HDA, HTA ω-3, HTA ω-6, NTA, and NPA, by adjusting the starting substrate.

### 1.2. OHOA, OA and C17:1n-9

The lipid compounds sodium salt of 2OHOA, sodium salt of OA and sodium salt of C17:1n-9 were purchased from Medalchemy, SL (Spain).

The chemical synthesis of C17:1n-9 is disclosed in WO1997049667. A solution of 8Z-heptadecene (66mg, 0.26mmol, 1 equivalent) and 2-methyl-2-butane (1.6mL, 15.1 mmol, 58 equivalents) in tBuOH (6.5mL) at 25°C, under an N₂ atmosphere, is treated by adding dropwise (2.5mL) a solution of NaClO₂ (80%, 208mg, 2.3 mmol, 9 equivalents) and NaH₂PO₄.H₂O (250mg, 1.8 mmol, 7 equivalents) in deionized water. The reaction mixture was allowed to stir for an additional 15 minutes, before being concentrated in vacuo. The residue is treated with water (30 mL) and the aqueous layer is extracted with EtOAc (3x30 mL). The organic layers are dried with (Na₂SO₄), filtered and concentrated in vacuo. Chromatography (SiO₂, 2x13 cm, 10-20% EtOAc-hexane gradient elution) afforded 27 mL (66 mg, 95%) as a clear oil. The synthesis of the sodium salt of C17:1n-9 of the present invention has been made from compound C17:1n-9. The salt is obtained under an acid base reaction, a liquid-liquid extraction is performed with MTBE/HCI and the pH is adjusted with NaOMe to obtain the sodium salt of C17:1n-9 with good yields.

### Example 2: Compositions with DHA-H and HPA

Some examples of compositions that do not limit the scope of the invention are described in general terms below.

**Table 1. Example Topical Use Formulation**

| Component | Composition %w/W | Composition %w/W | Composition %w/W |
|---|---|---|---|
| DHA-H | 3.6 | 0 | 1.3 |
| HPA | 0 | 3.6 | 1.3 |
| DMSO | 80.0 | 80.0 | 80.0 |
| Water | 16.4 | 16.4 | 16.4 |
| Total | 100 | 100 | 100 |

**Table 2. Example oral formulation**

| Component | Composition %w/W | Composition %w/W | Composition %w/W |
|---|---|---|---|
| DHA-H | 5 | 0 | 2.5 |
| HPA | 0 | 5 | 2.5 |
| Ethanol (96%v/v) | 5 | 5 | 5 |
| Water | 90 | 90 | 90 |
| Total | 100 | 100 | 100 |

**Table 3. Example oral formulation soft capsule**

| Component | Composition %w/W |
|---|---|
| HPA | 63.3 |
| Triglycerides | 25.5 |
| Glyceryl monostearate | 6.66 |
| Aroma | 2.22 |
| Dismutase superoxide | 1.11 |
| Colloidal silica | 1.11 |
| Total | 100 |

### Example 3: Cellular assays with DHA-H and HPA

To describe the metabolic conversion of DHA-H to HPA(C21:5 ω-3), as well as the conversion of LA-H to HDA (C17:2 ω-6), ALA-H to HTA ω-3 (C17:3 ω-3), GLA-H to HTA ω-6 (C17:3 ω-6), ARA-H to NTA (C19:4 ω-6), EPA-H to NPA (C19:5 ω-3), HEK293T (Human Embryonic Kidney Cells 293T) cell cultures were employed, which is an embryonic, non-tumoral cell line, widely used in human metabolism studies under physiological conditions.

HEK293T cells were cultured in Dulbecco's Modified Eagle's Medium (DMEM; Dubelcco's Modified Eagle's Medium, Biowest, France), supplemented with 10% FBS (Fetal Bovine Serum; Gibco, Thermo-Fisher), 2 mM L-Gln, 25 mM D(+)-glucose, 1 mM sodium pyruvate, and penicillin/streptomycin. Mouse neuroblastoma N2a cells were maintained in a 1:1 (v:v) mixture of DMEM and Opti-Mem (Gibco, Thermo-Fisher), supplemented with 5% FBS and penicillin/streptomycin. Both cell lines were incubated in an atmosphere of 5% CO₂ at 37°C.

HEK293T cells were incubated with DHA-H and DHA at 10, 30 and 100 µM for 24 hours, and at 30 µM for 6, 48 and 72 hours. These cells were also incubated with LA-H, ALA-H, GLA-H, ARA-H and EPA-H at 100 µM for 24 hours. HEK293T cells were also incubated with oxythiamine in the presence of DHA-H under the same conditions, at final oxythiamine concentrations of 1 and 10 mM. The HEK293T cells were separated from the plates by pipettes with cold phosphate buffer saline solution (PBS). Cells were recovered by centrifugation (1000 xg, 10 min at 4°C) and washed twice with cold PBS before being frozen at -80°C. To analyze DHA-H and DHA levels in the cell culture medium, 90 mm diameter plates were filled with 11 mL of complete cell culture medium containing 30 µM DHA-H or DHA in the presence or absence of attached HEK293T cells (5.10⁵ cells/plate). The plates were incubated as described above and 1 ml aliquots of the plates were collected at 0, 6, 24, 48 and 72 hours. Aliquots of the cell culture medium were immediately centrifuged at 1000 xg for 10 min at 4°C to remove any cell suspension and the cell-free aliquots were stored at -20°C.

U-118 MG, MIA-PaCa 2 and A549 cell lines were obtained from the European Collection of Cell Cultures (ECACC) via Sigma-Aldrich Co (St Louis, MO) and maintained in RPMI (Roswell Park Memorial Institute) culture medium (U-118 MG and A549) or DMEM (MIA-PaCa 2) supplemented with 10% FBS (Gibco, Thermo-Fisher), in an atmosphere of 5% CO₂ at 37°C. U-118 MG, MIA-PaCa 2, and A549 cells were treated under the conditions described in the description of the assay performed to obtain the results of table 4, eventually in the presence or absence of oxytymine (1 or 10 mM). Cell survival was analyzed in a Bürker chamber using trypan blue vital exclusion staining (Scharlab) or by cell proliferation kit II (Roche). Briefly, the cells were seeded in 96-well plates at a density of 3 × 10³ cells per well 24 h prior to treatment, and then cultured in the presence or absence of compounds of interest at the concentrations and for the times indicated in the figures. After different times, plaque viable cells were determined by the addition of XTT according to the manufacturer's instructions. Cells were incubated at 37°C in 5% CO₂ until a constant color was developed and absorbance was recorded at 495 nm using a microplate reader with a reference wavelength of 650 nm (FLUOstar Omega, BMG LABTECH, Germany).

SH-SY5Y human neuroblastoma cells were maintained in DMEM-F12 (Invitrogen) supplemented with 10% FBS (Sigma), penicillin/streptomycin (PAA), non-essential amino acids (Sigma), and 2 mM L-Gln (Sigma). Differentiation of these cells to a neuronal phenotype was carried out following a standard procedure. Briefly, the cells were seeded on plates pretreated with poly-L-lysine and 24 h later, the medium was replaced by a fresh medium supplemented with 10 µM retinoic acid (Sigma). The cells were then incubated in the dark for 5 days and the medium was replaced with a serum-free medium and supplemented with 50 ng/ml of human brain-derived neurotrophic factor (hBDNF; Alomone Labs; Tel Aviv, Israel). Finally, cells were incubated for 6 days to complete differentiation. Neurons were treated for 24 h with the compounds HDA, HTA ω-3, HTA ω-6, NTA, NPA and HPA, at 1.3 and 10 µm, for 24 h, prior to induction of excitotoxicity with NMDA (n-Methyl-D-Aspartate, 10 mM, Sigma) in a medium containing glycine (530 µM, Sigma) and calcium (10 mM, Sigma).

Treatment with DHA-H results in high cellular levels of HPA, compared to prodrug levels in cell cultures (figure 1B). In figure 1B, intracellular levels of DHA-H and HPA are shown in HEK293T cells under treatment with DHA-H. The accumulation of both compounds is evident as a function of treatment concentration or incubation time, but HPA levels are significantly higher than those of the prodrug from 24 hours of incubation and 30 µM of treatment. The increase in HPA levels is inhibited in the presence of concomitant treatment of oxythiamine (partial inhibition with 1 mM and almost total with 10 mM), a competitive antagonist of 2-hydroxyacyl-CoA lyase (see figure 1A). In this regard, it has also been found that endogenous levels of DHA (the non-hydroxylated native form) are not altered by this treatment with DHA-H (figure 1C).

Similarly, figure 8 shows that this same metabolic pathway is valid for other 2-hydroxylated polyunsaturated fatty acids, employed as prodrugs, such as LA-H, ALA-H, GLA-H, ARA-H, and EPA-H, resulting in HDA, HTA ω-3, HTA ω-6, NTA, NPA, respectively (chromatograms shown in figure 9). All of these metabolites have demonstrated therapeutic activity, as shown in **figure10** and table 4 below:

**Table 4. IC50 values in human glioblastoma (U118 MG), pancreatic cancer (MIA-PaCa 2) and human lung adenocarcinoma (A549) cell lines**

| | **IC50 (µM)** | | |
|---|---|---|---|
| | **U118 MG** | **MIA-PaCa 2** | **A549** |
| **HDA (C17:2 n-6)** | 144 ± 32 | 234 ± 18 | 159 ± 10 |
| **HTA n-3 (C17:3 n-3)** | 129 ± 03 | 216 ± 24 | 166 ± 30 |
| **HTA n-6 (C17:3 n-6)** | 235 ± 26 | 95 ± 31 | 212 ± 48 |
| **NTA (C19:4 n-6)** | 113 ± 13 | 62 ± 02 | 216 ± 03 |
| **NPA (C19:5 n-3)** | 90 ± 09 | 91 ± 18 | 143 ± 39 |
| **HPA (C21:5 n-3)** | 124 ± 16 | 58 ± 17 | 228 ± 03 |

The anti-tumor activity of the different metabolites described in figures 8 and 9 was determined by direct treatment with these molecules (HDA or C17:2 ω-6, HTA w-3 or C17:3 ω-3, HTA ω-6 or C17:3 ω-6, NTA or C19:4 ω-6, NPA or C19:5 ω-3, and HPA or C21:5 ω-3) in tumor cell cultures, on which the IC50 value for each of these compounds was determined (Inhibitory Concentration 50: concentration of study compound that induces death of 50% of the tumor cell population). The cell cultures used correspond to different types of cancer: U118-MG (human grade IV glioblastoma), MIA-PaCa 2 (pancreatic carcinoma) and A549 (small cell lung adenocarcinoma). The different compounds showed variable IC50 values on the different tumor lines, demonstrating the selectivity of some of them to induce the selective death of certain types of tumor cells.

### Example 4: In vivo trials with DHA-H and HPA

The 5xFAD model of Alzheimer's disease is a dual transgenic PS1/APP mouse that harbors five human mutations associated with familial AD (Tg6799 line): Swedish (K670N/M671L), Florida 151(I716V) and London (V717I) in APP; and clinical mutations M146L and L286V in human PS1. Both transgenes are expressed under the control of the Thy-1 promoter and mice show cognitive decline from 4 months of age (Oackley et al., 2006, Neurosci 26(40), 10129-10140. doi: 10.1523/jneurosci.1202-06.2006). 5xFAD transgenic animals and wild type (WT) were obtained from Jackson Laboratories (USA) and maintained in a B6/SJL genetic background by crossing heterozygous transgenic mice with B6/SJL WT (F1) reproducers. The animals were housed at a controlled temperature of 22°C (±2°C) and a humidity of 70%, in a 12h-12h light-dark cycle, with free access to a standard laboratory diet (Panlab A03, Barcelona, Spain).

Transgenic male WT and 5xFAD mice received DHA-H (or DHA) orally, dissolved in 5% ethanol, at a daily dose of 5, 20, 50 and 200 mg/kg, or vehicle alone. On the other hand, in an independent assay, these animals have also been treated with HPA (20 mg/kg) and DHA-H (20 g/kg) to compare the effect of both compounds in this model. These treatments were initiated when the mice reached 3 months of age (dosed 5 days/week) and continued until 7 months of age. During the last month of treatment, all animals were kept on a hypocaloric diet to perform selected behavioral spatial learning and memory testing (radial arm maze). A total of 46 animals were used for the study shown in figure 2: WT treated with vehicle (n=3), WT treated with DHA-H (20 mg/kg, n=3; and 200 mg/kg, n=3), WT treated with DHA(20 mg/kg, n=3); 5xFAD (n=5) treated with DHA-H (5 mg/kg, n=6; 20 mg/kg, n=5; 50 mg/kg, n=6; and 200 mg/kg, n=7), and 5xFAD (20 mg/kg, n=5) treated with DHA. A total of 20 animals were used in the study of **figure 6****:** WT treated with vehicle (n=5), 5xFAD treated with vehicle (n=5), 5xFAD treated with DHA-H (20 mg/kg, n=5) and HPA (20 mg/kg, n=5). After the behavioral test, mice were maintained on a normal diet (and treatment) for an additional week, after which they were anesthetized with an intraperitoneal injection of ketamine/xylazine (100/10mg/kg) and infused intracardiacally with 50 mL of heparinized saline. Animal brains were immediately removed and dissected by the midline on a cold surface. Once the cerebellum was removed, each cerebellum-free half was frozen in liquid nitrogen and stored at -80°C. NUDE (Swiss) Crl:NU (Ico) -Foxn1 ^{nu} mice (8-12 weeks old, 30-35 g, Charles River Laboratories, Paris, France) were maintained in a thermostatic cabinet (28°C, EHRET, Labor-U-Pharmatechnik) with a sterile air flow at a relative humidity of 40-60% and with 12-hour dark/light cycles. Their diet consisted of a standard diet with feed (Labdiet 22% rat-mouse breeding, Sodispan) *ad libitum.* To cause xenographic tumors, 7.5 × 10⁶ U-118 MG cells were inoculated subcutaneously on both sides of the animal's dorsal flank and after one week the tumors became visible with an approximate volume of 100 mm³. Animals were randomly divided into groups with a similar mean tumor volume and received daily oral treatments for 42 days: vehicle, DHA-H (200 mg/kg) and HPA (200 mg/kg). The study of **figure 3** comprises animals treated with vehicle (untreated controls; n=6) and treated with DHA-H (200 mg/kg; n=9). **Figure 7** **shows** animals treated with vehicle (untreated controls; n=6), treated with DHA-H (200 mg/kg; n=8) and treated with HPA (200 mg/kg; n=8). Tumor volumes (v) were calculated as v = A² × L/2, where A is the width of the tumor and L is its length. Upon completion of treatment, mice were sacrificed by cervical dislocation and xenographic tumors were dissected and frozen in liquid nitrogen and at -80°C. All the protocols used were approved by the Bioethics Committee of the University of the Balearic Islands, and comply with national and international guidelines on animal welfare. In the use of healthy mice and transgenic models of Alzheimer's disease (5xFAD), it was found that there was a significant accumulation of HPA at the brain level, while the parent molecule (DHA-H) could not be detected, nor changes in the endogenous levels of DHA (non-hydroxylated native form) **(figure 2A).**

### Radial arm maze test

The spatial behavior test was performed as described above, with some modifications Fiol-Deroque (et al., 2013, Biogerontology 14(6), 763-775. doi: 10.1007/s10522-013-9461-4). All animals were isolated and subjected to caloric restriction to 80-85% of normal body weight, and were kept in these conditions for one week before starting the test and until the end of the test. After the dietary restriction and 3 days before the start of the trials, the animals were trained twice a day in the eight-arm radial labyrinth test (LE766/8, Panlab SL, Spain) for 3 days. Each mouse was placed in the center of the maze and allowed to seek the reward, a 45 mg food pellet (Dustless Precision Pellets, Bio-Serv, USA), located at the end of each arm. Each session ended when the animal managed to find the eight primed arms or failed to complete all the arms in 10 minutes. The movement of each animal was recorded with a digital video tracking system (LE 8300 with Sedacomv1.3 software, Panlab, SL, Spain) and after training, the experimental paradigm began. In all experimental sessions (1 session per day), only four arms were primed compared to the training protocol, and each session ended when the animals managed to find all four primed arms or failed after 10 minutes. The performance was evaluated taking into account: (1) the time to perform the test; (2) the number of Working Memory Errors (WME, re-entry into a previously visited primed arm); (3) the number of Reference Memory Errors (RME, entry into a non-primed arm); and (4) the total number of errors (WME+RME). The test was repeated 5 days/week for 3 weeks. After the test, the animals were fed *ad libitum* for an extra week before slaughter.

In this sense, it can be observed that the levels of HPA at the brain level in the Alzheimer's model mice have a statistically significant inverse relationship with behavioral parameters in an evaluation test of spatial and associative memory (radial labyrinth test) (figure **2B).** These results suggest that moderate increases in brain HPA levels are significantly related to an improvement in spatial cognition. Likewise, direct administration of HPA has similar effects to administration of DHA-H on the parameters of the same behavioral parameters analyzed **(****figure 6****).**

### Example 5: Lipid extraction and fatty acid transmethylation relating to Examples 3 and 4

The HEK293T or U-118 MG cells used in the above examples were lysed with a cold hypotonic buffer (1 mM EDTA, 20 mM Tris-HCI [pH 7.4]) by pipetting up and down. The cell lysates were subjected to ultrasound pulses (4 cycles, 10 s/cycle, 10W) before lipid extraction. For brain analysis, the tissue of each animal was homogenized in cold PBS at 1:10 (p:v) in the presence of protease inhibitors (Roche), using a blade homogenizer (Polytron PT3100). Homogenates were ultrasounded, aliquots were made and stored at -80 °C. Only one aliquot of each sample, containing about 8 mg protein/aliquot, was subjected to lipid extraction. Protein content before lipid extraction was determined by a modified Lowry method (Bio-rad DC Protein Assay).

Margaric acid (C17:0) was added to the samples subjected to lipid extraction as an internal standard and the lipids were extracted with chloroform:methanol (Eggers and Schwudke, 2016). Briefly, 0.75 volumes of the aqueous phase (which already contained the biological sample) were mixed with 2 volumes of chloroform and 1 volume of methanol. This mixture was vortexed for 1 minute and centrifuged at 1000 xg for 10 minutes. The lower organic phase was collected and washed with 1 ml of PBS:methanol (1:1,v:v), and the resulting organic phase was dried under argon flow. The film containing the extracted lipids was transmethylated by incubation of the lipid mixture for 90 minutes at 100°C in 3 ml of methanol:acetyl chloride (10:1, v:v), under an argon atmosphere (Christie, 1993). The resulting fatty acid methyl esters (FAMEs) were extracted with hexane, adding 3 ml of H₂O and 1 ml of hexane to the transmethylation reaction, and vortexing the mixture thoroughly. After centrifugation at room temperature (1000 xg for 10 min), the upper phase containing the FAMEs was collected and the remaining volume was washed twice with 1 ml of hexane. The hexane phases were combined, evaporated under argon flow and resuspended in 60 µl of hexane (for the analysis of cell samples, cell culture medium and blood plasma) or in 200 µl (for the analysis of brain samples). To check if a fatty acid compound is hydroxylated, isolated FAME were subjected to a second derivatization with trimethylsilyl (Alderson et al., 2004, J Biol Chem 279(47), 48562-48568. doi: 10.1074/jbc.M406649200). Briefly, the FAMEs were dried under argon flow and the lipid film was dissolved in N,O-bis (trimethylsilyl) acetamide (0.1-5.0 mg lipid for 200-400 µl trimethylsilylation reagent), which in turn was heated in a capped vial at 70°C for 30 min. The solvent was evaporated and the lipid film was resuspended in hexane for analysis. When the fatty acid under study is hydroxylated, the retention time of the FAME changes as a result of this second derivatization. However, if the fatty acid under study is not hydroxylated, the resulting FAME shows the same retention time regardless of the second derivatization.

The levels of HPA generated from the treatment with the prodrug DHA-H in these cells were evaluated, in the presence or absence of oxythiamine (competitive inhibitor of α-oxidation) (figure 4A). The results showed that the addition of DHA-H to a U-118 MG cell culture results in a significant increase in HPA levels. This increase is inhibited in the presence of simultaneous treatment with 1 or 10 mM oxythiamine, demonstrating that the transformation of DHA-H into HPA is mediated by α-oxidation. Treatment with DHA-H on U-118 MG cells in culture had no effect on endogenous levels of DHA (non-hydroxylated native form). On the same cells in culture, viability assays were carried out with DHA-H in the presence or absence of 1 mM oxythiamine (figure 4B), proving that 1 mM oxythiamine has no effect on cell viability.

On the other hand, the addition of DHA-H (150 µM, 48 h) presents a significant anti-proliferative effect on U118-MG cells. However, this effect is partially reversed (in a statistically significant manner) in the presence of 1 mM oxythiamine. At this time, it should be remembered that this concentration of oxythiamine is sufficient to completely inhibit the increase in HPA levels from DHA-H. These results then show that the anti-proliferative effect mediated by DHA-H on U-118 MG cells is mediated, at least in part, by HPA, since the inhibition of the formation of this compound from DHA-H translates into a lower anti-proliferative effect of DHA-H (figure 4B). The anti-proliferative effect that HPA has on a culture of U-118 MG cells was also studied, compared to the administration of the DHA-H prodrug and the native form of DHA. The antiproliferative effect on U-118 MG is much higher for HPA relative to DHA-H and DHA (see figure 5A). When compared to DHA-H, this effect can be explained by differences in intracellular levels of HPA, induced by DHA-H and HPA (see figure 5B). Indeed, FIG. 5C shows that uptake of the hydroxylated form of DHA is prevented compared to that of the non-hydroxylated analogue.

### Example 6: In vitro assays with 2OHOA and C17:1n-9

The concentrations of 2OHOA sodium salt used in the experiments described below and the duration of the treatments varied according to the type of assay, being either 200 µM or 400 µM and 24 or 72 hours. In some experimental series, C17:1n-9 sodium salt solutions were used at a concentration of 200 µM for 24 or 72h.

To prepare these solutions, we started from a stock aliquot at 100 mM. To prepare this starting aliquot, the corresponding milligrams of the lipid compound (powder) were dissolved in absolute ethanol and autoclaved distilled water (vol.1:1, normally an aliquot of 1ml is prepared so that 500 µl of ethanol and 500 µl of water are added) inside the culture hood, the solution was introduced 10 min into the culture oven at 37°C so that the lipid compound was dissolved and subsequently subjected to stirring.

### 6.1. Incorporation and metabolization of 2OHOA U-118 MG glioma and non-tumor cells

To confirm the incorporation of 2OHOA into glioma cell membranes and to determine if changes in fatty acid profile occur following treatment with 2OHOA, total lipids were analyzed by gas chromatography on U-118 MG human glioma cells incubated in the absence (control) or presence of 400 µM 2OHOA sodium salt for 24h. Analysis of fatty acid levels in glioma cells revealed an absence of changes in OA levels following treatment with 2OHOA sodium salt relative to control (figure 12). In addition, cell incorporation of 2OHOA was observed after 24 hours of treatment due to the identification of a peak in the chromatogram, only in treated cells, which corresponded to its standard. On the other hand, it is worth noting the appearance of a new peak, practically exclusively, in the chromatogram of the treated cells. Elevated levels of this new peak were detected in the treated cells, accumulating almost double (19.71 ± 0.39 nmol/mg protein) than 2OHOA (10.81 ± 0.34 nmol/mg protein). After several studies to determine its identity, it was confirmed that this new peak corresponded to the cis-8-heptadecenoic fatty acid (C17:1n-9). The formation of C17:1n-9 is a consequence of α oxidation of 2OHOA (figure 11).

### 6.2. Analysis of the composition of fatty acids in different glioma and tumor cells after treatment with 2OHOA sodium salt

The fatty acid composition of the lipid membranes in other glioma cell lines (U-251 MG and SF-295) was analyzed in comparison to non-tumor cells, human fibroblasts (MRC-5), and primary cultures of mouse astrocytes, after incubation in the absence or presence of sodium salt of 2OHOA sodium salt (400 µM, 24 hours) by gas chromatography. No significant change in the amount of OA was observed after treatment with 2OHOA sodium salt in any of the cell lines analyzed (figure 13). However, the incorporation of 2OHOA was observed, as well as the formation of the metabolite C17:1n-9, both in other glioma cell lines and in non-tumor cells, after 24 hours of treatment with 2OHOA (figure 13). The formation of the metabolite C17:1n-9, from the incorporation of 2OHOA, differs between tumor and non-tumor cells. Glioma cells (U-251 MG and SF-295) showed a significant increase in their C17:1n-9 levels; accumulating 97.42% and 108.03% more than 2OHOA (19.16 ± 0.53 vs. 9.21 ± 0.41 and 18.38 ± 1.97 vs. 9.31 ± 1.44 nmol/mg nmol/mg, respectively) (figures 13B and 13d, table 5). In contrast, in non-tumor cells, detected levels of 2OHOA were significantly higher than those of their metabolite C17:1n-9. 46% more 2OHOA was accumulated compared to its metabolite C17:1n-9 in human MRC-5 fibroblasts (26.31 ± 4.32 vs. 14.00 ± 1.92 nmol/mg) and 38.27% in mouse astrocytes (12.28 ± 0.90 vs. 7.58 ± 0.70 nmol/mg) (figures 13f and 13H, table 5).

**Table 5: Levels of the fatty acids 2OHOA and C17:1n-9 in different glioma and non-tumor cell lines after treatment with 2OHOA. Quantification values of 2OHOA and C17:1n-9 fatty acids in different glioma lines, U-118 MG, U-251 MG and SF-295 (above) and non-tumor, MRC-5 and astrocytes, (below) after treatment with 2OHOA (400 µM for 24 hours) determined by gas chromatography. The results correspond to the mean ± SEM of three independent experiments expressed in nmoles and normalized per mg of protein.**

| | **U-119MG** | | **U.251MG** | | **SF-295** | |
|---|---|---|---|---|---|---|
| | **Control** | **2OHOA** | **Control** | **2OHOA** | **Control** | **2OHOA** |
| **2OHOA** | 0.00±0.00 | 10.81±0.34 | 0.00±0.00 | 9.31±1.44 | 0.00±0.00 | 9.21±0.41 |
| **C17:1n-9** | 0.00±0.57 | 19.71±0.39 | 1.65±0.66 | 18.38±1.97 | 1.08±0.59 | 19.16±0.53 |

| | **MRC-5** | | | **ASTROCYTES** | | |
|---|---|---|---|---|---|---|
| | **Control** | | **2OHOA** | **Control** | | **2OMOA** |
| | **2OHOA** 0.00±0.00 | | 26.31±4.32 | 0.00 ±0.00 | | 12.28±0.90 |
| **C17:1n-9** | 1.08±0.46 | | 14.00±1.92 | 2.30 ± 0.34 | | 7.58 ± 0.70 I |

### 6.3. Effect of 2OHOA, C17:1n-9 on cell viability and proliferation of glioma cells

In order to evaluate the antiproliferative effect of C17:1n-9, its IC₅₀, which corresponds to the amount of a compound needed to reduce cell viability *in vitro* by 50%, as well as its effect on the regulation of proteins involved in the mechanism of action of 2OHOA, were determined. To do this, glioma cell lines (U-118 MG, U-251 MG and SF-295) and non-tumor cell lines (MRC-5 and astrocytes) were treated with increasing concentrations of C17:1n-9, OA and 2OHOA sodium salt for 72 hours. Upon completion of treatment, IC₅₀ was determined by violet crystal staining technique. Results of the cell viability assays showed that the three compounds, 2OHOA, OA and C17:1n-9, had an antiproliferative effect on all glioma cells tested, in a concentration-dependent manner, after 72 hours of treatment. Moreover, in the non-tumor cells studied, MRC-5 and astrocytes, no effect of 2OHOA on their cell viability was observed, but the OA and C17:1n-9 fatty acids did produce an antiproliferative effect on the same non-tumor cells (figures 14 and 15). IC₅₀ values of 2OHOA sodium salt were 432.75 ± 10.77, 429.96 ± 9.67, and 399.14 ± 11.47 µM in U-118 MG, U-251 MG, and SF-295 glioma cells, respectively (table 6). The IC₅₀ of 2OHOA was 1000 µM for non-tumor cells, MRC-5 and astrocytes. For compound C17:1n-9, the IC₅₀ values were 222.04 ± 9.09, 220.35 ± 7.93, and 248.85 ± 6.02 µM in glioma cells U-118 MG, U-251 MG, and SF-295, respectively.

Thus, C17:1n-9 induced a highly similar antiproliferative effect on both glioma and non-tumor cells. Meanwhile, treatment with 2OHOA only affected the viability of the different glioma cell lines, without affecting the viability of the non-tumor cells. The IC₅₀ values of 2OHOA were 1.90, 1.95, and 1.60 times greater than those of the metabolite C17:1n-9 in the U-118 MG, U-251 MG, and SF-295 glioma cells, respectively (table 6). In addition, the IC₅₀ values of 2OHOA were 1.92, 1.80 and 1.56 times higher than those of its non-hydroxy analogue OA. The fact that C17:1n-9 has shown a higher antiproliferative potency may be due to the fact that it has a higher accumulation capacity in the cells than 2OHOA.

**Table 6. IC₅₀ values for different cell lines after treatment with 2OHOA, OA and C17:1n-9. Summary of IC₅₀ of glioma cell lines (U-118 MG, U-251 MG, and SF-295) and non-tumor cells (MRC-5 and astrocytes), calculated from results obtained in figures 16 and 17. The IC₅₀ values obtained correspond to the average of three independent experiments and calculated using a dose-response equation using the statistical program GraphPad prism 6.0 (sigmoid model).**

| | **U-118MG** | **U-251 MG** | **SF-295** | **MRC-5** | **Astrocytes** |
|---|---|---|---|---|---|
| **2OHOA** | 432.75 ± 10.77 | 429.96 ± 9.67 | 399.14 ± 11.47 | 1000 | 1000.00 |
| **OA** | 225.68 ± 7.30 | 236.96 ± 6.52 | 256.06 ± 5.79 | 236.31 ± 6.73 | 256.355.14 |
| **C17:1n-9** | 222.04 ± 9.09 | 220.35 ± 7.93 | 248.85 ± 6.02 | 248.03 ± 7.28 | 231.81 6.41 |

### 6.4. Analysis of the effect of different fatty acids on proliferation and death markers in different cell lines

The effect of the metabolite C17:1n-9 on different signaling pathways that are altered by the effect of 2OHOA was analyzed. For this purpose, the different glioma cell lines (U-118 MG, U-251 MG and SF-295) and non-tumoral (MRC-5 and mouse astrocytes) were treated with doses close to IC₅₀ of each of the compounds (200 µM C17:1n.9, 200 µM OA or 400 µM 2OHOA) for 72 hours and their effect on different signaling proteins was analyzed by Western Blot.

The results showed that treatment with 2OHOA increased levels of BIP, CHOP and cJun phosphorylation and decreased phosphorylation of Akt and cyclin D3 levels. Instead, treatment with C17:1n-9 did not produce changes in any of these proteins (figure 16). This suggests that death induced by the metabolite C17:1n-9 is triggered via routes other than those of 2OHOA.

### 6.5. Analysis of fatty acid composition in U-118 MG glioma cells after inhibition of α-oxidation and determination of the effect of oxythiamine on cell survival of U-118 MG glioma cells

To confirm the formation of C17:1n-9 acid from 2OHOA by α-oxidation, oxythiamine chloride was used, which inhibits the enzyme 2-hydroxyfitanoyl-CoA lyase (HACL1, key enzyme in α-oxidation), among other functions. To do this, the U-118 MG glioma cells were first pre-incubated with 1 or 10 mM oxythiamine for 90 minutes, then treated with 400 µM of the 2OHOA sodium salt for 24 hours and the fatty acids were analyzed by gas chromatography. In the analysis of certain fatty acids detected by gas chromatography, a significant reduction in C17:1n-9 was observed in U-118 MG glioma cells pre-incubated with oxythiamine and treated with 2OHOA sodium salt relative to cells treated only with 2OHOA sodium salt without oxythiamine (figure 17 A). This reduction was 51.35% after pre-incubation with 1 mM oxythiamine (17.17 ± 1.07 to 8.35 ± 0.36 nmol/mg protein); and 58.45% with 10 mM oxythiamine (7.13 ± 0.39 nmol/protein), relative to the amount of metabolite that was reached in 2OHOA-treated cells without the presence of oxythiamine. In contrast, no significant differences were detected in the amounts of 2OHOA between cells treated with 2OHOA sodium salt and up to 3 mM oxythiamine compared to those treated with 2OHOA sodium salt only (figure 17A). However, a significant increase in the amount of 2OHOA of 12.33% was observed in cells treated with 2OHOA sodium salt and 4mM oxythiamine (12.41 ± 0.75 to 15.74 ± 0.24 nmol/protein); and 52.94% with 10 mM oxythiamine (18.98 ± 0.42 nmol/protein). Thus, oxythiamine inhibited the formation of C17:1n-9 and increased the amounts of 2OHOA from 4 mM, due to inhibition of α-oxidation, confirming the metabolism pathway of 2OHOA to C17:1n-9.

Next, to determine whether inhibition of 2OHOA metabolism through α-oxidation of 2OHOA has effects on cell viability, cell survival of U-118 MG glioma cells following incubation in the absence or presence of 2OHOA (400 mM, 72 hours), and pre-incubated with oxythiamine at the doses described above, was studied by vital exclusion staining with trypan blue. Oxythiamine induced a significant decrease in the survival of U-118 MG glioma cells. In detail, at 1 mM induced 12.16 ± 0.5% death, 21.17 ± 1.76% death at 2 mM, until reaching a maximum cell survival inhibition of 27.13 ± 0.41% at 10 mM oxythiamine (figure 7B). These results confirm the *in vitro* antitumor effect of oxythiamine that was already known.

On the other hand, incubation of the cells with 2OHOA sodium salt induced 24.71 ± 1.88% cell death; and after the combination with 1 mM oxyamine there was a recovery in cell viability of 5% (20.94 ± 1.97% death); and of 17.26% (11.71 ± 1.14% death) in the case of 2 mM oxyamine (figure 17B).

In view of figures 17A and B, it is observed that the levels of C17:1n-9 do not vary much between the lowest and highest concentration of oxythiamine. This can be interpreted on the basis that, at low concentrations (1.2 mM), oxythiamine is less cytotoxic on its own, but its effect in decreasing the production of C17:1n-9 are very evident. At these concentrations we see the effect of lowering levels of C17:1n-9, but at higher concentrations of oxythiamine, we begin to see more cytotoxicity produced by oxythiamine itself, an effect that seems to be enhanced with high concentrations of 2OHOA sodium salt. At high concentrations more accumulated 2OHOA is detected, which would indicate that the administration of the sodium salt of 2OHOA also produces cytotoxicity by itself and that it adds to the cytotoxic effect of oxythiamine at such concentrations.

### 6.6. Effect of metabolite C17:1n-9 on the action of 2OHOA

To study whether the metabolite C17:1n-9 can participate in the action of 2OHOA, the effect of pre-incubation with oxythiamine on cell survival and 2OHOA-regulated proteins was studied. To do this, the cell survival of different glioma and non-tumor cell lines treated with 2OHOA (400 µM, 72 hours) and pre-incubated or not with 2 mM oxyamine (90 minutes) was analyzed by counting the cells with the vital exclusion stain with trypan blue. In addition, Western-blot 2OHOA-modulated proteins were studied. In glioma cells, a significant decrease in cell survival was observed after incubation with 2 mM oxythiamine for 72 hours. Oxythiamine induced 18.51 ± 0.58% and 17.35 ± 0.63% cell death in U-251 MG and SF-295 cells, respectively (figure 18A and 18b). These results support the *in vitro* anti-tumor effect of oxythiamine on glioma cells. Treatment of cells with 2OHOA induced 23.22 ± 1.32% and 23.97 ± 1.25% cell death in U-251 MG and SF-295, respectively. Following combination with 2 mM oxythiamine, there was a significant recovery in cell viability of 12% (14.07± 1.62% death in U-251 MG cells) and 17.25% (10.85± 0.58% death) in SF-295 cells. In contrast, in non-tumor cells, none of the treatments tested produced an effect on cell survival (figure 18c and 18d).

As for the study of proteins involved in different signaling pathways and cell death in glioma cells, oxythiamine had an effect on the levels of BiP, CHOP, c-Jun phosphorylation, Akt phosphorylation, and D3 cyclin in glioma cells in the same sense as 2OHOA, although milder (figure 19), when combined, modulation induced by 2OHOA was inhibited. This fact confirms that the metabolism of 2OHOA in C17:1n-9 is necessary to enhance its anti-tumor action. On the contrary, no change in such signaling proteins in non-tumor cell lines was observed after any of the treatments (figure 19). Although 2OHOA has an antiproliferative activity when its metabolism in C17:1n-9 is inhibited, the formation of the metabolite C17:1n-9 has a great impact on the mechanism of action of 2OHOA, enhancing its antiproliferative effect, and confirms that 2OHOA is also a prodrug that gives rise to an active metabolite 17:1n-9.

### Example 7: In vivo trials with 2OHOA and C17:1n-9

### 7.1 Analysis of the composition of fatty acids in rat plasma after 24 hours of treatment with 2OHOA

The pharmacokinetic profile of 2OHOA and its metabolite C17:1n-9 in animal plasma was studied. In this case, rats were used as an animal model of experimentation. Rats have a higher volume than mice, and are the most suitable model for studying the effect of continued administration of the maximum tolerated dose of 2OHOA (2 g/Kg) defined in preclinical studies.

For the present study, 2 g of 2OHOA/Kg sodium salt was administered to rats 12-14 weeks of age orally for 15 days. Subsequently, plasma samples were extracted at different times (0, 1, 2, 3, 4, 6, 8 and 24h) from day 1 (acute treatment) and 15 (chronic treatment). Finally, the fatty acid profile in plasma samples was analyzed by gas chromatography. After analyzing the chromatograms, the detection of 2OHOA and C17:1n-9 fatty acids in plasma samples collected after acute treatment (first day administration) was notable (figure 20A).

The two compounds, 2OHOA and C17:1n-9, showed a very similar pharmacokinetic profile in rat plasma following acute treatment (figure 20B). A significant increase in 2OHOA and C17:1n-9 levels was observed, reaching a maximum plasma concentration at 2 hours of administration with 2OHOA (26.23 ± 5.79 nmol 2OHOA/ml plasma and 60.47 ± 6.53 nmol C17:1n-9/ml plasma). There was a subsequent decrease in plasma 2OHOA and C17:1n-9 levels reaching trough values at 24 hours (2.80 ± 0.69 and 14.03 ± 2.20 nmol /ml plasma, respectively). However, the initial levels were not reached especially in the case of C17:1n-9 (1.22 ± 0.33 and 8.45 ± 2.52 nmol/ml plasma, respectively). The levels of the C17:1n-9 metabolite after chronic treatment were higher than those of 2OHOA (figure 21B). The differences between the two compounds were significant prior to administration of 2OHOA (0 hours; 1.22 ± 0.33 nmol 2OHOA/mL plasma, relative to 8.45 ± 2.52 nmol C17:1n-9/plasma), after 8 hours (7.12 ± 1.56 nmol 2OHOA/mL plasma, relative to 23.31 ± 5.18 nmol C17:1n-9/plasma) and at 24 hours (2.80 ± 0.69 nmol 2OHOA/mL plasma, relative to 14.03 ± 2.21 nmol C17:1n-9/plasma).

### 7.2. Analysis of the composition in fatty acids of xenographic tumors of immunosuppressed mice

In order to study the effects in animal models of the formation of C17:1n-9 as a product of the metabolization of 2OHOA by α-oxidation, the levels of the metabolite C17:1n-9, compared to those of 2OHOA, were detected and analyzed in a model of xenographic tumors in immunodepressed mice. To do this, U-118 MG glioblastoma cells were injected into immudepressed mice and, one week later, treatment of mice with vehicle or 2OHOA sodium salt (200 mg/kg) was initiated orally and daily for 42 days. Once treatment was complete, mice were euthanized and tumors were removed, lipids were processed for 2OHOA and C17:1n-9 fatty acids by gas chromatography. Fatty acid 2OHOA was not detected in the xenographic tumors of mice treated with this compound, as no peak in the retention time corresponding to 2OHOA was observed (figure 22A). In contrast, the metabolite of 2OHOA, C17:1n-9 fatty acid (0.25 ± 0.04 nmol C17:1n-9/g tissue), was detected in the tumors of mice treated with 2OHOA (figure 22B).

### 7.3. Correlation between the volume of tumours and the amount of the metabolite C17:1n-9

The possible correlation was studied between the levels of the C17:1n-9 metabolite present in tumors with respect to the volume of tumors, as an indication of the relationship between the incorporation and metabolization of 2OHOA and the efficacy of the compound in tumors. In the graphs obtained, a negative correlation was observed between the amount of C17:1n-9present in the tumors and the volume of the tumors (figure 23). A coefficient of determination r of - 0.8248 and a p-value of 0.0001 were obtained for the tumors of mice treated with 2OHOA between the amount of C17:1n-9 and the volume of the tumors. That is, the smaller the volume of the tumor, the more of the C17:1n-9 metabolite was detected in it. These results show that the metabolite C17:1n-9 has marked antitumor activity and 2OHOA is an effective prodrug of this compound.

### 7.4. Fatty acid composition analysis in human patients with advanced glioma after treatment with 2OHOA

2OHOA and C17:1n-9 fatty acids were detected and quantified in plasma samples from 8 patients who responded, or not, to treatment with 12 g/day of 2OHOA sodium salt for at least one 3-week cycle in clinical phase I/IIA of 2OHOA (MIN-001-1203). Plasma samples were obtained at different times (0, 2, 4, 6, 8 hours and after 8, 15, 21 and 28 days after treatment with 2OHOA) and were subsequently given for fatty acid analysis using the gas chromatography technique.

In the chromatograms, 2OHOA and its C17:1n-9 metabolite were detected in all plasma samples from patients analyzed (figure 24A). A very similar pharmacokinetic profile was observed in all patients, both those who showed clinical response (responders) and those who did not (non-responders) (figure 24B). Both compounds reached peak levels at 4 hours of administration with 2OHOA. Analyzing the results of all patients, responders and non-responders, values of 53.08 ± 6.52 nmol of 2OHOA/ml of plasma and 122.80 ± 10.61 nmol of C17:1n-9/ml of plasma were obtained 4 hours after the first intake of the drug (figure 24C). Subsequently, the levels of 2OHOA and C17:1n-9 gradually decreased until 8 hours after treatment (25.39 ± 3.99 and 92.89 ± 9.39 nmol/ ml of plasma, respectively). At 8 days of treatment (192 hours) a significant increase in the amounts of the two plasma compounds, 192 hours (25.39 ± 3.99 nmol/ml 2OHOA plasma and 141.10 ± 16.35 nmol/ml C17:1n-9 plasma) was observed. Compounds 2OHOA and C17:1n-9 accumulated in patients' plasma over time, as observed after 15 days (360 hours) of treatment with 2OHOA (184.70 ± 25.60 and 366.9 ± 72.47 nmol/ml 2OHOA and C17:1n.9 plasma, respectively) (figure 24C).

It should be noted that, similarly to what happened in cells and animals, the levels of the metabolite C17:1n-9 in the plasma of all patients were higher than those of 2OHOA (figure 14B), being significantly higher after 8 hours of administration of 2OHOA (figure 24C). C17:1n-9 levels were observed to be 3.66 and 2.20 times higher than 2OHOA in patients who had been treated with 2OHOA for 8 and 15 days, respectively (92.89 ± 9.39 and 311.10 ± 37.38 and nmol C17:1n-9/ml plasma compared to 25.39 ± 3.99 and 141.10 ± 16.35 nmol 2OHOA/ml plasma, respectively). Finally, at 21 days of treatment with 2OHOA C17:1n-9 levels were 1.90 times higher than those of 2OHOA (366.9 ± 72.47 nmol C17:1n-9/ml plasma compared to 184.70 ± 25.60 nmol 2OHOA/ml plasma, respectively).

## Claims

1. A compound selected from the group consisting of:
a compound of formula (II), or a pharmaceutically or nutraceutically acceptable salt or ester thereof:
COOH -(CH₂)*ₐ*-(CH=CH-CH₂)*_{b}*-(CH₂)*_{c}*-CH₃ (II)
and a compound of formula (III), or a pharmaceutically or nutraceutically acceptable salt or ester thereof:
COOH -(CH₂)*ₐ*-(CH=CH-CH₂)*ₘ*-(CH₂)₃-(CH=CH-CH₂)_{(*b-1-m*)}-(CH₂)*_{c}*-CH₃ (III);
wherein ***a*** is an integer between 1 and 14; ***b*** is an integer between 1 and 7; c is an integer between 0 and 14; ***m*** is an integer between 0 and (***b*** - 1); and wherein ***a***+3***b***+***c***+3 is an even integer.

2. A compound according to claim 1, wherein ***c*** is 0, 3 or 6 and ***m***=0.

3. A compound of formula (II), or a pharmaceutically or nutraceutically acceptable salt or ester thereof, according to claim 1, wherein: ***a***=6, ***b*** = 1 and ***c*** = 6; or ***a***=6, ***b** =* 2 and ***c*** = 3; or ***a***= 6, ***b*** = 3 and ***c*** = 0; or ***a***= 3, ***b*** =3 and ***c***=3; or ***a***= 2, ***b***=4 and ***c***=3; or ***a***=2, ***b**=*5 and c=0.

4. A compound of formula (III), or a pharmaceutically or nutraceutically acceptable salt or ester thereof, according to claim 1, wherein ***a***=1, ***b**=6, **c***=0 and ***m***=*0.*

5. A compound according to any one of claims 1 to 4 wherein said pharmaceutically or nutraceutically acceptable salt is a sodium salt.

6. A compound according to any of claims 1 or 5, for use as a medicament.

7. A compound according to any one of claims 1 to 5, for use in inducing neuroregeneration and in preventing and/or preventing a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

8. A compound for use according to claim 7, wherein the prevention and/or treatment or induction of neuroregeneration is **characterized by** administering a compound or prodrug of formula (I), or a pharmaceutically acceptable salt or ester thereof:
COOH -CHOH-(CH₂)*ₐ*-(CH=CH-CH₂)*_{b}*-(CH₂)*_{c}*-CH₃ (I)
wherein the values of ***a***, ***b*** and ***c*** are equal to the values of ***a***, ***b*** and ***c*** of the compound of formula (II) or of the compound of formula (III); and wherein said compound of formula (I) is metabolized to produce a therapeutically effective amount of a compound of formula (II) or a compound of formula (III).

9. A compound for use according to claim 7, wherein said compound is administered before, after or in conjunction with a compound of formula (I) or a pharmaceutically acceptable salt or ester thereof:
COOH -CHOH-(CH₂)*ₐ*-(CH=CH-CH₂)*_{b}*-(CH₂)*_{c}*-CH₃ (I)
wherein ***a*** is an integer between 1 and 14; ***b*** is an integer between 1 and 7; ***c*** is an integer between 0 and 14 and ***a***+3***b*** +***c***+3 is an even integer; and wherein said values of ***a***, ***b*** and ***c*** are equal to or different from the values of ***a***, ***b*** and ***c*** of the compound of formula (II) or the compound of formula (III).

10. A pharmaceutical composition comprising at least one first compound selected from the group consisting of:
- a compound of formula (II), or a pharmaceutically acceptable salt or ester thereof:
COOH-(CH₂)*ₐ*-(CH=CH-CH₂)*_{b}*-(CH₂)*_{c}*-CH₃ (II)
and
- a compound of formula (III), or a pharmaceutically acceptable salt or ester thereof:
COOH -(CH₂)*ₐ*-(CH=CH-CH₂)*ₘ*-(CH₂)₃-(CH=CH-CH₂)_{(*b-1-m*)}-(CH₂)*_{c}*-CH₃ (III);
wherein ***a*** is an integer between 1 and 14; ***b*** is an integer between 1 and 7; ***c*** is an integer between 0 and 14; ***m*** is an integer between 0 and (***b***-1); and ***a***+3***b***+***c***+3 is an even integer; and at least one pharmaceutically acceptable excipient.

11. A pharmaceutical composition according to claim 10, further comprising a second compound of formula (I), or a pharmaceutically acceptable salt or ester thereof:
COOH -CHOH-(CH₂)*ₐ*-(CH=CH-CH₂)*_{b}*-(CH₂)*_{c}*-CH₃ (I)
wherein ***a*** is an integer between 1 and 14; ***b*** is an integer between 1 and 7; ***c*** is an integer between 0 and 14 and ***a***+3***b*** +***c***+3 is an even integer; and wherein said values of ***a***, ***b*** and c of the second compound are equal to or different from the values of ***a***, ***b*** and ***c*** of the at least first compound.

12. A pharmaceutical composition according to any one of claims 10 or 11, wherein ***c*** is 0, 3 or 6 and ***m***=0.

13. A pharmaceutical composition according to any one of claims 10 or 11, wherein the at least first compound is a compound of formula (II), or a pharmaceutically acceptable salt or ester thereof, wherein: ***a***=6, ***b***=1 and ***c***=6; or ***a***=6, ***b***=2 and ***c***=3; or ***a***=6, ***b***=3 and ***c***=0; or ***a***=3, ***b***=3 and ***c***=3; or ***a***=2, ***b***=4 and ***c***=3; or ***a***=2, ***b***=5 and ***c***=0.

14. A pharmaceutical composition according to any one of claims 10 or 11, wherein the at least one first compound is a compound of formula (III), or a pharmaceutically acceptable salt or ester thereof, wherein ***a***=1, ***b***=6, ***c***=0 and ***m***=0.

15. A pharmaceutical composition according to any one of claims 10 to 14, wherein the pharmaceutically acceptable salt is a sodium salt.

16. A pharmaceutical composition according to claim 15, for use as a medicament.

17. A pharmaceutical composition according to any one of claims 10 to 15, for use in inducing neuroregeneration and in preventing and/or treatment of a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

18. A pharmaceutical composition according to claim 17, wherein the disease or pathology is a cancer.

19. A pharmaceutical composition according to claim 18, wherein said pharmaceutical composition is administered before, after, or in conjunction with a radiotherapeutic treatment, a chemotherapeutic treatment, or an electric field treatment for the treatment of tumors.

20. A nutraceutical composition comprising at least one first compound selected from the group consisting of:
- a compound of formula (II), or a nutraceutically acceptable salt or ester thereof:
COOH-(CH₂)*ₐ*-(CH=CH-CH₂)*_{b}*-(CH₂)*_{c}*-CH₃ (II)
and
- a compound of formula (III), or a nutraceutically acceptable salt or ester thereof:
COOH -(CH₂)*ₐ*-(CH=CH-CH₂)*ₘ*-(CH₂)₃-(CH=CH-CH₂)_{(*b-1-m*)}-(CH₂)*_{c}*-CH₃ (III);
wherein ***a*** is an integer between 1 and 14; ***b*** is an integer between 1 and 7; ***c*** is an integer between 0 and 14; ***m*** is an integer between 0 and (***b***-1); and ***a***+3***b***+***c***+3 is an even integer; and at least one nutraceutically acceptable excipient.

21. A nutraceutical composition according to claim 20, further comprising a second compound of formula (I), or a nutraceutically acceptable salt or ester thereof:
COOH -CHOH-(CH₂)*ₐ*-(CH=CH-CH₂)*_{b}*-(CH₂)*_{c}*-CH₃ (I)
wherein ***a*** is an integer between 1 and 14; ***b*** is an integer between 1 and 7; ***c*** is an integer between 0 and 14 and ***a***+3***b*** +***c***+3 is an even integer; and wherein said values of ***a***, ***b*** and ***c*** of the second compound are equal to or different from the values of ***a***, ***b*** and ***c*** of the at least first compound.

22. A nutraceutical composition according to any one of claims 20 or 21, wherein ***c*** is 0, 3 or 6 and ***m***=0.

23. A nutraceutical composition according to any one of claims 20 or 21, wherein the at least first compound is a compound of formula (II), or a nutraceutically acceptable salt or ester thereof, wherein: ***a***=6, ***b*** = 1 and ***c***=6; or ***a***= 6, ***b***=2 and ***c***=3; or ***a***= 6, ***b***=3 and ***c***=0; or ***a***=3, ***b***=3 and ***c***=3; or ***a***=2, ***b***=4 and ***c***=3; or ***a***=2, ***b***=5 and ***c***=0.

24. A nutraceutical composition according to any one of claims 20 or 21, wherein the at least first compound is a compound of formula (III), or a nutraceutically acceptable salt or ester thereof, wherein ***a***=1, ***b***=6, ***c***=0 and ***m***=0.

25. A nutraceutical composition according to any one of claims 20 to 24, wherein the nutraceutically acceptable salt is a sodium salt.

26. A nutraceutical composition according to any one of claims 20 to 25, for use in the prevention of a disease or pathology selected from the group consisting of: a neurological or neurodegenerative disease; a cancer; a neoplasm; an inflammatory disease; a cardiovascular disease; a skin and subcutaneous tissue pathology; a metabolic pathology; neuropathic pain; paralysis; sleep disorders; a digestive pathology; a musculoskeletal and connective tissue disease; a genitourinary pathology; and a metabolic disease.

27. *In vitro* method for determining the efficacy of a therapeutic or preventive treatment of a disease or pathology with a compound of formula (I), or with a pharmaceutically acceptable salt or ester thereof: COOH -CHOH-(CH₂)*ₐ*-(CH=CH-CH₂)*_{b}*-(CH₂)*_{c}*-CH₃ (I) in a subject, wherein said method comprises determining *in vitro* in a biological sample of said subject, the amount of a compound:
- of formula (II):
COOH -(CH₂)*ₐ*-(CH=CH-CH₂)*_{b}*-(CH₂)*_{c}*-CH₃ (II)
or
- of formula (III):
COOH-(CH₂)*ₐ*-(CH=CH-CH₂)*ₘ-*CH₂)₃-(CH=CH-CH₂)_{(*b-1-m*)}-(CH₂)*_{c}*-CH₃ (III)
or its carboxylate anion, or of a derivative formed therefrom *in vivo* or *in vitro,* wherein said amount is related to the efficacy of treating said disease or pathology; and wherein a is an integer between 1 and 14; ***b*** is an integer between 1 and 7; ***c*** is an integer between 0 and 14; ***m*** is an integer between 0 and (***b** -* 1); and wherein ***a***+3***b***+***c***+3 is an even integer.

28. A method according to claim 27, wherein ***c*** is 0, 3 or 6 and ***m***=0.

29. A method according to claim 27, wherein said method comprises determining *in vitro* in a biological sample of said subject, the amount of a compound of formula (II):
COOH -(CH₂)*ₐ*-(CH=CH-CH₂)*_{b}*-(CH₂)*_{c}*-CH₃ (II)
or its carboxylate anion, or a derivative formed therefrom *in vivo* or *in vitro,* and wherein: ***a***=6, ***b***=1 and ***c***=6; or ***a***=6, ***b***=2 and ***c***=3; or ***a***=6, ***b***=3 and ***c***=0; or ***a***=3, ***b***=3 and ***c***=3; or ***a***=2, ***b***=4 and ***c***=3; or ***a***=2, ***b***=5 and ***c***=0.

30. A method according to claim 27, wherein said method comprises determining *in vitro* in a biological sample of said subject, the amount of a compound of formula (III):
COOH -(CH₂)*ₐ*-(CH=CH-CH₂)*ₘ*-(CH₂)₃-(CH=CH-CH₂)_{(*b-1-m*)}-(CH₂)*_{c}*-CH₃ (III)
or its carboxylate anion, or a derivative formed therefrom *in vivo or in vitro,* and in which ***a***=1, ***b*=**6, ***c***=0 and ***m***=0.

31. A method according to any one of claims 27 to 30, wherein the pharmaceutically acceptable salt is a sodium salt.
